# EUROPEAN PATENT APPLICATION

(11) **EP 1 816 122 A2**
(43) Date of publication of application: **08.08.2007**
(21) Application number: 07100713.2
(22) Date of filing: 18.01.2007
(51) Int. Cl.: C07D 211/42, C07D 401/12, C07D 401/06, A61K 31/445, A61K 31/4545, A61K 31/453, A61P 25/28, A61P 31/18, A61P 43/00, A61P 25/18, A61P 33/06

(54) **3,4,5-substituted piperidines as therapeutic compounds**

(30) Priority: 19.01.2006 CH 882006
(71) Applicant: Speedel Experimenta AG, 4123 Allschwil (CH)
(72) Inventor: Herold, Peter, 4123, Allschwil (CH); Mah, Robert, 4123, Allschwil (CH); Stutz, Stefan, 4123, Allschwil (CH); Stojanovic, Aleksandar, 4123, Allschwil (CH); Tschinke, Vincenzo, 4123, Allschwil (CH); Jotterand, Nathalie, 4123, Allschwil (CH); Schumacher, Christoph, 4123, Allschwil (CH); Behnke, Dirk, 4123, Allschwil (CH)
(74) Representative: Maué, Paul Georg

(57) **Abstract**

Use of compounds of the general formula (I) 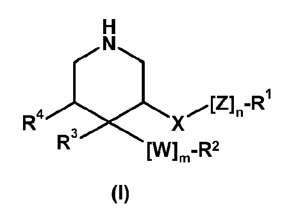 and pharmaceutically acceptable salt thereof, in which R¹, R², R³, R⁴, W, X and Z, n and m have the definitions illustrated in detail in the description, as beta-secretase, cathepsin D, plasmepsin II and/or HIV protease inhibitors.

## Description

### Field of the Invention

The present invention relates to the use of 3,4,5-trisubstituted piperidines as beta-secretase-, cathepsin D-, plasmepsin II- and/or HIV-protease-inhibitors.

### Background of the Invention

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias

### Alzheimer's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and

HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

### Detailed Description of the Invention

Firstly, the present invention relates to the use as beta-secretase-, cathepsin D-, plasmepsin II- and/or HIV-protease-inhibitors of compounds of the general formula 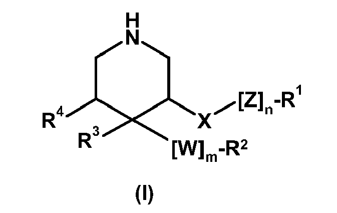 in which
(A) R¹ is heterocyclyl, optionally substituted with oxo or oxide, or as specified under (E) or (F), in particular azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl,dihydroimidazolyl, 1,3-dihydroindolyl, 2,3-dihydroindolyl, dihydro-1H-pyrido[2,3-b][1,4]oxazinyl, 1,1-dioxodihydro-2H-benzo[1,4]thiazinyl,indazolyl, indolyl, 3H-isobenzofuranyl, [1,5]naphthyridyl,oxazolyl, 2-oxoazepanyl, 3-oxo-4H-benzo[1,4]oxazinyl, 2-oxobenzoxazolyl, 3-oxo-4H-benzo[1,4]thiazinyl, 2-oxodihydrobenzo[e][1,4]diazepinyl, 2-oxodihydrobenzo[d][1,3]oxazinyl, 2-oxodihydro-1 H-quinazolinyl, 4-oxodihydroimidazolyl, 2-oxo-1,3-dihydroindolyl, 1-oxo-3H-isobenzofuranyl, 2-oxopiperidinyl 2-oxo-1H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, piperidinyl, pyrazolyl, 1 H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1 a,2,7b-tetrahydrocyclopropa[c]chromenyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl;
(B) R¹ is aryl when R² is tetrazolyl or imidazolyl which radicals may be substituted by 1-3 halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₂₋₈-alkenyloxy-C₁₋₈-alkyl, C₁₋₈-alkoxy-C₁₋₈-alkylamino-C₁₋₈-alkyl, C₁₋₈-alkoxy-C₁₋₈-alkylsulfanyl-C₁₋₈-alkyl, C₁₋₈-alkoxy-C₃₋₈-cycloalkyl-C₀₋₈-alkoxy-C₁₋₈-alkyl, C₁₋₈-alkylsulfanyl-C₁₋₈-alkoxy-C₁₋₈-alkyl, C₁₋₈-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, optionally halogen-substituted C₁₋₈-alkoxy-C₁₋₈-alkoxy-C₁₋₈-alkyl, or heterocyclyl-C₀₋₈-alkoxy-C₁₋₈-alkyl groups, or a C₁₋₈-alkylenedioxy group, and/or by an L1-T1-L2-T2-L3-T3-L4-T4-L5-U radical; or
(C) R¹ is aryl when X is -O-CH-R¹¹-CO-NR⁹-; or
(D) R¹ is aryl when Z is -alk-NR⁹- where alk denotes C₁₋₈-alkylene, and n is 1; or
(E) R¹ is aryl which is substituted by 1-4 acetamidinyl-C₁₋₈-alkoxy, acetamidinyl-C₁₋₈-alkyl, acyl-C₁₋₈-alkoxy-C₁₋₈-alkyl, (N-acyl)-C₁₋₈-alkoxy-C₁₋₈-alkylamino, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, 1-C₁₋₈-alkoxy-C₁₋₈-alkylimidazol-2-yl, 2-C₁₋₈-alkoxy-C₁₋₈-alkyl-4-oxoimidazol-1-yl, 1-C₁₋₈-alkoxy-C₁₋₈-alkyltetrazol-5-yl, 5-C₁₋₈-alkoxy-C₁₋₈-alkyltetrazol-1-yl, 6-alkoxyaminocarbonyl-C₁₋₈-alkoxy, C₁₋₈-alkoxyaminocarbonyl-C₁₋₈-alkyl, C₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkylcarbonylamino, (N-C₁₋₈-alkyl)-C₁₋₈-alkoxycarbonylamino, (N-C₁₋₈-alkyl)-C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylamidinyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, C₁₋₈-alkylaminocarbonylamino-C₁₋₈-alkoxy, C₁₋₈-alkylaminocarbonylamino-C₁₋₈-alkyl, di-C₁₋₈-alkylaminocarbonyl-C₁₋₈-alkyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylcarbamoyl, di-C₁₋₈-alkylcarbamoyl, C₀₋₈-alkylcarbonyl, C₀₋₈-alkylcarbonylamino-C₁₋₈-alkoxy, C₀₋₈-alkylcarbonylamino, C₀₋₈-alkylcarbonylamino-C₁₋₈-alkyl, C₁₋₈-alkylcarbonyloxy-C₁₋₈-alkoxy, C₁₋₈-alkylcarbonyloxy-C₁₋₈-alkyl, C₁₋₈-alkylsulphonyl, C₁₋₈-alkylsulphonyl-C₁₋₈-alkoxy, C₁₋₈-alkylsulphonyl-C₁₋₈-alkyl, C₁₋₈-alkylsulphonylamino-C₁₋₈-alkoxy, C₁₋₈-alkylsulphonylamino-C₁₋₈-alkyl, carbamoyl, carbamoyl-C₁₋₈-alkoxy, carbamoyl-C₁₋₈-alkyl, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cycloalkylcarbonylamino-C₁₋₈-alkoxy, C₃₋₈-cycloalkylcarbonylamino-C₁₋₈-alkyl, cyclopropyl-C₁₋₈-alkyl, O,N-dimethylhydroxylamino-C₁₋₈-alkyl, halo-C₁₋₈-alkoxy, halo-C₁₋₈-alkyl, halogen, hydroxy-C₁₋₈-alkoxy-C₁₋₈-alkoxy, hydroxy-C₁₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, (N-hydroxy)aminocarbonyl-C₁₋₈-alkoxy, (N-hydroxy)aminocarbonyl-C₁₋₈-alkyl, 2-oxooxazolidinyl-C₁₋₈-alkoxy, 2-oxooxazolidinyl-C₁₋₈-alkyl, O-methyloximyl-C₁₋₈-alkyl or trifluoromethyl; or
(F) R¹ is aryl which is substituted by 1-4 3-acetamidomethylpyrrolidinyl 3-C₁₋₈-alkoxy-C₁₋₈-alkylpyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolylalkoxy, imidazolylalkyl, 2-methylimidazolylalkoxy, 2-methylimidazolylalkyl, 3-methyl[1,2,4]oxadiazol-5-ylalkoxy, 5-methyl[1,2,4]oxadiazol-3-ylalkoxy, 3-methyl-[1,2,4]oxadiazol-5-ylalkyl, 5-methyl[1,2,4]oxadiazol-3-ylalkyl, 4-methylpiperazinyl, 5-methyltetrazol-1-ylalkoxy, 5-methyltetrazol-1-ylalkyl, morpholinyl, [1,2,4]oxadiazol-5-ylalkoxy, [1,2,4]oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]triazol-1-ylalkoxy, [1,2,4]triazol-4-ylalkoxy, [1,2,4]triazol-1-ylalkyl, [1,2,4]triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazo-4-ylalkyl or thiomorpholinyl;
R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl, furyl, tetrazolyl or imidazolyl, which radicals may be substituted by 1-3 halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₂₋₈-alkenyloxy-C₁₋₈-alkyl, C₁₋₈-alkoxy-C₁₋₈-alkylamino-C₁₋₈-alkyl, C₁₋₈-alkoxy-C₁₋₈-alkylsulfanyl-C₁₋₈-alkyl, C₁₋₈-alkoxy-C₃₋₈-cycloalkyl-C₀₋₈-alkoxy-C₁₋₈-alkyl, C₁₋₈-alkylsulfanyl-C₁₋₈-alkoxy-C₁₋₈-alkyl, C₁₋₈-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, optionally halogen-substituted C₁₋₈-alkoxy-C₁₋₈-alkoxy-C₁₋₈-alkyl, or heterocyclyl-C₀-₈-alkoxy-C₁₋₈-alkyl groups or a C₁₋₈-alkylenedioxy group, and/or by an L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, or are absent;
T1, T2, T3 and T4 are each independently
   (a) a bond, or are absent, or are one of the groups
   (b) -CH(OH)-
   (c) -CH(OR⁶)-
   (d) -CH(NR⁵R⁶)-
   (e) -CO-
   (f) -CR⁷R⁸-
   (g) -O-oder-NR⁶-
   (h) -S(O)₀₋₂-
   (i) -SO₂NR⁶-
   (j) -NR⁶SO₂-
   (k) -CONR⁶-
   (l) -NR⁶CO-
   (m) -O-CO-
   (n) -CO-O-
   (o) -O-CO-O-
   (p) -O-CO-NR⁶-
   (q) -N(R⁶)-CO-N(R⁶)-
   (r) -N(R⁶)-CO-O-
   (s) Pyrrolidinylen, Piperidinylen oder Piperazinylen
   (t) -C(R¹¹)(R¹²),
   -where the bonds starting from (b)-(t) lead to a saturated or aromatic carbon atom of the adjacent group if the bond starts from a heteroatom, and where not more than two groups (b)-(f), three groups (g)-(h) and one group (i)-(t) is/are present;
R³ is hydrogen, hydroxyl, C₁₋₈-alkoxy or C₁₋₈-alkenyloxy;
R⁴ is optionally halogen- and/or hydroxy-substituted C₁₋₈alkyl, optionally halogen- and/or hydroxy-substituted C₁₋₈-alkoxy-C₁₋₈-alkyl , optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₀₋₈-alkylcarbonyl-C₀₋₈-alkyl, C₁₋₈-alkoxy-C₀₋₈-alkylcarbonyl-C₀₋₈-alkyl, optionally N-C₁₋₈-alkylated C₁₋₈-alkoxycarbonylamino-C₁₋₈-alkyl, optionally N-C₁₋₈-alkylated C₁₋₈-alkoxy-C₁₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₁₋₈-alkylamino-C₁₋₈-alkyl, optionally N-C₁₋₈-alkylated or optionally halogen-substituted heterocyclyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocydyl-C₀₋₈-alkylamino-C₀₋₈-alkylcarbonyl-C₀₋₈-alkyl, C₁₋₈-alkylsulphonyl-C₁₋₈-alkyl, C₂₋₈-alkinyl, heterocyclyl-C₂₋₈-alkinyl, optionally N-mono- or N,N-di-C₁₋₈-alkylated amino-C₂₋₈-alkinyl, heterocyclylcarbonyl-C₀₋₈-alkyl, heterocyclyloxy-C₁₋₈-alkyl, optionally N-mono- or N,N-di-C₁₋₈-alkylated amino, C₁₋₈-alkylcarbonyl-C₁₋₈-alkoxy, C₁₋₈-alkylcarbonyloxy, aryl-C₁₋₈-alkoxy, aryloxy, optionally N-mono- or N,N-di-C₃₋₈-cycloalkyl-C₁-C₆-alkylated carbamoyl-C₁₋₈-alkoxy, optionally N-mono- or N,N-di-C₁-C₆-alkylated carbamoyloxy, hydroxyl, hydroxy-C₁₋₈-alkoxy, hydroxy-C₁₋₈-alkoxy-C₁₋₈-alkoxy, optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₁₋₈-alkoxy, optionally N-mono- or N,N-di-C₁₋₈-alkylated or optionally hydroxy-substituted amino-C₀₋₈-alkylcarbonyl-C₁₋₈-alkoxy, hydroxy-C₀₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₋₈-alkoxycarbonylamino-C₁₋₈-alkoxy, optionally N-C₁₋₈-alkylated C₁₋₈-alkoxy-C₁₋₈-alkylamino-C₁₋₈-alkoxy, optionally N-C₁₋₈-alkylated or optionally halogen-substituted C₁₋₈-alkylcarbonylamino-C₁₋₈-alkoxy, cyano-C₁₋₈-alkoxy, optionally N-C₁₋₈-alkylated or optionally halogen-substituted C₃₋₈-cycloalkyl-C₀₋₈-alkylcarbonylamino-C₁₋₈-alkoxy, optionally N-C₁₋₈-alkylated hydroxy-C₁₋₈-alkylamino-C₁₋₈-alkoxy, optionally N-C₁₋₈-alkylated or optionally halogen-substituted heterocyclyl-C₀₋₈-alkylcarbonylamino-C₁₋₈-alkoxy, C₃₋₈-cycloalkyl-C₀₋₈-alkoxy, C₃₋₈-cycloalkyloxy-C₁₋₈-alkoxy, heterocyclyl-C₀₋₈-(optionally hydroxy-substituted)alkoxy, optionally N-C₁₋₈-alkylated heterocyclyl-C₀₋₈-alkylamino-C₀₋₈-alkylcarbonyl-C₀₋₈-alkoxy, C₁₋₈-alkylsulphonyl-C₁₋₈-alkoxy, C₂₋₈-alkinyl-oxy, heterocyclyl-C₂₋₈-alkinyl-oxy, optionally N-mono- or N,N-di-C₁₋₈-alkylated amino-C₂₋₈-alkinyl-oxy, heterocydylcarbonyl-C₀₋₈-alkoxy, heterocyclyloxy-C₁₋₈-alkyoxy or oxo;
R⁵ and R⁶ are each independently hydrogen, C₁₋₈-alkyl, C₂₋₈-alkenyl, aryl-C₁₋₈-alkyl or acyl, or, together with the N atom to which they are bonded, are a 5- to 6-membered heterocyclic ring which may contain an additional N, O or S atom or an -SO- or -SO₂- group, where the additional N atom may optionally be substituted by C₁₋₈-alkyl radicals;
R⁷ and R⁸, together with the carbon atom to which they are bonded, are a 3-8-membered ring which may contain one or two -O- or-S- atoms or-SO- or -SO₂- groups;
R⁹ is hydrogen, C₁₋₈-alkyl, C₁₋₈-alkoxy-C₁₋₈-alkyl, acyl, aryl-C₁₋₈-alkyl, C₃₋₈-cycloalkyl or C₃₋₈-cycloalkyl-C₁₋₈-alkyl;
R¹⁰ is carboxy-C₁₋₈-alkyl, C₁₋₈-alkoxycarbonyl-C₁₋₈-alkyl, C₁₋₈-alkyl or hydrogen;
R¹¹ is hydrogen, halogen, acyl, C₂₋₈-alkenyl, C₁₋₈-alkyl, or aryl-C₁₋₈-alkyl;
R¹² is hydrogen, halogen or C₁₋₈-alkyl;
R¹¹ and R¹², together with the C-atom to which they are attached, may also be C₃₋₈-cycloalkyl;
U is hydrogen, C₁₋₈-alkyl, cyano, trifluoromethyl, optionally substituted C₃₋₁₂-cycloalkyl, aryl, or heterocyclyl;
X is a bond, oxygen or sulphur or is >CR¹¹R¹², >CHOR⁹, -O-CO-, >CO, >C=NOR¹⁰, -O-CR¹¹R¹²-, -O-CR¹¹R¹²-CO-NR⁹-, -CO-NR⁹- or -NR⁹-, where a bond starting from a nitrogen, oxygen or sulphur atom leads to a saturated C atom of the Z group or to R¹;
W is oxygen or sulphur;
Z is C₁₋₈-alkylene, C₂₋₈-alkenylene, hydroxyl substituted-C₁₋₈-alkylene, -O-, -N-, -S-, -O-alk-, -NR⁹-alk, -S-alk-, -alk-O-, -alk-S- or -alk-NR⁹-, where alk denotes C₁₋₈-alkylene; and where
   (a) if Z is -O- or -S-, X is -CR¹¹R¹²-; and
   (b) if X is a bond, Z is C₁₋₈-alkylene, C₂₋₈-alkenylene, -NR⁹-alk-, -alk-NR⁹-, -alk-O-or -alk-S-;
n is 1 or, when X is -O-CO-, is 0 or 1;
m is 0 or 1;
and their salts, preferably their pharmaceutically acceptable salts.

Unless otherwise noted, alkyl and alkoxy radicals refer to C₁₋₈-alkyl and C₁₋₈-alkoxy radicals, preferably to C₁₋₄-alkyl and C₁₋₄-alkoxy radicals. C₁₋₈-alkyl and alkoxy radicals may be linear or branched. Examples of C₁₋₈-alkyl and alkoxy radicals are methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy. C₁₋₈-alkylenedioxy radicals are preferably methylenedioxy, ethylenedioxy and propylenedioxy. Examples of C₁₋₈-alkanoyl radicals are acetyl, propionyl and butyryl. Cycloalkyl is a saturated, cyclic hydrocarbon radical having 3 to 12 carbon atoms, for example cyclopropyl, cyclobutyl, cyclopentyl, cyclohexyl, cycloheptyl, bicyclo[2.2.1]heptyl, cyclooctyl, bicyclo[2.2.2]octyl and adamantyl. Cycloalkyl may be unsubstituted or substituted one or more times, e.g. substituted once or twice by C₁₋₈-alkanoyl, C₂₋₈-alkenyl, C₂₋₈-alkinyl, C₁₋₈-alkoxy, C₁₋₈-alkoxy-C₁₋₈-alkoxy, C₁₋₈-alkoxy-C₁₋₈-alkyl, C₁₋₈-alkoxycarbonylamino, C₁₋₈-alkyl, C₀₋₈-alkylcarbonylamino, C₁₋₈-alkylcarbonyloxy, C₁₋₈-alkylenedioxy, optionally N-mono- or N,N-di-C₁₋₈-alkylated amino, aryl, optionally N-mono- or N,N-di-C₁₋₈-alkylated carbamoyl, optionally esterified carboxy, cyano, C₃₋₈-cycloalkoxy, halogen, heteroaryl, heterocyclyl, hydroxy, oxo, polyhalo-C₁₋₈-alkoxy or polyhalo-C₁₋₈-alkyl. C₁₋₈-Alkylene radicals may be linear or branched and are, for example, methylene, ethylene, propylene, 2-methylpropylene, 2-methylbutylene, 2-methylpropyl-2-ene, butyl-2-ene, butyl-3-ene, propyl-2-ene, tetra-, penta- and hexamethylene; C₂₋₈-alkenylene radicals are, for example, vinylene and propenylene; C₂₋₈alkinylene radicals is, for example, ethinylene; acyl radicals are alkanoyl radicals, preferably C₁₋₈-alkanoyl radicals, or aroyl radicals such as benzoyl. Aryl refers to mono- or polynuclear aromatic radicals which may be substituted one or more times, such as, for example, phenyl, substituted phenyl, naphthyl, substituted naphthyl, tetrahydronaphthyl or substituted tetrahydronaphthyl, preferably phenyl or substituted phenyl. Examples of substituents on such aryl radicals are C₁₋₈-alkyl, trifluoromethyl, nitro, amino, C₂₋₈-alkenyl, C₁₋₈-alkoxy, C₁₋₈-alkylcarbonyloxy, hydroxy, halogen, cyano, carbamoyl, carboxy and C₁₋₈-alkylenedioxy, and optionally halogen-, C₁₋₈-alkyl-, C₁₋₈-alkoxy- or dihydroxy-C₁₋₈-alkylaminocarbonyl-substituted phenyl, phenoxy, phenylthio, phenyl-C₁₋₈-alkyl or phenyl-C₁₋₈-alkoxy. Further examples of substituents on aryl or heterocyclyl radicals are C₁₋₈-alkoxycarbonylphenyl, hydroxy-C₁₋₈-alkylphenyl, benzyloxy, pyridylcarbonylamino-C₁₋₈-alkyl, C₂₋₈-alkenyloxy, C₁₋₈-alkoxy-C₁₋₈-alkoxy, C₁₋₈-alkoxy-C₁₋₈-alkoxy-C₁₋₈-alkyl, methoxybenzyloxy, hydroxybenzyloxy, phenaethyloxy, methylenedioxybenzyloxy, dioxolanyl-C₁₋₈-alkoxy, cyclopropyl-C₁₋₈-alkyl, cyclopropyl-C₁₋₈-alkoxy, hydroxy-C₁₋₈-alkoxy, carbamoyloxy-C₁₋₈-alkoxy, pyridylcarbamoyloxy-C₁₋₈-alkoxy, benzoyloxy-C₁₋₈-alkoxy, C₁₋₈-alkoxycarbonyl, C₀₋₈-alkylcarbonylamino, C₀₋₈-alkylcarbonylamino-C₁₋₈-alkyl, C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₀₋₈-alkylcarbonylamino-C₁₋₈-alkoxy, C₃₋₈-cycloalkylcarbonylamino-C₁₋₈-alkyl, C₃₋₈-cycloalkylcarbonylamino-C₁₋₈-alkoxy, C₁₋₈-alkoxy-C₁₋₈-alkyl, hydroxy-C₁₋₈-alkyl, hydroxy-C₁₋₈-alkoxy-C₁₋₈-alkyl, hydroxy-C₁₋₈-alkoxy-C₁₋₈-alkoxy, C₁₋₈-alkoxycarbonylamino-C₁₋₈-alkyl, C₁₋₈-alkoxycarbonylamino-C₁₋₈-alkoxy, C₁₋₈-alkylaminocarbonylamino-C₁₋₈-alkyl, C₁₋₈-alkylaminocarbonylamino-C₁₋₈-alkoxy, C₁₋₈-alkylaminocarbonyl-C₁₋₈-alkyl, C₁₋₈-alkylaminocarbonyl-C₁₋₈-alkoxy, C₁₋₈-alkylaminocarbonyl-C₁₋₈-alkoxy-C₁₋₈-alkyl, di-C₁₋₈-alkylaminocarbonyl-C₁₋₈-alkyl, di-C₁₋₈-alkylaminocarbonyl-C₁₋₈-alkoxy, C₁₋₈-alkylcarbonyloxy-C₁₋₈-alkyl, C₁₋₈-alkylcarbonyloxy-C₁₋₈-alkoxy, cyano-C₁₋₈-alkyl, cyano-C₁₋₈-alkoxy, 2-oxoxazolidinyl-C₁₋₈-alkyl, 2-oxoxazolidinyl-C₁₋₈-alkoxy, C₁₋₈-alkoxycarbonyl-C₁₋₈-alkyl, C₁₋₈-alkoxycarbonyl-C₁₋₈-alkoxy, C₁₋₈-alkylsulphonylamino-C₁₋₈-alkyl, C₁₋₈-alkylsulphonylamino-C₁₋₈-alkoxy, (N-C₁₋₈-alkyl)-C₁₋₈-alkylsulphonylamino-C₁₋₈-alkyl, (N-C₁₋₈-alkyl)-C₁₋₈-alkylsulphonylamino-C₁₋₈-alkoxy, C₁₋₈-alkylamino-C₁₋₈-alkyl, C₁₋₈-alkylamino-C₂₋₈-alkoxy, di-C₁₋₈-alkylamino-C₁₋₈-alkyl, di-C₁₋₈-alkylamino-C₂₋₈-alkoxy, C₁₋₈-alkylsulphonyl-C₁₋₈-alkyl, C₁₋₈-alkylsulphonyl-C₁₋₈-alkoxy, carboxy-C₁₋₈-alkyl, carboxy-C₁₋₈-alkoxy, carboxy-C₁₋₈-alkoxy-C₁₋₈-alkyl, C₁₋₈-alkoxy-C₁₋₈-alkylcarbonyl, acyl-C₁₋₈-alkoxy-C₁₋₈-alkyl, (N-C₁₋₈-alkyl)-C₁₋₈-alkoxycarbonylamino, (N-hydroxy)-C₁₋₈-alkylaminocarbonyl-C₁₋₈-alkyl, (N-hydroxy)-C₁₋₈-alkylaminocarbonyl-C₁₋₈-alkoxy, (N-hydroxy)aminocarbonyl-C₁₋₈-alkyl, (N-hydroxy)aminocarbonyl-C₁₋₈-alkoxy, C₁₋₈-alkoxyaminocarbonyl-C₁₋₈-alkyl, 6-alkoxyaminocarbonyl-C₁₋₈-alkoxy, (N-C₁₋₈-alkoxy)-C₁₋₈-alkylaminocarbonyl-C₁₋₈-alkyl, (N-C₁₋₈-alkoxy)-C₁₋₈-alkylaminocarbonyl-C₁₋₈-alkoxy, (N-acyl)-C₁₋₈-alkoxy-C₁₋₈-alkylamino, C₁₋₈-alkoxy-C₁₋₈-alkylcarbamoyl, (N-C₁₋₈-alkyl)-C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, (N-C₁₋₈-alkyl)-C₁₋₈-alkoxy-C₁₋₈-alkylcarbonylamino, 1-C₁₋₈-alkoxy-C₁₋₈-alkylimidazol-2-yl, 1-C₁₋₈-alkoxy-C₁₋₈-alkyltetrazol-5-yl, 5-C₁₋₈-alkoxy-C₁₋₈-alkyltetrazol-1-yl, 2-C₁₋₈-alkoxy-C₁₋₈-alkyl-4-oxoimidazol-1-yl, carbamoyl-C₁₋₈-alkyl, carbamoyl-C₁₋₈-alkoxy, C₁₋₈-alkylcarbamoyl, di-C₁₋₈-alkylcarbamoyl, C₁₋₈-alkylsulphonyl, C₁₋₈-alkylamidinyl, acetamidinyl-C₁₋₈-alkyl, O-methyloximyl-C₁₋₈-alkyl, O,N-dimethylhydroxylamino-C₁₋₈-alkyl, C₃₋₈-cycloalkyl-C₁₋₈-alkanoyl, aryl-C₁₋₈-alkanoyl, heterocyclyl-C₁₋₈-alkanoyl; and optionally halogen-, C₁₋₈-alkyl-, C₁₋₈-alkoxy- or dihydroxy-C₁₋₈-alkylaminocarbonyl-substituted pyridyl, pyridyloxy, pyridylthio, pyridylamino, pyridyl-C₁₋₈-alkyl, pyridyl-C₁₋₈-alkoxy, pyrimidinyl, pyrimidinyloxy, pyrimidinylthio, pyrimidinylamino, pyrimidinyl-C₁₋₈-alkyl, pyrimidinyl-C₁₋₈-alkoxy, thienyl, thienyl-C₁₋₈-alkyl, thienyl-C₁₋₈-alkoxy, furyl, furyl-C₁₋₈-alkyl, furyl-C₁₋₈-alkoxy.

The term heterocyclyl refers to mono-, bi- or polycyclic, saturated and unsaturated heterocyclic radicals having 1 to 4 nitrogen and/or 1 or 2 sulphur or oxygen atoms, which may be substituted one or more times, in particular once, twice or three times. The term heterocyclyl further encompasses the above oxo-substituted radicals.

Examples of unsaturated heterocyclyl radicals are benzo[1,3]dioxolyl, benzofuranyl, benzoimidazolyl, benzooxazolyl, benzothiazolyl, benzo[b]thienyl, quinazolinyl, quinolyl, quinoxalinyl, chromenyl, dihydrobenzofuranyl, 1,3-dihydrobenzoimidazolyl, 3,4-dihydro-2H-benzo[1,4]oxazinyl, dihydro-3H-benzo[1,4]oxazinyl, 1,4-dihydrobenzo[d][1,3]oxazinyl, dihydro-2H-benzo[1,4]thiazinyl, 3,4-dihydro-1 H-quinazolinyl, 3,4-dihydro-1 H-quinolinyl, 2,3-dihydroindolyl, dihydro-1H-pyrido[2,3-b][1,4]oxazinyl, 1,1-dioxodihydro-2H-benzo[1,4]thiazinyl, furyl, imidazolyl, imidazo[1,5-a]pyridinyl, imidazo[1,2-a]pyrimidinyl, indazolyl, indolyl, isobenzofuranyl, isoquinolyl, [1,5]naphthyridyl, oxazolyl, 1-oxidopyridyl, 2-oxobenzoimidazolyl, 3-oxo-4H-benzo[1,4]oxazinyl, 2-oxobenzoxazolyl, 3-oxo-4H-benzo[1,4]thiazinyl, 2-oxo-1 H-quinolinyl, 2-oxo-2H-chromenyl, 2-oxodihydrobenzo[e][1,4]-diazepinyl, 2-oxo-1,3-dihydrobenzoimidazole, 2-oxodihydrobenzo[d][1,3]oxazinyl, 2-oxo-3,4-dihydro-1H-quinazolinyl, 2-oxo-3,4-dihydro-1H-quinolinyl, 4-oxo-dihydroimidazolyl, 2-oxo-1,3-dihydroindolyl, 1-oxo-3H-isobenzofuranyl, 2-oxo-1H-pyrido[2,3-b][1,4]oxazinyl, 2-oxo-1,3,4,5-tetrahydrobenzo[b]azepinyl, 2-oxotetrahydrobenzo[e][1,4]diazepinyl, 4-oxo-3H-thieno[2,3-d]pyrimidinyl, 5-oxo-4H-[1,2,4]triazinyl, C₁₋₈alkylenedioxy-substituted phenyl, phthalazinyl, pyranyl, pyrazinyl, pyrazolyl, pyridyl, pyrimidinyl, 1H-pyrrolizinyl, pyrrolo[3,2-c]pyridinyl, pyrrolo[2,3-c]pyridinyl, pyrrolo[3,2-b]pyridinyl, 1 H-pyrrolo[2,3-b]pyridyl, pyrrolyl, 1,3,4,5-tetrahydrobenzo[b]azepinyl, tetrahydroquinolinyl, tetrahydroquinoxalinyl, tetrahydroisoquinolinyl, thiazolyl, thienyl, triazinyl, triazolyl, 1,1,3-trioxodihydro-2H-1λ6-benzo[1,4]thiazinyl, [1,2,3]triazolo[1,5-a]pyridinyl or [1,2,4]triazolo[4,3-a]pyridinyl.

The term saturated heterocyclyl refers to 3-16-membered, mono-, bi- or polycyclic saturated heterocyclic radicals having 1 to 4 nitrogen and/or 1 or 2 sulphur or oxygen atoms. Preference is given to 3-8-membered, particularly preferably 5- or 6-membered, monocyclic radicals which optionally have a 3-8-membered fused-on ring which may be carbocyclic or heterocyclic. A further preferred group of heterocyclic radicals are bi- or polycyclic heterocycles which optionally have a spirocyclic or bridged ring. Preferred heterocyclic radicals have in each ring 1 nitrogen, oxygen or sulphur atom, 1-2 nitrogen atoms and 1-2 oxygen atoms or 1-2 nitrogen atoms and 1-2 sulphur atoms, with at least 1, preferably 1-7, carbon atoms being present in each ring.

Examples of saturated heterocyclyl radicals are azepanyl, azetidinyl, aziridinyl, 3,4-dihydroxypyrrolidinyl, 2,6-dimethylmorpholinyl, 3,5-dimethylmorpholinyl, dioxanyl, [1,4]dioxepanyl, dioxolanyl, 4,4-di-oxothiomorpholinyl, dithianyl, dithiolanyl, 2-hydroxymethylpyrrolidinyl, 4-hydroxypiperidinyl, 3-hydroxypyrrolidinyl, 4-methylpiperazinyl, 1-methylpiperidinyl, 1-methylpyrrolidinyl, morpholinyl, oxathianyl, oxepanyl, 2-oxoazepanyl, 2-oxoimidazolidinyl, 2-oxooxazolidinyl, 2-oxopiperidinyl, 4-oxopiperidinyl, 2-oxopyrrolidinyl, 2-oxotetrahydro-pyrimidinyl, 4-oxothiomorpholinyl, piperazinyl, piperidinyl, pyrrolidinyl, tetrahydrofuranyl, tetrahydropyranyl, tetrahydrothiophenyl, tetrahydrothiopyranyl, thiepanyl or thiomorpholinyl.

Examples of bi- or polycyclic heterocyclyl radicals are 2,5-dioxabicyclo[4.1.0]heptanyl, 2-oxabicyclo[2.2.1]heptanyl, 2-oxabicyclo[4.1.0]heptanyl, 3-oxabicyclo[4.1.0]heptanyl, 7-oxabicyclo[2.2.1]heptanyl, 2-oxabicyclo[3.1.0]hexanyl, 3-oxabicyclo[3.1.0]hexanyl, 1-oxaspiro[2.5]octanyl, 6-oxaspiro[2.5]octanyl, 3-oxabicyclo[3.3.1]nonanyl, 2-oxo-1a,7b-dihydro-1 H-cyclopropa[c]chromenyl or 1,1 a,2,7b-tetrahydrocyclopropa[c]chromenyl.

Heterocyclyl may be unsubstituted or substituted one or more times, e.g. once or twice, by C₁₋₈-alkanoyl, C₂₋₈-alkenyl, C₂₋₈-alkinyl, C₁₋₈-alkoxy, C₁₋₈-alkoxy-C₁₋₈-alkoxy, C₁₋₈-alkoxy-C₁₋₈-alkyl, C₁₋₈-alkoxycarbonylamino, C₁₋₈-alkyl, C₀₋₈-alkylcarbonylamino, C₁₋₈-alkylcarbonyloxy, C₁₋₈-alkylenedioxy, optionally N-mono- or N,N-di-C₁₋₈-alkylated amino, aryl, optionally N-mono- or N,N-di-C₁₋₈-alkylated carbamoyl, optionally esterified carboxy, cyano, C₃₋₈-cycloalkoxy, halogen, heteroaryl, heterocyclyl, hydroxy, nitro, oxide, oxo, polyhalo-C₁₋₈-alkoxy or polyhalo-C₁₋₈-alkyl.

The aryl, aroyl and heterocyclyl radicals in the case of R¹, R⁴, R⁹ and U may additionally be substituted also by heterocyclylalkyl, heterocyclylalkoxy, heterocyclylalkoxyalkyl or heterocyclyl such as, for example, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-yl-alkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1 ,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyl-tetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy, N-methylpiperazinoalkyl, N-methylpiperazinoalkoxy, N-methylpiperazinoalkoxyalkyl, alkylaminoalkyl, alkylaminoalkoxy, alkylaminoalkoxyalkyl, mono- and polyhydroxyalkyl, mono- and polyhydroxyalkoxy, mono- and polyhydroxyalkoxyalkyl and mono- and polyhydroxyalkoxyalkoxy, carbamoylalkyloxy, C₁₋₈-alkoxy, amino-C₁₋₈-alkoxy, hydroxy-C₁₋₈-alkoxy, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₈-alkoxy-C₁₋₈-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxoxazolidinyl, 2-oxopyrrolidinyl, 2-oxo[1,3]oxazinyl, 2-oxo-tetrahydropyrimidinyl and the like or by the radical -O-CH₂CH(OH)CH₂NRx, where NRx is a mono- or di-C₁₋₈-alkylamino, piperidino, morpholino, piperazino or N-methylpiperazino radical.

The term polyhydroxyalkyl refers to C₁₋₈-alkyl radicals which may be substituted by 2-8 hydroxy groups, such as, for example, glyceryl, arabityl, sorbityl etc.

Examples of 5- and 6-membered heterocyclic rings represented by NR⁵R⁶ are pyrrolidinyl, piperidinyl, piperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl and the like.

Examples of 3-8-membered rings represented by CR⁷R⁸ are cyclopentyl, cyclohexyl, cycloheptyl, 1,3-dioxolanyl, 1,3-dioxanyl, 1,3-dithiolanyl and 1,3-dithianyl. Halogen is, for example, fluorine, chlorine, bromine or iodine.

The compounds of the formula (I) and their pharmaceutically acceptable salts have at least two asymmetric carbon atoms, if R⁴ is oxo and at least three asymmetric carbon atoms, if R⁴ is not oxo and may therefore be present in the form of optically pure diastereomers, diastereomer mixtures, diastereomeric racemates, mixtures of diastereomeric racemates or as meso compounds. The invention encompasses all of theses forms. Diastereomer mixtures, diastereomeric racemates or mixtures of diastereomeric racemates may be separated by customary methods, for example by column chromatography, thin-layer chromatography, HPLC and the like.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of the formula (I).

Such salts are formed for example by compounds of the formula (I) having an acidic group, e.g. a carboxy or sulpho group, and are for example their salts with suitable bases, such as non-toxic metal salts derived from metals of group Ia, Ib, IIa and IIb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as optionally hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N.N-di-lower-alkyl-N-(hydroxy-lower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formula I having a basic group, e.g. an amino group, can form acid addition salts, e.g. with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose 6-phosphate, N-cyclohexylsulphamic acid (to form cyclamates) or with other acidic organic compounds such as ascorbic acid. Compounds of the formula (I) having acidic and basic groups may also form inner salts.

Pharmaceutically unsuitable salts may also be used for isolation and purification.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate.

The compounds of the formula (I) also include those compounds in which one or more atoms are replaced by their stable, non-radioactive isotopes; for example, a hydrogen atom by deuterium.

Preferred inventive compounds are those of the general formula (1a) 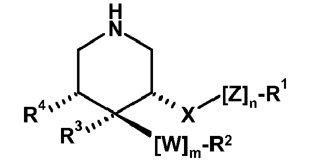 and their pharmaceutically acceptable salts
in which R¹, R², R³, R⁴, W, X and Z, n and m are each as defined above for the compounds of the formula (I).

A further preferred group of compounds of the formula (I), and particularly preferably of the formula (Ia), and their pharmaceutically acceptable salts are compounds in which

R¹ is aryl under the conditions as indicated for (B), (C) or (D), or is heterocyclyl, optionally substituted by oxo or oxide or as indicated under (E) or (F), where heterocyclyl is particularly preferably selected from azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, 1,1-dioxodihydro-2H-benzo[1,4]thiazinyl, indazolyl, indolyl, 3H-isobenzofuranyl, [1,5]naphthyridyl, oxazolyl, 2-oxoazepanyl, 3-oxo-4H-benzo[1,4]oxazinyl, 2-oxobenzoxazolyl, 3-oxo-4H-benzo[1,4]thiazinyl, 2-oxodihydrobenzo[e][1,4]diazepinyl, 2-oxodihydrobenzo[d][1,3]oxazinyl, 2-oxodihydro-1H-quinazolinyl, 4-oxodihydroimidazolyl, 2-oxo-1,3-dihydroindolyl, 1-oxo-3H-isobenzofuranyl, 2-oxopiperidinyl 2-oxo-1H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, piperidinyl, pyrazolyl, 1H-pyrido[2,3-b][1,4]oxazinyl, pyridyl, 1H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydrocyclopropa[c]chromenyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl.

A further preferred group of compounds of the formula (I), or more preferably of the formula (Ia), and their pharmaceutically acceptable salts is that of compounds in which
R¹ is as defined in claim 1 as specified for (A), (B), (C), (D), (E) or (F), more preferably as specified for (A), (C), (E) or (F);
R² is phenyl, pyridyl, cyclohexyl, tetrazolyl, or phenyl, pyridyl, cyclohexyl or tetrazolyl, each of which is substituted by halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₁₋₈-alkylenedioxy, C₂₋₈alkenyloxy-C₁₋₈alkyl, C₁₋₈alkoxy-C₁₋₈alkyl, C₁₋₈alkoxy-C₁₋₈alkylamino-C₁₋₈alkyl, C₁₋₈alkoxy-C₁₋₈alkylsulphanyl-C₁₋₈alkyl, C₁₋₈alkoxy-C₀₋₈alkyl-C₃₋₈cycloalkyl-C₀₋₈alkoxy-C₁₋₈alkyl, C₁₋₈alkylsulphanyl-C₁₋₈alkoxy-C₁₋₈alkyl, C₁₋₈alkylsulphanyl-C₁₋₈alkyl, C₁₋₈alkylsulphonyl-C₁₋₈alkoxy-C₁₋₈alkyl, C₃₋₈cycloalkyl-C₀₋₈alkoxy-C₁₋₈alkoxy-C₁₋₈alkyl, C₃₋₈cycloalkyl-C₀₋₈alkoxy-C₁₋₈alkyl, optionally halogen-substituted C₁₋₈alkoxy-C₁₋₈alkoxy-C₁₋₈alkyl, or heterocyclyl-C₀₋₈alkoxy-C₁₋₈alkyl or by an L1-T1-L2-T2-L3-T3-L4-T4-L5-U radical; or naphthyl or acenaphthyl;
L1, L2, L3, L4 and L5 are each independently a bond, C₁₋₈-alkylene, C₂₋₈-alkenylene or C₂₋₈-alkynylene, or are absent;
T1, T2, T3 and T4 are each independently
   (a) a bond, or are absent, or are one of the groups
   (b) -CH(OH)-
   (c) -CH(OR⁶)-
   (d) -CH(NR⁵R⁶)-
   (e) -CO-
   (f) -CR⁷R⁸-
   (g) -O- or -NR⁶-
   (h) -S(O)₀₋₂-
   (i) -SO₂NR⁶-
   (j) -NR⁶SO₂-
   (k) -CONR⁶-
   (l) -NR⁶CO-
   (m) -O-CO-
   (n) -CO-O-
   (o) -O-CO-O-
   (p) -O-CO-NR⁶-
   (q) -N(R⁶)-CO-N(R⁶)-
   (r) -N(R⁶)-CO-O-
   (s) pyrrolidinylene, piperidinylene or piperazinylene
   (t) -C(R¹¹)(R¹²)-,
   where the bonds starting from (b)-(t) lead to a saturated or aromatic carbon atom of the adjacent group if the bond starts from a heteroatom, and where not more than two groups (b)-(f), three groups (g)-(h) and one group (i)-(t) is/are present;
R³ is hydrogen, hydroxyl, C₁₋₈-alkoxy or C₁₋₈-alkenyloxy;
R⁴ is optionally halogen- and/or hydroxy-substituted C₁₋₈alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₀₋₈-alkylcarbonyl-C₀₋₈-alkyl, C₁₋₈-alkoxy-C₀₋₈-alkylcarbonyl-C₀₋₈-alkyl, optionally N-C₁₋₈-alkylated C₁₋₈-alkoxycarbonylamino-C₁₋₈-alkyl, optionally N-C₁₋₈-alkylated C₁₋₈-alkoxy-C₁₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₁₋₈-alkylamino-C₁₋₈-alkyl, optionally N-C₁₋₈-alkylated or optionally halogen-substituted heterocyclyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocyclyl-C₀₋₈-alkylamino-C₀₋₈-alkylcarbonyl-C₀₋₈-alkyl, C₁₋₈-alkylsulphonyl-C₁₋₈-alkyl, C₂₋₈-alkinyl, heterocyclyl-C₂₋₈-alkinyl, optionally N-mono- or N,N-di-C₁₋₈-alkylated amino-C₂₋₈-alkinyl, heterocyclylcarbonyl-C₀₋₈-alkyl, heterocyclyloxy-C₁₋₈-alkyl, optionally N-mono- or N,N-di-C₁₋₈-alkylated amino, C₁₋₈-alkylcarbonyl-C₁₋₈-alkoxy, C₁₋₈-alkylcarbonyloxy, aryl-C₁₋₈-alkoxy, aryloxy, optionally N-mono- or N,N-di-C₃₋₈-cycloalkyl-C₁-C₆-alkylated carbamoyl-C₁₋₈-alkoxy, optionally N-mono- or N.N-di-C₁-C₆-alkylated carbamoyloxy, hydroxyl, hydroxy-C₁₋₈-alkoxy, hydroxy-C₁₋₈-alkoxy-C₁₋₈-alkoxy, optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₁₋₈-alkoxy, optionally N-mono- or N,N-di-C₁₋₈-akylated or optionally hydroxy-substituted amino-C₀₋₈-alkylcarbonyl-C₁₋₈-alkoxy, hydroxy-C₀₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₋₈-alkoxycarbonylamino-C₁₋₈-alkoxy, optionally N-C₁₋₈-alkylated C₁₋₈-alkoxy-C₁₋₈-alkylamino-C₁₋₈-alkoxy, optionally N-C₁₋₈-alkylated or optionally halogen-substituted C₁₋₈-alkylcarbonylamino-C₁₋₈-alkoxy, cyano-C₁₋₈-alkoxy, optionally N-C₁₋₈-alkylated or optionally halogen-substituted C₃₋₈-cycloalkyl-C₀₋₈-alkylcarbonylamino-C₁₋₈-alkoxy, optionally N-C₁₋₈-alkylated hydroxy-C₁₋₈-alkylamino-C₁₋₈-alkoxy, optionally N-C₁₋₈-alkylated or optionally halogen-substituted heterocyclyl-C₀₋₈-alkylcarbonylamino-C₁₋₈-alkoxy, C₃₋₈-cycloalkyl-C₀₋₈-alkoxy, C₃₋₈-cycloalkyloxy-C₁₋₈-alkoxy, heterocyclyl-C₀₋₈-(optionally hydroxy-substituted)alkoxy, optionally N-C₁₋₈-alkylated heterocyclyl-C₀₋₈-alkylamino-C₀₋₈-alkylcarbonyl-C₀₋₈-alkoxy, C₁₋₈-alkylsulphonyl-C₁₋₈-alkoxy, C₂₋₈-alkinyl-oxy, heterocyclyl-C₂₋₈-alkinyl-oxy, optionally N-mono- or N,N-di-C₁₋₈-alkylated amino-C₂₋₈-alkinyl-oxy, heterocyclylcarbonyl-C₀₋₈-alkoxy, heterocyclyloxy-C₁₋₈-alkyoxy or oxo;
R⁵ and R⁶ are each independently hydrogen, C₁₋₈-alkyl or acyl, or, together with the N atom to which they are bonded, are a 5- or 6-membered heterocyclic ring which may contain an additional N, O or S atom;
R⁷ and R⁸, together with the carbon atom to which they are bonded, are a 3-8-membered ring which may contain one or two -O- or -S- atoms;
R⁹ is hydrogen, C₁₋₈-alkyl, acyl, arylalkyl, C₃₋₈-cycloalkyl or C₃₋₈-cycloalkyl-C₁₋₈-alkyl;
R¹¹ is hydrogen or C₁₋₈-alkyl;
R¹² is hydrogen or C₁₋₈-alkyl;
R¹¹ and R¹², together with the C-atom to which they are attached, may also be C₃₋₈-cycloalkyl;
U is hydrogen, C₁₋₈-alkyl, C₃₋₁₂-cycloalkyl, cyano, aryl or heterocyclyl;
X is oxygen, sulphur or a -CR¹¹R¹²-, -CHOR⁹-, -O-CO-, -CO-, -O-CR¹¹R¹²-, -O-CR¹¹R¹²-CO-NR⁹- or -CO-NR⁹- group;
W is oxygen or sulphur;
Z is C₁₋₈-alkylene, O or -alk-O- , where alk denotes C₁₋₈-alkylene;
n is 1 or, when X is -O-CO-, is 0 or 1;
m is 0 or also, when R³ is hydrogen, is 1;
and pharmaceutically acceptable salts thereof.

Preference is further given to compounds of the formulae (I) and (Ia) in which W is absent (m is 0). X is preferably oxygen, sulphur, -O-CHR¹¹-, -O-CHR¹¹-CO-NR⁹- or -CO-. Z is preferably methylene or -alk-O-

A group of preferred R¹ radicals includes the abovementioned substituted phenyl and naphthyl radicals, and also tetrahydronaphthyl and methyl-substituted tetrahydronaphthyl.

Likewise preferred radicals R¹ are azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, 1,1-dioxodihydro-2H-benzo[1,4]thiazinyl,indazolyl, indolyl, 3H-isobenzofuranyl, [1,5]naphthyridyl, oxazolyl, phthalazinyl, piperidinyl, pyrazolyl, 1H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydrocyclopropa[c]chromenyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl and azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, 1,1-dioxodihydro-2H-benzo[1,4]thiazinyl, indazolyl, indolyl, 3H-isobenzofuranyl, [1,5]naphthyridyl, oxazolyl, phthalazinyl, piperidinyl, pyrazolyl, 1 H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydrocyclopropa[c]chromenyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl, each of which is substituted by 1-3 acetamidinyl-C₁₋₈-alkyl, 3-acetamidomethylpyrrolidinyl, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkanoyloxy, C₂₋₈-alkenyl, C₂₋₈-alkenyloxy, C₂₋₈-alkenyloxy-C₁₋₈-alkyl, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, 1-C₁₋₈-alkoxy-C₁₋₈-alkylimidazol-2-yl, 2-C₁₋₈-alkoxy-C₁₋₈-alkyl-4-oxo-imidazol-1-yl, 3-C₁₋₈-alkoxy-C₁₋₈-alkylpyrrolidinyl, 1-C₁₋₈-alkoxy-C₁₋₈-alkyltetrazol-5-yl, 5-C₁₋₈-alkoxy-C₁₋₈-alkyltetrazol-1-yl, C₁₋₈-alkoxyaminocarbonyl-C₁₋₈-alkoxy, C₁₋₈-alkoxyaminocarbonyl-C₁₋₈-alkyl, C₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino, C₁₋₈-alkoxycarbonylamino-C₁₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkylcarbonylamino, (N-C₁₋₈-alkyl)-C₁₋₈-alkoxycarbonylamino, (N-C₁₋₈-alkyl)-C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylamidinyl, C₁₋₈-alkylamino, Di-C₁₋₈-alkylamino, C₁₋₈-alkylamino-C₂₋₈-alkoxy, di-C₁₋₈-alkylamino-C₂₋₈-alkoxy, C₁₋₈-alkylamino-C₁₋₈-alkyl, C₁₋₈-alkylaminocarbonyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, C₁₋₈-alkylaminocarbonylamino-C₁₋₈-alkoxy, C₁₋₈-alkylaminocarbonylamino-C₁₋₈-alkyl, di-C₁₋₈-alkylaminocarbonyl-C₁₋₈-alkyl, di-C₁₋₈-alkylamino-C₁₋₈-alkyl, C₁₋₈-alkylcarbamoyl, di-C₁₋₈-alkylcarbamoyl, C₀₋₈-alkylcarbonylamino, C₀₋₈-alkylcarbonylamino-C₁₋₈-alkoxy, C₀₋₈-alkylcarbonylamino-C₁₋₈-alkyl, C₁₋₈-alkylcarbonyloxy-C₁₋₈-alkoxy, C₁₋₈-alkylcarbonyloxy-C₁₋₈-alkyl, C₁₋₈-alkylendioxy, C₁₋₈-alkylsulphonyl, C₁₋₈-alkylsulphonyl-C₁₋₈-alkoxy, C₁₋₈-alkylsulphonyl-C₁₋₈-alkyl, C₁₋₈-alkylsulphonylamino-C₁₋₈-alkoxy, C₁₋₈-alkylsulphonylamino-C₁₋₈-alkyl, amino, amino-C₂₋₇-alkoxy, amino-C₁₋₈-alkyl, aryl-C₁₋₈-alkanoyl, benzoyloxy-C₂₋₈-alkoxy, carbamoyl, carbamoyl-C₁₋₈-alkoxy, carbamoyl-C₁₋₈-alkyl, carboxy, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cycloalkyl-C₁₋₈-alkanoyl, C₃₋₈-cyclo-alkyl-C₀₋₈-alkoxy, C₃₋₈-cyclo-alkyl-C₀₋₈-alkyl, C₃₋₈-cycloalkylcarbonylamino, C₃₋₈-cycloalkylcarbonylamino-C₁₋₈-alkoxy, C₃₋₈-cydoalkylcarbonylamino-C₁₋₈-alkyl, 3,4-dihydroxypyrrolidinyl, O,N-dimethylhydroxylamino-C₁₋₈-alkyl, 2,6-dimethylmorpholinyl, 3,5-dimethylmorpholinyl, dioxanyl, dioxolanyl, dioxolanyl-C₁₋₈-alkoxy, 4,4-dioxothiomorpholinyl, dithianyl, dithiolanyl, optionally C₁₋₈-alkoxy, C₁₋₈-alkyl, dihydroxy-C₁₋₈-alkylaminocarbonyl or halogen-substituted furyl, furyl-C₁₋₈-alkoxy, furyl-C₁₋₈-alkyl, pyridyl, pyridyl-C₁₋₈-alkoxy, pyridyl-C₁₋₈-alkyl, pyridylamino, pyridyloxy, pyridylthio, pyrimidinyl, pyrimidinyl-C₁₋₈-alkoxy, pyrimidinyl-C₁₋₈-alkyl, pyrimidinyl-amino, pyrimidinyloxy, pyrimidinylthio, thienyl, thienyl-C₁₋₈-alkoxy or thienyl-C₁₋₈-alkyl, halogen, heterocyclyl-C₁₋₈-alkanoyl, hydroxy, hydroxy-C₂₋₈-alkoxy, hydroxy-C₂₋₈-alkoxy-C₁₋₈-alkoxy, hydroxy-C₂₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, (N-hydroxy)aminocarbonyl-C₁₋₈-alkoxy, (N-hydroxy)aminocarbonyl-C₁₋₈-alkyl, hydroxybenzyloxy, 2-hydroxymethylpyrrolidinyl, 4-hydroxypiperidinyl, 3-hydroxypyrrolidinyl, imidazolyl-C₁₋₈-alkoxy, imidazolyl-C₁₋₈-alkyl, methoxybenzyloxy, methylenedioxybenzyloxy, 2-methylimidazolyl-C₁₋₈-alkoxy, 2-methylimidazolyl-C₁₋₈-alkyl, 3-methyl-[1,2,4]-oxadiazol-5-yl-C₁₋₈-alkoxy, 5-methyl-[1,2,4]-oxadiazol-3-yl-C₁₋₈-alkoxy, 3-methyl-[1,2,4]-oxadiazol-5-yl-C₁₋₈-alkyl, 5-methyl-[1 ,2,4]-oxadiazol-3-yl-C₁₋₈-alkyl, O-methyloximyl-C₁₋₈-alkyl, 4-methylpiperazinyl, N-methylpiperazino-C₁₋₈-alkoxy, N-methylpiperazino-C₁₋₈-alkoxy-C₁₋₈-alkyl, N-methylpiperazino-C₁₋₈-alkyl, 5-methyltetrazol-1-yl-C₁₋₈-alkoxy, 5-methyltetrazol-1-yl-C₁₋₈-alkyl, morpholinyl, morpholino-C₁₋₈-alkoxy, morpholino-C₁₋₈-alkoxy-C₁₋₈-alkyl, morpholino-C₁₋₈-alkyl, nitro, [1,2,4]-oxadiazol-5-yl-C₁₋₈-alkoxy, [1,2,4]-oxadiazol-5-yl-C₁₋₈-alkyl, oxazol-4-yl-C₁₋₈-alkoxy, oxazol-4-yl-C₁₋₈-alkyl, oxide, oxo, 2-oxoimidazolidinyl, 2-oxo[1,3]oxazinyl, 2-oxoxazolidinyl, 2-oxoxazolidinyl-C₁₋₈-alkoxy, 2-oxoxazolidinyl-C₁₋₈-alkyl, 4-oxopiperidinyl, 2-oxopyrrolidinyl, 2-oxopyrrolidinyl-C₁₋₈-alkoxy, 2-oxopyrrolidinyl-C₁₋₈-alkyl, 2-oxotetrahydropyrimidinyl, 4-oxo-thiomorpholinyl, optionally C₁₋₈-alkoxy-, C₁₋₈-alkoxycarbonyl-, C₁₋₈-alkyl-, C₁₋₈-alkylamino-, di-C₁₋₈-alkylamino-, halogen-, hydroxy-, hydroxy-C₁₋₈-alkyl- or trifluoromethyl-substituted phenoxy, phenyl, phenyl-C₁₋₈-alkoxy, phenyl-C₁₋₈-alkyl or phenylthio, piperazinyl, piperazino-C₁₋₈-alkoxy, piperazino-C₁₋₈-alkoxy-C₁₋₈-alkyl, piperazino-C₁₋₈-alkyl, piperidinyl, piperidino-C₁₋₈-alkoxy, piperidino-C₁₋₈-alkoxy-C₁₋₈-alkyl, polyhalo-C₁₋₈-alkoxy, polyhalo-C₁₋₈-alkyl, pyridylcarbamoyloxy-C₁₋₈-alkoxy, pyridylcarbonylamino-C₁₋₈-alkyl, pyrrolidinyl, pyrrolyl, tetrazol-1-yl-C₁₋₈-alkoxy, tetrazol-2-yl-C₁₋₈-alkoxy, tetrazol-5-yl-C₁₋₈-alkoxy, tetrazol-1-yl-C₁₋₈-alkyl, tetrazol-2-yl-C₁₋₈-alkyl, tetrazol-5-yl-C₁₋₈-alkyl, thiazol-4-yl-C₁₋₈-alkoxy, thiazol-4-yl-C₁₋₈-alkyl, thiomorpholinyl, [1,2,4]-triazol-1-yl-C₁₋₈-alkoxy, [1,2,4]-triazol-4-yl-C₁₋₈-alkoxy, [1,2,4]-triazol-1-yl-C₁₋₈-alkyl, [1,2,4]-triazol-4-yl-C₁₋₈-alkyl and the radical -O-CH₂CH(OH)CH₂NRx, where NRx is a mono- or di-C₁₋₈-alkylamino, N-methylpiperazino, morpholino, piperazino or piperidino radical.

R¹ is very particularly preferably optionally substituted benzimidazolyl or a substituted radical selected from chromenyl, 3,4-dihydro-2H-benzo[1,4]oxazinyl, 1a,7b-dihydro-1H-cyclopropa[c]-chromenyl, indazolyl, indolyl, phenyl and 1,1a,2,7b-tetrahydrocyclopropa[c]chromenyl.

Preferrred R² radicals are phenyl or pyridyl, or phenyl or pyridyl, each of which is substituted by halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₁₋₈-alkylenedioxy, C₂₋₈-alkenyloxy-C₁₋₈-alkyl, C₁₋₈-alkoxy-C₁₋₈-alkylamino-C₁₋₈-alkyl, C₁₋₈-alkoxy-C₁₋₈-alkylsulfanyl-C₁₋₈-alkyl, C₁₋₈-alkoxy-C₃₋₈-Cycloalkyl-C₀₋₈-alkoxy-C₁₋₈-alkyl, C₁₋₈-alkylsulfanyl-C₁₋₈-alkoxy-C₁₋₈-alkyl, C₁₋₈-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, optionally halogen-substituted C₁₋₈-alkoxy-C₁₋₈-alkoxy-C₁₋₈-alkyl or heterocylyl-C₀₋₈-alkoxy-C₁₋₈-alkyl.

R² radicals which are likewise preferred are phenyl or pyridyl, each of which is substituted by an L1-T1-L2-T2-L3-T3-L4-T4-L5-U radical, where L1 and L2 are preferably absent or are C₁₋₈-alkylene, and L3 is absent, and U is hydrogen, C₁₋₈-alkyl, cyclo-C₃₋₈-alkyl, phenylpiperidinyl, phenylpiperazinyl, phenylpyrrolidinyl, phenyl, phenyl which is substituted by C₁₋₈-alkyl, C₁₋₈-alkoxy, C₁₋₈-alkylthio, C₁₋₈-alkylsulphinyl, C₁₋₈-alkylenedioxy, halogen, benzoyl-C₁₋₈-alkyl, halogen-C₁₋₈-alkyl, C₁₋₈-alkanoyloxy or hydroxyl; or naphthyl; or pyridyl, thienyl, pyrazinyl, triazolyl, imidazolyl, phenyloxadiazolyl, thienyloxadiazolyl, furyloxadiazolyl, phenyloxazolyl, benzothiazolyl, furyl, pyrimidinyl, nitrobenzothiazolyl, phenyltetrazolyl, piperidinyl, tetrahydropyranyl or morpholinyl.

In the case of the T1-T4 groups, preference is given to the definitions (a)-(c), (e)-(h), (k)-(n) and (r)-(t).

Examples of particularly preferred R² radicals are phenyl, or phenyl substituted by
2-benzothiazolylthio-C₁₋₈-alkyl,
2-benzyloxy-3-methoxypropoxy,
2-benzoyloxy-3-methoxypropoxy,
2,3-dihydroxypropoxy,
2-hydroxy-3-benzylaminopropoxy,
2-hydroxy-3-phenoxypropoxy,
2-hydroxy-3-phenylthiopropoxy,
2-methoxy-3-phenoxypropoxy,
2-methoxy-3-benzyloxypropoxy,
2-methyl-3-fluorophenylbutyryloxy-C₁₋₈-alkoxy,
2-methyl-3-phenoxypropoxy,
2-C₁₋₈-alkenyloxy-4-phenylbutyl,
3,4,5-trimethoxyphenyloxadiazolyl-C₁₋₈-alkoxy,
6-nitro-2-benzothiazolylthio-C₁₋₈-alkyl,
adamantyloxy-C₁₋₈-alkoxy,
adamantyl-C₁₋₈-alkoxy-C₁₋₈-alkoxy,
benzamido-C₁₋₈-alkoxy,
benzamido-C₁₋₈-alkyl,
benzo[1,3]dioxolyloxy-C₁₋₈-alkoxy,
benzoyl-C₁₋₈-alkoxy and ketals thereof,
benzoyl-C₁₋₈-alkyl and ketals thereof,
benzoyl-C₁₋₈-alkylaminocarbonyl-C₁₋₈-alkyl,
benzoyl-C₁₋₈-alkoxycarbonyl-C₁₋₈-alkyl,
benzoyl-C₁₋₈-alkylaminocarbonyl,
benzoyloxy,
benzoyloxy-C₁₋₈-alkylbenzoyloxy-C₁₋₈-alkoxy,
benzoyloxy-C₁₋₈-alkoxy,
benzoyloxy-C₁₋₈-alkyl,
benzothiazolylthio-C₁₋₈-alkoxy,
benzothiazolylthio-C₁₋₈-alkyl,
benzylcarbamoyl-C₁₋₈-alkoxy,
benzyloxy-C₁₋₈-alkoxycarbonyloxy-C₁₋₈-alkyl,
benzyloxy-C₁₋₈-alkoxy,
benzylthio-C₁₋₈-alkoxy,
bicyclooxy-C₁₋₈-alkoxy,
bicyclo-C₁₋₈-alkoxy-C₁₋₈-alkoxy,
carbamoyloxy-C₁₋₈-alkoxy,
carbamoyloxy-C₁₋₈-alkyl,
carboxy-C₁₋₈-alkoxy,
carboxy-C₁₋₈-alkyl,
cyano,
cyano-C₁₋₈-alkoxy,
cyano-C₁₋₈-alkyl,
cyanophenyl-C₁₋₈-alkoxy,
cydohexylcarbonyloxy-C₁₋₈-alkyl,
cydohexyloxy-C₁₋₈-alkoxy,
cydopropylcarbonyloxy-C₁₋₈-alkyl,
dioxolanyl-C₁₋₈-alkoxy,
furyloxadiazolyl-C₁₋₈-alkoxy,
furoyloxy-C₁₋₈-alkoxy,
halophenoxy-C₁₋₈-alkyl,
halobenzoyl-C₁₋₈-alkoxy,
halobenzoyloxy-C₁₋₈-alkyl,
halobenzoyloxy-C₁₋₈-alkoxy,
halobenzyloxy-C₁₋₈-alkoxy,
halogen,
halo-C₁₋₈-alkyl,
halophenoxy,
halophenoxy-C₁₋₈-alkoxy,
halophenoxy-C₁₋₈-alkoxy-C₁₋₈-alkyl,
halophenyl-C₁₋₈-alkoxy-C₁₋₈-alkyl,
halophenyloxadiazolyl-C₁₋₈-alkoxy,
N-halophenylpyrrolidin-3-yloxy,
hydroxyl,
hydroxybenzoyloxy-C₁₋₈-alkyl,
hydroxybenzoyloxy-C₁₋₈-alkoxy,
hydroxy-C₁₋₈-alkoxy,
hydroxy-C₁₋₈-alkyl,
imidazolylcarbonyloxy-C₁₋₈-alkyl,
methoxybenzoyl-C₁₋₈-alkyl,
methoxybenzyloxy-C₁₋₈-alkoxy,
methylenedioxybenzoyl-C₁₋₈-alkoxy,
morpholino-C₁₋₈-alkoxy,
morpholinocarbonyloxy-C₁₋₈-alkoxy,
morpholinocarbonyloxy-C₁₋₈-alkyl,
N-methylaminophenylcarbonyloxy-C₁₋₈-alkyl,
N-methylbenzylamino-C₁₋₈-alkoxy,
1-methylcyclohexyloxy-C₁₋₈-alkoxy,
1-methylcyclohexyl-C₁₋₈-alkoxy-C₁₋₈-alkoxy,
N-methylpyrrolylcarbonyloxy-C₁₋₈-alkoxy,
4-methyltetrahydropyran-4-yloxy-C₁₋₈-alkoxy,
4-methyltetrahydropyran-4-yl-C₁₋₈-alkoxy-C₁₋₈-alkoxy,
N-C₁₋₈-alkylbenzamido-C₁₋₈-alkyl,
naphthyl-C₁₋₈-alkoxy,
nicotinoyloxy-C₁₋₈-alkoxy,
nicotinoyloxy-C₁₋₈-alkyl,
C₁₋₈-alkanoylbenzoyloxy-C₁₋₈-alkyl,
C₁₋₈-alkanoyloxy-C₁₋₈-alkoxy,
C₁₋₈-alkanoyloxy-C₁₋₈-alkyl,
C₂₋₈-alkenylbenzyloxy-C₁₋₈-alkoxy,
C₂₋₈-alkenyloxy,
C₂₋₈-alkenyloxybenzyloxy-C₁₋₈-alkoxy,
C₁₋₈-alkoxy,
C₁₋₈-alkoxybenzoyloxy-C₁₋₈-alkyl,
C₁₋₈-alkoxycarbonyl,
C₁₋₈-alkoxy-C₁₋₈-alkyl,
C₁₋₈-alkoxybenzoylamino-C₁₋₈-alkyl,
C₁₋₈-alkoxybenzylcarbonyloxy-C₁₋₈-alkyl,
C₁₋₈-alkoxybenzyloxy-C₁₋₈-alkoxy,
C₁₋₈-alkoxybenzylthio-C₁₋₈-alkoxy,
C₁₋₈-alkoxycarbonyl-C₁₋₈-alkoxy,
C₁₋₈-alkoxycarbonyl-C₁₋₈-alkyl,
C₁₋₈-alkoxyphenyloxadiazolyl-C₁₋₈-alkoxy,
C₁₋₈-alkoxyphenyloxy-C₁₋₈-alkoxy,
C₁₋₈-alkyl,
C₁₋₈-alkylbenzyloxy-C₁₋₈-alkoxy,
C₁₋₈-alkylphenoxy-C₁₋₈-alkoxy,
C₁₋₈-alkylenedioxy,
C₁₋₈-alkylenedioxybenzyloxy-C₁₋₈-alkoxy,
C₁₋₈-alkylsulphonylbenzoyl-C₁₋₈-alkoxy,
C₁₋₈-alkylthiobenzoyloxy-C₁₋₈-alkoxy,
C₁₋₈-alkylthiobenzyloxy-C₁₋₈-alkoxy,
benzoyloxybenzyl-C₁₋₈-alkoxy,
hydroxybenzyl-C₁₋₈-alkoxy,
C₁₋₈-alkoxybenzyl-C₁₋₈-alkoxy,
C₁₋₈-alkoxybenzylcarbonyloxy-C₁₋₈-alkoxy,
phenoxybenzyloxy-C₁₋₈-alkoxy,
phenoxycarbonyl-C₁₋₈-alkyl,
phenoxy-C₂₋₈-alkenyloxy,
phenoxy-C₂₋₈-alkynyloxy,
phenyl-C₁₋₈-alkanoylamino-C₁₋₈-alkyl,
phenyl-C₂₋₈-alkenyloxy,
phenyl-C₁₋₈-alkoxy,
phenyl-C₁₋₈-alkyl,
phenyl-C₁₋₈-alkylaminocarbonyl,
phenyl-C₁₋₈-alkylcarbonyl-C₁₋₈-alkoxy,
phenyl-C₁₋₈-alkylaminocarbonyl-C₁₋₈-alkyl,
phenylaminocarbonyloxy-C₁₋₈-alkoxy,
phenylaminocarbonyloxy-C₁₋₈-alkyl,
phenylhydroxy-C₁₋₈-alkyl,
phenyloxadiazolyl-C₁₋₈-alkoxy,
phenyloxadiazolyl-C₁₋₈-alkyl,
phenyloxazolyl-C₁₋₈-alkoxy,
phenyloxy-C₁₋₈-alkoxy,
phenylsulphamoyl-C₁₋₈-alkyl,
phenylsulphinyl-C₁₋₈-alkyl,
phenylsulphonyl-C₁₋₈-alkoxy,
phenylsulphonyl-C₁₋₈-alkyl,
phenyltetrazolylthio-C₁₋₈-alkyl,
phenylthio-C₁₋₈-alkoxy,
phenylthio-C₁₋₈-alkyl,
pyrazinylcarbonyloxy-C₁₋₈-alkyl,
pyridylaminocarbonyloxy-C₁₋₈-alkoxy,
pyridylaminocarbonyloxy-C₁₋₈-alkyl,
pyridylcarbamoyloxy,
pyridyl-C₁₋₈-alkoxy-C₁₋₈-alkoxy,
pyridyl-C₁₋₈-alkoxy-C₁₋₈-alkyl,
pyridyloxadiazolyl-C₁₋₈-alkoxy,
pyridylthio-C₁₋₈-alkyl,
pyrimidinyloxy-C₁₋₈-alkoxy,
pyrimidinylthio-C₁₋₈-alkyl,
thienoyloxy-C₁₋₈-alkoxy,
thienoyloxy-C₁₋₈-alkyl,
thienyloxadiazolyl-C₁₋₈-alkoxy,
triazolyl-C₁₋₈-alkoxy,
trifluoromethylbenzyloxy-C₁₋₈-alkoxy, or
trifluoromethyl.

Examples of very particularly preferred R² radicals are phenyl substituted by
adamantyloxy-C₁₋₈-alkoxy,
adamantyl-C₁₋₈-alkoxy-C₁₋₈-alkoxy,
bicyclooxy-C₁₋₈-alkoxy,
bicyclo-C₁₋₈-alkoxy-C₁₋₈-alkoxy,
halobenzyloxy-C₁₋₈-alkoxy,
halophenoxy-C₁₋₈-alkoxy,
halophenoxy-C₁₋₈-alkoxy-C₁₋₈-alkyl,
halophenyl-C₁₋₈-alkoxy-C₁₋₈-alkyl,
N-halophenylpyrrolidin-3-yloxy,
1-methylcyclohexyloxy-C₁₋₈-alkoxy,
1-methylcyclohexyl-C₁₋₈-alkoxy-C₁₋₈-alkoxy,
4-methyltetrahydropyran-4-yloxy-C₁₋₈-alkoxy,
4-methyltetrahydropyran-4-yl-C₁₋₈-alkoxy-C₁₋₈-alkoxy,
C₁₋₈-alkoxy,
C₁₋₈-alkoxybenzyloxy-C₁₋₈-alkoxy,
C₁₋₈-alkylbenzyloxy-C₁₋₈-alkoxy or
C₁₋₈-alkylphenoxy-C₁₋₈-alkoxy.

A group of very particularly preferred R² radicals are the above mentioned substituted phenyl radicals, where a substituent is present in para-position to the position where the rest of the molecule is attached.

The above-specified compound groups are not to be regarded as closed, but rather it is possible in a sensible manner, for example in order to replace general by more specific definitions, to exchange parts of these compound groups with one another or for the definitions given or to omit them.

The compounds of the formula (I) and their pharmaceutically acceptable salts may be prepared in an analogous manner to the preparation processes known from the literature. Similar preparation processes are described, for example, in WO 97/09311. Details of the specific preparation variants can be taken from the examples.

Depending on the existance of asymmetric carbon atoms, the compounds of this invention may therefore be present in the form of isomeric mixtures, particularly as racemate, or in form of pure isomers, particularly as optical antipodes.

The compounds of the formula (I) and their pharmaceutically acceptable salts can also be prepared in optically pure form. Separation into antipodes can take place by methods known per se, either preferably at an early stage in the synthesis by salt formation with an optically active acid such as, for example, (+)- or (-)-mandelic acid and separation of the diastereomeric salts by fractional crystallization or preferably at a rather late stage by derivatizing with a chiral auxiliary component such as, for example, (+)- or (-)-camphanoyl chloride, and separation of the diastereomeric products by chromatography and/or crystallization and subsequent cleavage of the linkage to the chiral auxiliary. The pure diastereomeric salts and derivatives can be analysed to determine the absolute configuration of the contained piperidine by conventional spectroscopic methods, with X-ray spectroscopy on single crystals representing a particularly suitable method.

The compounds of formula (I) and (Ia), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 µl buffer, 10 µl inhibitor in DMSO, 10 µl peptide substrate in DMSO and 20 µl enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the betasectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

### In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) or preferred formula (1a) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (Ia), respectively, and their pharmaceutically useful salts for the prevention, delay of progression or the treatment of Alzheimer Disease, malaria or HIV infection.
Subject of the present invention is also the use of the compounds of formula (I) and (Ia), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formula (Ia) or a pharmaceutically acceptable salt thereof is applied.
Subject of the present invention is also a pharmaceutical preparation that contains for the inhibition of beta-secretase, cathepsin D, plasmepsin and/or HIV-protease a compound of the general formula (I), or preferred of formula (Ia) or a pharmaceutically acceptable salt thereof as well as commonly used ingredients.
Subject of the present invention is also a pharmaceutical preparation for the prevention, delay of progression or treatment of Alzheimer Disease, malaria and HIV infection that contains a compound of the general formula (I), or preferred of formula (Ia) or a pharmaceutically acceptable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is determined in the solvent system A. The ratio of solvents relative to one another is always reported in parts by volume. Chemical names for end products and intermediates were generated with the aid of the program AutoNom 2000 (automatic nomenclature). Unless stated otherwise, the absolute stereochemistry of the 3-hydroxy(or alkoxy)-4-phenyl-5-alkoxypiperidine unit is (3S,4S,5R) (or 3S,4R,5R, depending on the 3-alkoxy group).

| **Nro** | **Structure** | **Appearance** | **R_{f} (System)** | **Rt (Method)** |
|---|---|---|---|---|
| 1 | 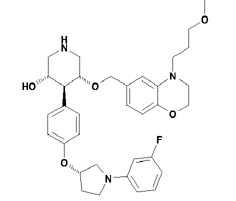 | colourless solid | 0.29 (A) | 4.28 (I) |
| 2 | 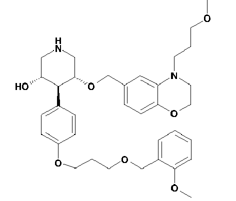 | colourless oil | 0.15 (A) | 4.14 (I) |
| 3 | 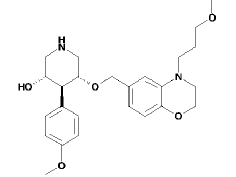 | yellowish oil | 0.05 (C) | 3.29 (I) |
| 4 | 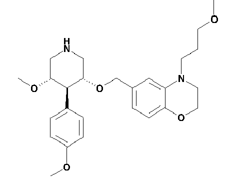 | yellowish oil | 0.15 (C) | 3.63 (I) |
| 5 | 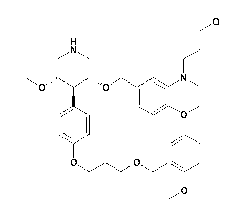 | orange oil | 0.55 (A) | 4.43 (I) |
| 6 | 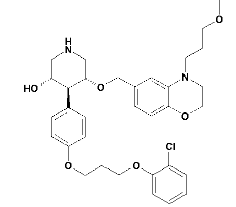 | orange oil | 0.06 (C) | 4.49 (I) |
| 7 | 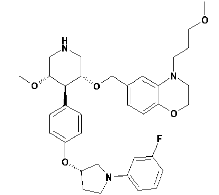 | colourless oil I | 0.39 (A) | 4.56 (I) |
| 8 | 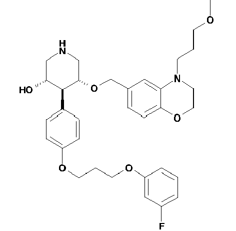 | orange oil | 0.20 (C) | 4.40 (I) |
| 9 | 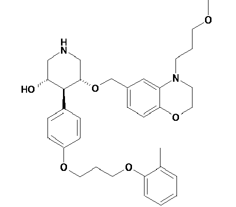 | yellowish oil | 0.18 (C) | 4.63 (I) |
| 10 | 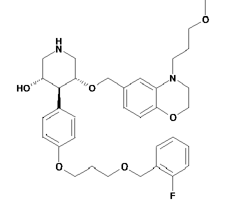 | yellowish oil | 0.15 (C) | 4.30 (I) |
| 11 | 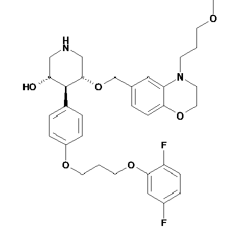 | yellowish oil | 0.15 (C) | 4.33 (I) |
| 12 | 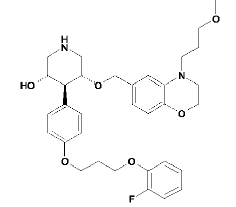 | yellowish oil | 0.15 (C) | 4.28 (I) |
| 13 | 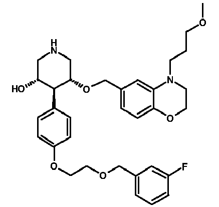 | yellowish oil | 0.11 (A) | 3.99 (I) |
| 14 | 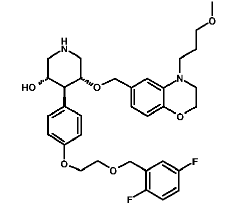 | yellowish oil | 0.14 (A) | 4.04 (I) |
| 15 | 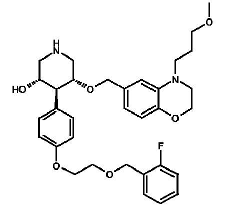 | yellowish oil | 0.15 (A) | 3.99 (I) |
| 16 | 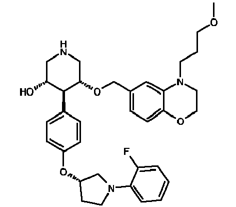 | colourless resin | 0.25 (A) | 4.21 (I) |
| 17 | 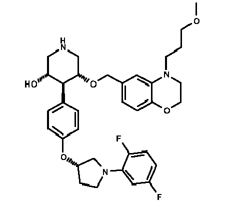 | colourless glass | 0.29 (A) | 4.35 (I) |
| 18 | 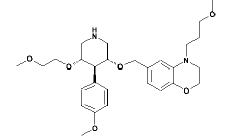 | colourless oil | 0.22 (C) | 3.50 (I) |
| 19 | 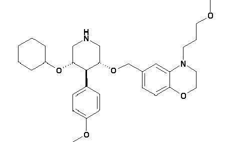 | yellow oil | 0.39 (C) | 4.19 (I) |
| 20 | 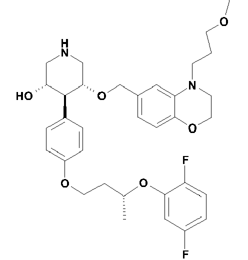 | colourless oil | 0.09 (C) | 4.52 (I) |
| 21 | 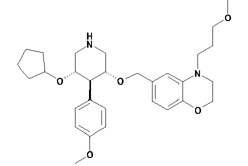 | yellow oil | 0.21 (C) | 4.04 (I) |
| 22 | 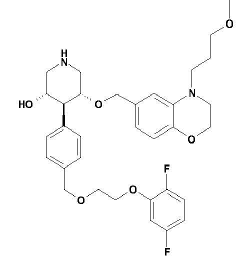 | colourless solid | 0.10 (C) | 4.18 (I) |
| 23 | 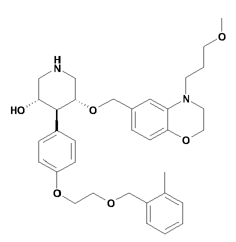 | yellowish solid | 0.30 (A) | 4.13 (I) |
| 24 | 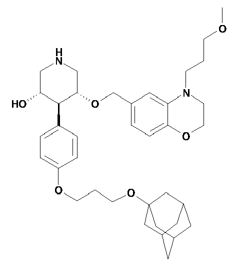 | yellowish oil | 0.09 (A) | 5.14 (I) |
| 25 | 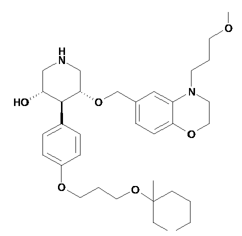 | yellowish oil | 0.08 (A) | 4.05 (I) |
| 26 | 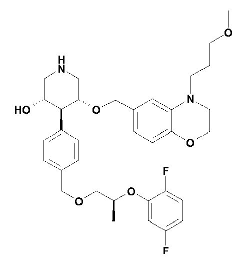 | yellowish oil | 0.19 (C) | 4.34 (I) |
| 27 | 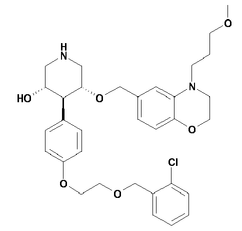 | yellowish solid | 0.17 (A) | 4.29 (I) |
| 28 | 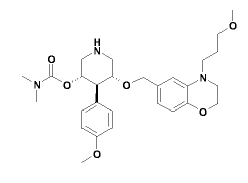 | yellow oil | 0.28 (A) | 3.67 (I) |
| 29 | 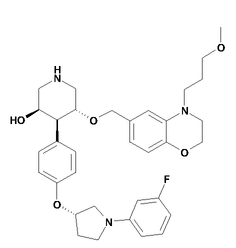 | yellow resin | 0.19 (A) | 4.50 (I) |
| 30 | 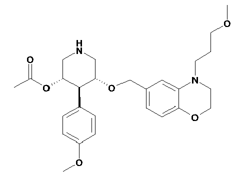 | colourless solid | 0.18 (C) | 3.65 (I) |
| 31 | 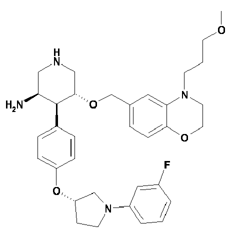 | beiger Feststoff | 0.13 (A) | 3.89 (I) |
| 32 | 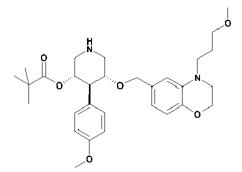 | beige solid | 0.25 (C) | 4.25 (I) |
| 33 | 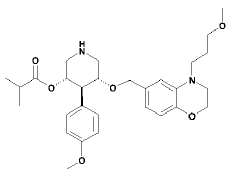 | yellowish oil | 0.28 (C) | 4.05 (I) |
| 34 | 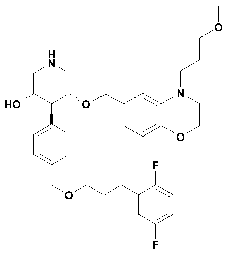 | colourless oil | 0.13 (C) | 4.49 (I) |
| 35 | 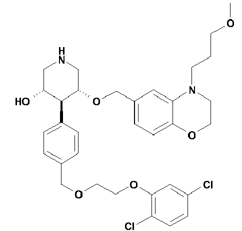 | colourless solid | 0.33 (C) | 4.64 (I) |
| 36 | 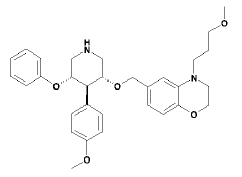 | colourless solid | 0.43 (A) | 4.14 (I) |
| 37 | 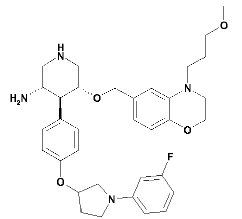 | beige solid | 0.13 (A) | 3.89 (I) |
| 38 | 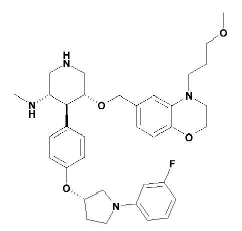 | yellowish solid | 0.28 (A) | 3.96 (I) |
| 39 | 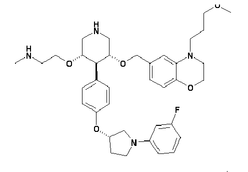 | redish oil | 0.10 (A) | 3.99 (I) |
| 40 | 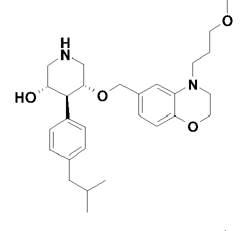 | colourless oil | 0.22 (A) | 4.28 (I) |
| 41 | 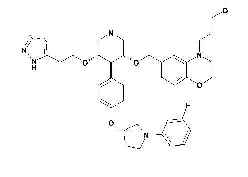 | yellowish foam | 0.09 (K) | 4.26 (I) |
| 42 | 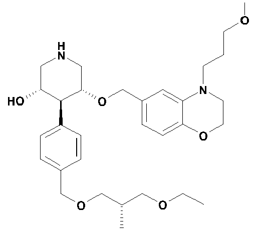 | yellowish resin | 0.21 (A) | 3.87 (I) |
| 43 | 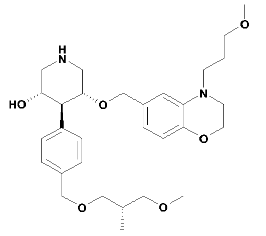 | colourless resin | 0.22 (A) | 3.66 (I) |
| 44 | 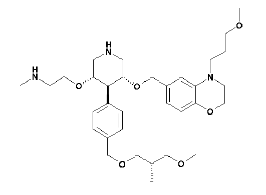 | colourless oil | 0.06 (D) | 3.30 (I) |
| 45 | 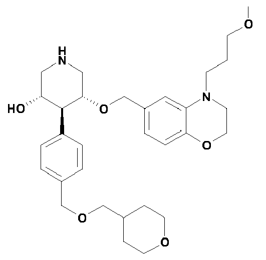 | colourless oil | 0.16 (A) | 3.83 (I) |
| 46 | 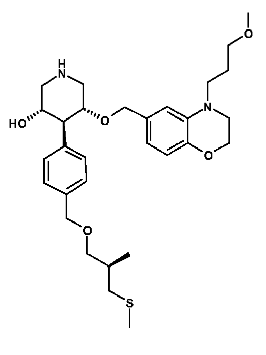 | yellowish resin | 0.10 (C) | 4.16 (I) |
| 47 | 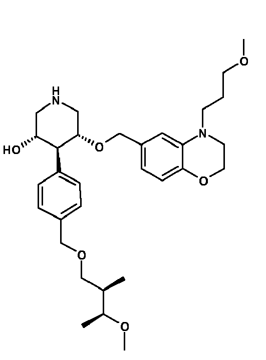 | yellowish oil | 0.33 (A) | 3.75 (I) |
| 48 | 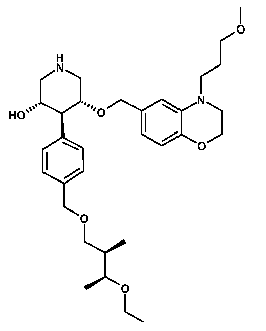 | yellowish oil | 0.33 (A) | 3.99 (I) |
| 49 | 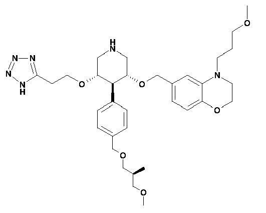 | beige solid | 0.1 (K) | 3.60 (I) |
| 50 | 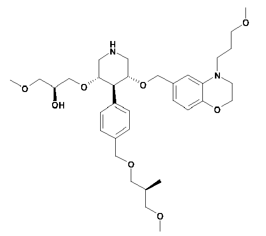 | yellowish oil | 0.35 (A) | 3.73 (I) |
| 51 | 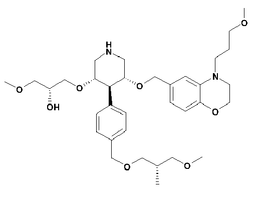 | yellowish oil | 0.35 (A) | 3.75 (I) |
| 52 | 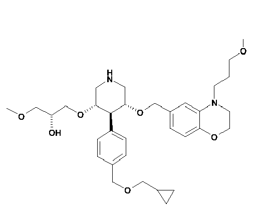 | colourless oil | 0.24 (A) | 3.76 (I) |
| 53 | 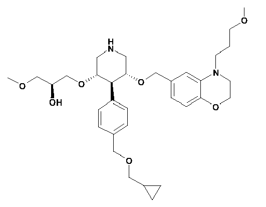 | yellowish oil | 0.24 (A) | 3.73 (I) |
| 54 | 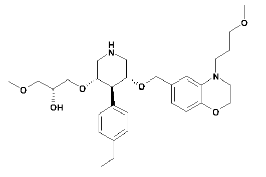 | yellowish oil | 0.19 (A) | 3.78 (I) |
| 55 | 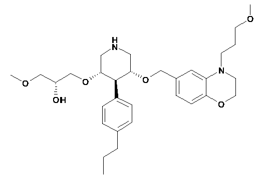 | yellowish oil | 0.17 (C) | 4.00 (I) |
| 56 | 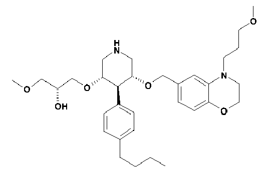 | yellowish oil | 0.17 (C) | 4.23 (I) |
| 57 | 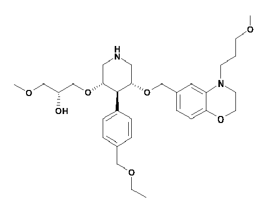 | yellowish oil | 0.13 (A) | 3.51 (I) |
| 58 | 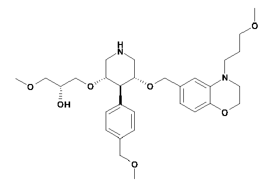 | yellow oil | 0.35 (H) | 3.32 (I) |
| 59 | 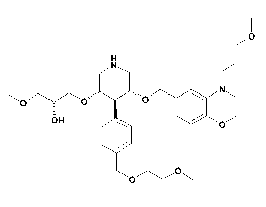 | yellow oil | 0.21 (A) | 3.29 (I) |
| 60 | 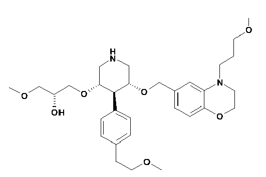 | yellowish oil | 0.11 (C) | 3.42 (I) |
| 61 | 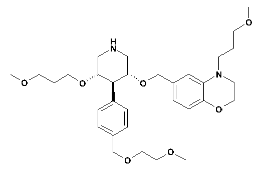 | yellow oil | 0.3 (A) | 3.58 (I) |
| 62 | 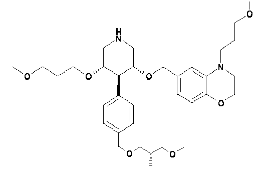 | yellow oil | 0.41 (A) | 4.03 (I) |
| 63 | 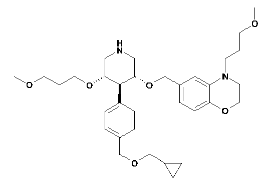 | yellow oil | 0.38 (A) | 4.14 (I) |
| 64 | 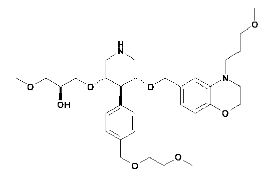 | yellow oil | 0.5 (U) | 3.28 (I) |
| 65 | 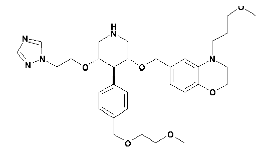 | yellowish oil | 0.28 (A) | 3.11 (I) |
| 66 | 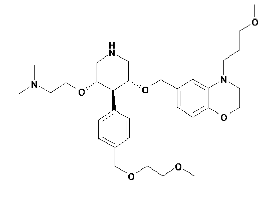 | yellowish oil | 0.11 (A) | 2.99 (I) |
| 67 | 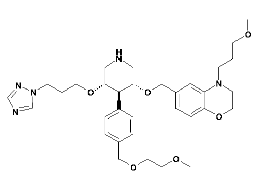 | yellowish oil | 0.28 (A) | 3.22 (I) |
| 68 | 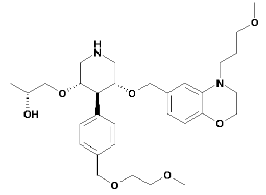 | colourless oil | 0.21 (A) | 3.32 (I) |
| 69 | 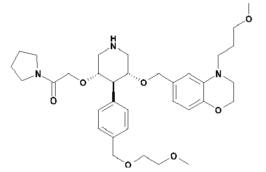 | colourless oil | 0.25 (A) | 3.46 (I) |
| 70 | 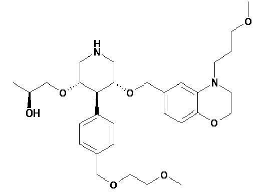 | yellowish resin | 0.306 (A) | 3.30 (I) |
| 71 | 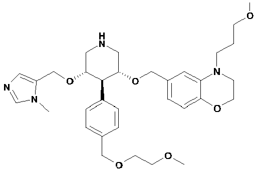 | yellowish oil | 0.18 (A) | 3.02 (I) |
| 72 | 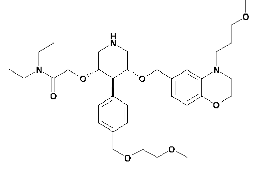 | yellow oil | 0.27 (A) | 3.64 (I) |
| 73 | 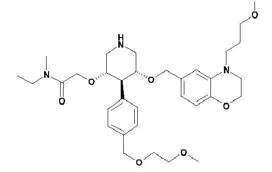 | yellow oil | 0.3 (D) | 3.47 (I) |
| 76 | 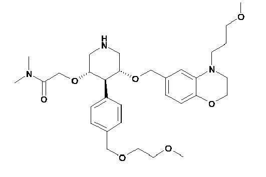 | yellowish oil | 0.29 (A) | 3.35 (I) |
| 77 | 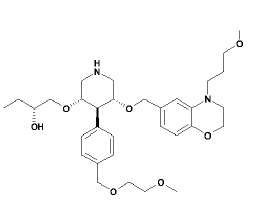 | yellowish resin | 0.14 (A) | 3.43 (I) |
| 78 | 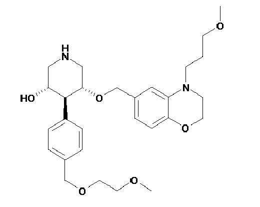 | yellowish resin | 0.18 (A) | 3.22 (I) |
| 79 | 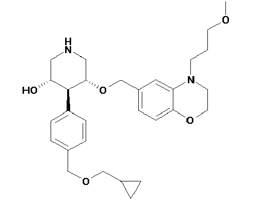 | yellow oil | 0.25 (A) | 3.64 (I) |
| 80 | 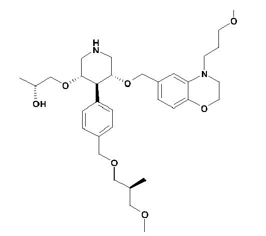 | yellowish resin | 0.42 (A) | 3.77 (I) |
| 81 | 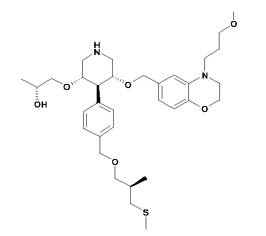 | yellowish resin | 0.47 (A) | 4.02 (I) |
| 82 | 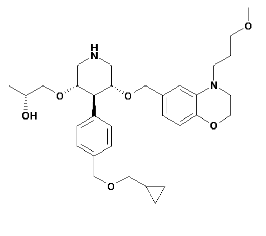 | yellowish oil | 0.20 (A) | 3.76 (I) |
| 84 | 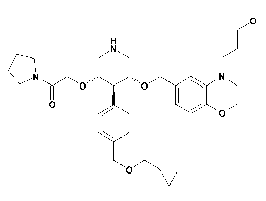 | yellowish oil | 0.23 (A) | 3.97 (I) |
| 85 | 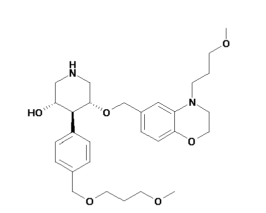 | yellowish oil | 0.20 (A) | 3.30 (I) |
| 86 | 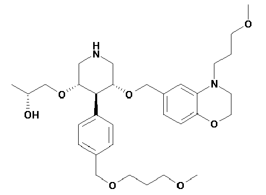 | yellowish oil | 0.20 (A) | 3.44 (I) |
| 87 | 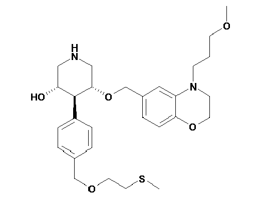 | yellowish resin | 0.38 (A) | 3.63 (I) |
| 88 | 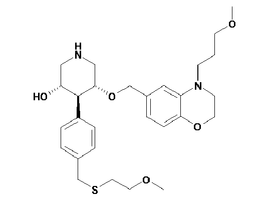 | yellowish oil | 0.20 (A) | 3.47 (I) |
| 89 | 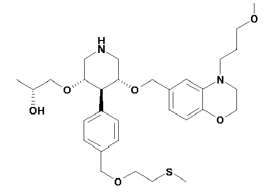 | yellow oil | 0.40 (A) | 3.72 (I) |
| 94 | 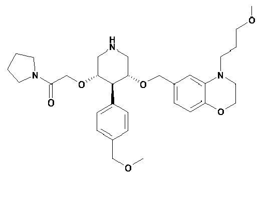 | yellowish oil | 0.24 (A) | 3.54 (I) |
| 96 | 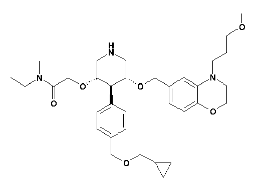 | yellowish oil | 0.28 (A) | 4.02 (I) |
| 97 | 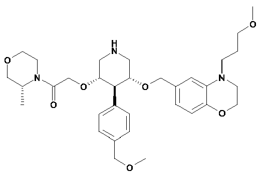 | yellowish oil | 0.31 (A) | 3.48 (I) |
| 98 | 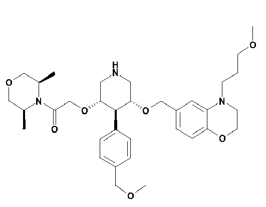 | yellowish oil | 0.27 (A) | 3.62 (I) |
| 104 | 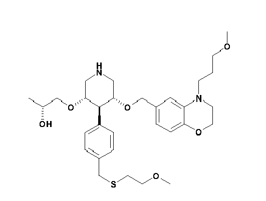 | yellow oil | 0.18 (A) | 3.62 (I) |
| 109 | 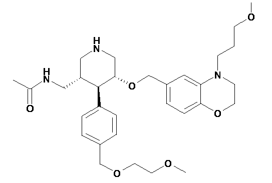 | yellow oil | 0.09 (A) | 3.26 (I) |
| 110 | 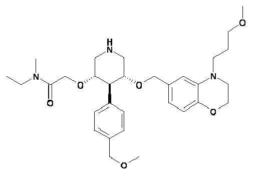 | yellowish oil | 0.27 (A) | 3.50 (I) |
| 111 | 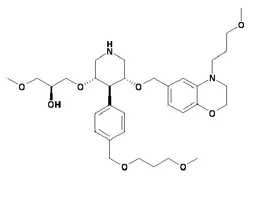 | redish oil | 0.20 (A) | 3.47 (I) |
| 112 | 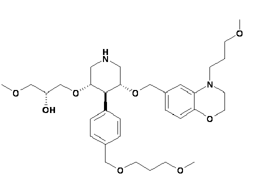 | redish oil | 0.20 (A) | 3.49 (I) |
| 113 | 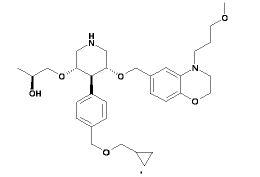 | yellowish oil | 0.28 (A) | 3.74 (I) |
| 114 | 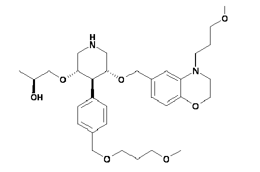 | yellow oil | 0.30 (A) | 3.44 (I) |
| 115 | 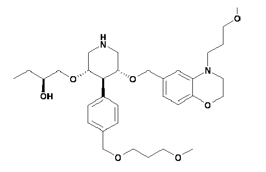 | yellow oil | 0.31 (A) | 3.65 (I) |
| 116 | 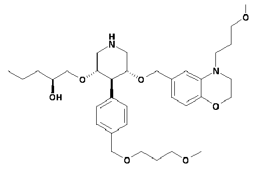 | yellow oil | 0.29 © | 3.83 (I) |
| 117 | 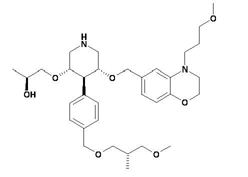 | yellowish oil | 0.29 (A) | 3.82 (I) |
| 118 | 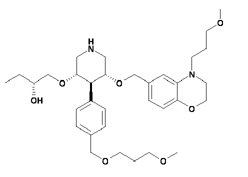 | yellow oil | 0.24 (A) | 3.59 (I) |
| 119 | 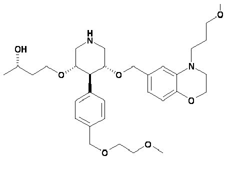 | colourless oil | 0.25 (A) | 3.34 (I) |
| 120 | 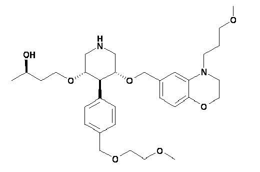 | colourless oil | 0.30 (A) | 3.32 (I) |
| 121 | 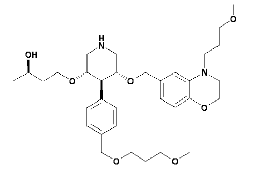 | yellowish resin | 0.32 (H) | 3.54 (I) |
| 122 | 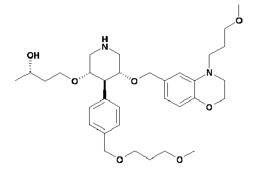 | yellow oil | 0.26 (A) | 3.55 (I) |
| 123 | 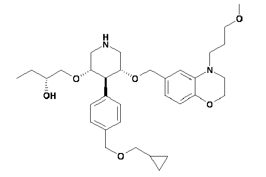 | yellowish oil | 0.30 (A) | 3.98 (I) |
| 124 | 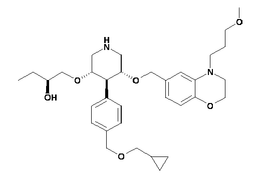 | yellowish oil | 0.31 (A) | 4.01 (I) |
| 125 | 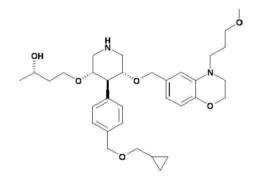 | colourless oil I | 0.34 (A) | 3.80 (I) |
| 126 | 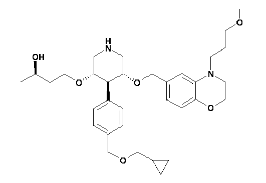 | beige oil | 0.20 (A) | 3.87 (I) |
| 127 | 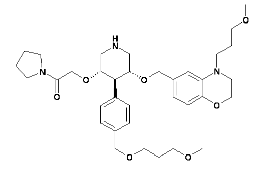 | orange resin | 0.28 (H) | 3.71 (I) |
| 128 | 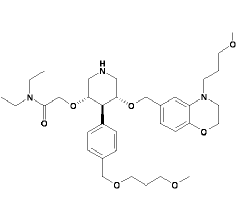 | orange resin | 0.30 (H) | 3.82 (I) |
| 129 | 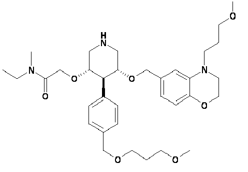 | orange resin | 0.30 (H) | 3.70 (I) |
| 130 | 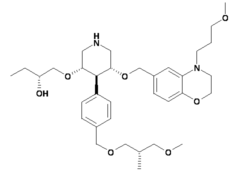 | yellow oil | 0.31 (A) | 3.97 (I) |
| 131 | 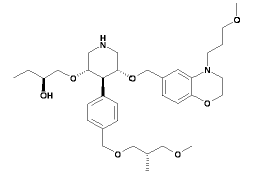 | yellow oil | 0.32 (A) | 4.00 (I) |
| 132 | 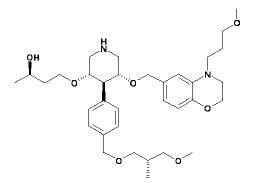 | yellowish oil | 0.20 © | 3.85 (I) |
| 133 | 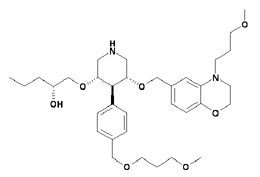 | yellow oil | 0.30 (A) | 3.86 (I) |
| 134 | 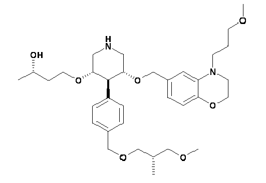 | yellowish oil | 0.22 (A) | 3.86 (I) |
| 135 | 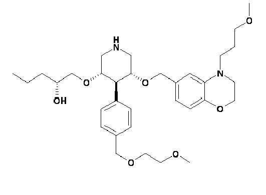 | yellowish oil | 0.08 (A) | 3.64 (I) |
| 136 | 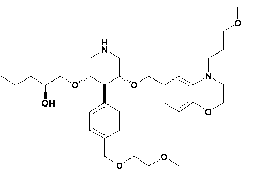 | yellow oil | 0.10 (C) | 3.63 (I) |
| 138 | 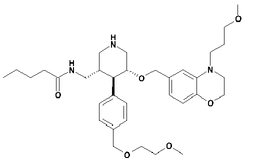 | yellow wax | 0.37 (U) | 3.72 (I) |
| 147 | 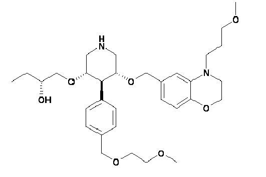 | yellowish resin | 0.14 (A) | 3.43 (I) |
| 148 | 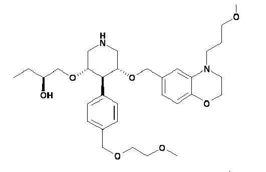 | yellow oil | 0.33 (A) | 3.50 (I) |
| 149 | 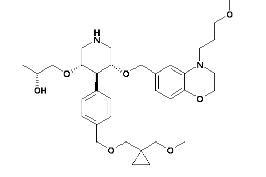 | yellow oil | 0.15 (A) | 3.70 (I) |
| 151 | 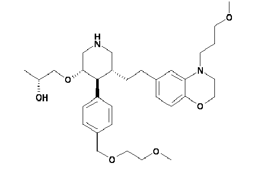 | yellowish oil | 0.18 (A) | 3.54 (I) |
| 153 | 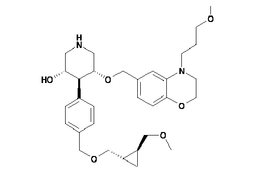 | yellow oil | 0.11 (A) | 3.47 (I) |
| 155 | 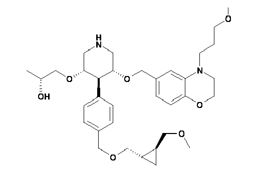 | yellow oil | 0.23 (A) | 3.62 (I) |
| 156 | 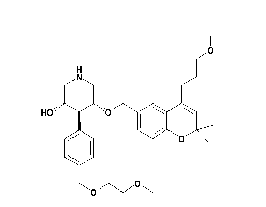 | colourless oil | 0.24 (A) | 3.82 (I) |
| 157 | 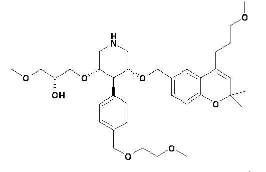 | colourless oil | 0.29 (A) | 3.97 (I) |
| 160 | 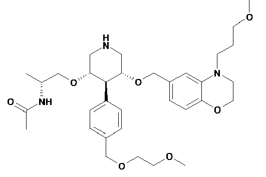 | yellowish oil | 0.16 (A) | 3.33 (I) |
| 161 | 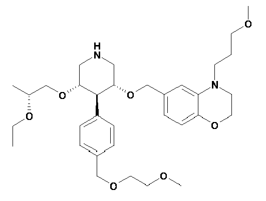 | yellow oil | 0.39 (A) | 3.73 (I) |
| 164 | 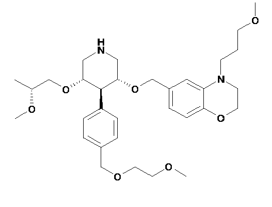 | yellow oil | 0.24 (A) | 3.58 (I) |
| 165 | 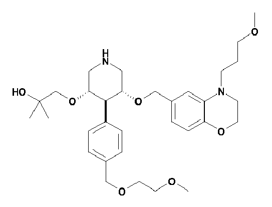 | yellowish oil | 0.31 (C) | 3.44 (I) |
| 166 | 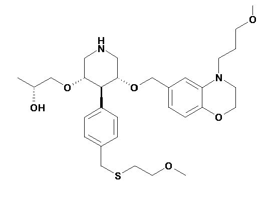 | yellow oil | 0.18 (A) | 3.62 (I) |

Thin-layer chromatography eluent systems:
- A: Dichloromethane-methanol-25% conc. ammonia = 200:20:1
- B: Dichloromethane-methanol-25% conc. ammonia = 200:20:0.5
- C: Dichloromethane-methanol-25% conc. ammonia = 200:10:1
- D: Dichloromethane-methanol-25% conc. ammonia = 90:10:1
- E: Dichloromethane-methanol-water-conc. acetic acid = 750:270:50:5
- F: Dichloromethane-methanol = 1:4
- G: Dichloromethane-methanol-25% conc. ammonia = 200:5:1
- H: Dichloromethane-methanol = 9:1
- I: Dichloromethane-methanol-25% conc. ammonia = 40:10:1
- J: Dichloromethane-methanol-25% conc. ammonia = 80:10:1
- K: Dichloromethane-methanol-25% conc. ammonia = 60:10:1
- L: Dichloromethane-methanol-25% conc. ammonia = 90:20:1
- M: Dichloromethane-methanol-25% conc. ammonia = 200:40:1
- N: Dichloromethane-methanol-25% conc. ammonia = 200:20:1 + 10% methanol
- O: Dichloromethane-methanol-25% conc. ammonia = 200:100:2
- P: Dichloromethane-methanol-25% conc. ammonia = 95:5:1
- Q: Dichloromethane-methanol-25% conc. ammonia = 200:15:2
- R: Dichloromethane-methanol-25% conc. ammonia = 200:20:2
- S: Dichloromethane-methanol-25% conc. ammonia = 200:15:1
- T: Dichloromethane-methanol-25% conc. ammonia = 200:50:1
- U: Dichloromethane-methanol-25% conc. ammonia = 200:30:1

HPLC gradients on Hypersil BDS C-18 (5 µm); column: 4 × 125 mm
- I: 90% water*/10% acetonitrile* to 0% water*/100% acetonitrile* in 5 minutes + 2.5 minutes (1.5 ml/min)
- II: 95% water*/5% acetonitrile* to 0% water*/100% acetonitrile* in 40 minutes (0.8 ml/min
- *: containing 0.1 % trifluoroacetic acid

The following abbreviations are used:
- Rf: ratio of distance travelled by a substance to separation of the eluent front from the start point in thin-layer chromatography
- Rt: retention time of a substance in HPLC (in minutes)
- m.p.: melting point (temperature)

### General Method A: (N-BOC deprotection)

15 ml of methanol and 2.5 ml of 2N HCl are successively added to a solution of 1 mmol "N-BOC derivative" in 5 ml of chloroform, and the mixture is stirred at 60°C for 18 hours. The reaction mixture is cooled to room temperature, poured into 1M aqueous sodium bicarbonate solution (40 ml) and extracted with tert-butyl methyl ether (2 × 60 ml). The organic phases are washed with brine (1 × 60 ml), dried with sodium sulphate and evaporated. The title compound is obtained from the residue by flash chromatography (SiO₂ 60F).

### General Method B: (hydrogenation)

A solution of 1 mmol of "substrate" in 15 ml of tetrahydrofuran/methanol 1:1 is hydrogenated in the presence of 100-200 mg of Pd/C 10% at 15-20°C for 2-20 hours. The reaction mixture is clarified by filtration and the filtrate is evaporated. The title compound is obtained from the residue by flash chromatography (SiO₂ 60F).

### General Method C: (9-BBN reduction)

A solution of 1 mmol of "lactam" in 3 ml of tetrahydrofuran is mixed with 3.2-6.4 mmol of 9-BBN (0.5M in tetrahydrofuran) and stirred under reflux for 1-2 hours (conversion checked by HPLC). The reaction mixture is cooled to room temperature and, after addition of 3.2-6.4 mmol of ethanolamine, evaporated. The residue is stirred in ethyl acetate/heptane 1:1 (30 ml) at 0°C overnight and clarified by filtration, and the filtrate is evaporated. The title compound is obtained from the residue by flash chromatography (SiO₂ 60F).

### General Method D: (O-alkylation)

1.1 mmol of sodium hydride (60% dispersion in oil) are added to a solution of 1 mmol of "alcohol", 1.0-2.0 mmol of "benzyl halide" in 2.0 ml of N,N-dimethylformamide while stirring at -10°C. The reaction mixture is stirred at -10°C for 1 hour and at room temperature for 18 hours. The mixture is poured into 1M aqueous sodium bicarbonate solution (50 ml) and extracted with tert-butyl methyl ether (2 × 50 ml). The organic phases are washed successively with water (1 × 50 ml) and brine (1 × 60 ml), dried with sodium sulphate and evaporated. The title compound is obtained from the residue by flash chromatography (SiO₂ 60F).

### General Method E: (chlorination)

A solution of 40 mmol of "benzyl alcohol" in 6.40 ml of pyridine and 100 ml of dichloromethane is slowly added dropwise to a solution, precooled to 0-5°C, of 7.65 ml of thionyl chloride in 20 ml of dichloromethane. The reaction mixture is stirred at 0°C and then at room temperature for 1 hour each, and then poured into 200 ml of ice-water. The mixture is extracted with dichloromethane (2 × 200 ml). The organic phases are washed successively with 1M aqueous sodium bicarbonate solution (2 × 200 ml) and brine, dried with sodium sulphate and evaporated. The title compound is obtained from the residue by flash chromatography (SiO₂ 60F).

### General Method F: (phenol alkylation I)

A mixture of 20 mmol of "phenol" in 60 ml of N,N-dimethylformamide with 4.15 g of potassium carbonate and 30 mmol of "halide" or "tosylate" is stirred at 100°C for 24 hours. The reaction mixture is then evaporated. The residue is mixed with 1M aqueous sodium bicarbonate solution (40 ml) and extracted with ethyl acetate (2 × 60 ml). The organic phases are washed with brine (1 × 60 ml), dried with sodium sulphate and evaporated. The title compound is obtained from the residue by flash chromatography (SiO₂ 60F).

### General Method G: (phenol alkylation II)

A suspension of 1 mmol of "tosylate", 2 mmol "phenol", 2 mmol of potassium carbonate and 20 ml of acetonitrile is stirred at 90°C for 24 h. The reaction mixture is then evaporated. The residue is mixed with saturated aqueous sodium bicarbonate solution and extracted with ethyl acetate (2×). The organic phases are washed with brine, dried with sodium sulphate and evaporated. The title compound is obtained from the residue by flash chromatography (SiO₂ 60F).

### General Method H: (tosylation)

A solution of 12 mmol of p-toluenesulphonyl chloride in 15 ml of dichloromethane is added dropwise to a solution of 10 mmol of "alcohol", 15 mmol of triethylamine, 1 mmol of 4-dimethylaminopyridine in 90 ml of dichloromethane at 0°C. The reaction mixture is stirred at room temperature for 2-18 hours. The reaction mixture is diluted with dichloromethane and then washed with water and brine, dried with sodium sulphate and evaporated. The title compound is obtained from the residue by flash chromatography (SiO₂ 60F).

### General Method I: (phenol alkylation III)

A suspension of 1 mmol of "phenol", 1.0-1.5 mmol of "tosylate" or "bromide", 1.5 mmol of caesium carbonate and 2 ml of acetonitrile is stirred at 80°C for 2 hours. The reaction mixture is cooled, poured into water and extracted with ethyl acetate (2×). The organic phases are washed with brine, dried with sodium sulphate and evaporated. The title compound is obtained from the residue by flash chromatography (SiO₂ 60F).

### General Method J (alcohol desilylation)

A solution of 1 mmol of "silyl ether" in 5 ml of tetrahydrofuran is mixed with 1.5-2.0 mmol of tetrabutylammonium fluoride (1M solution in tetrahydrofuran), and the solution is stirred at room temperature for 1-2 hours. The reaction solution is then diluted with water and extracted 2× with tert-butyl methyl ether. The combined organic phases are dried with sodium sulphate and evaporated. The title compound is obtained from the residue by flash chromatography (SiO₂ 60F).

### General Method K (borane reduction)

A solution of 1 mmol of "lactam" in 3 ml of tetrahydrofuran is mixed with 3.0-6.0 mmol of borane-tetrahydrofuran complex (1 M in tetrahydrofuran) and stirred at room temperature for 1-3 hours (conversion checked by HPLC or TLC). The reaction mixture is mixed with 3.0-6.0 mmol of methanol and evaporated. The title compound is obtained from the residue by flash chromatography (SiO₂ 60F).

### General Method L (N-Tos deprotection I)

0.44 mmol of sodium dihydrogen phosphate and 0.90 mmol of sodium amalgam (10% Na) are successively added at room temperature to a solution of 0.09 mmol of "tosylamide" in 10 ml of methanol. The reaction mixture is stirred for 2-18 hours, diluted with water and extracted with ethyl acetate. The organic phase is separated off and washed with brine, dried with sodium sulphate and evaporated. The title compound is obtained from the residue by flash chromatography (SiO₂ 60F).

### General Method M (O-alkylation II)

1 mmol of methylmagnesium bromide (35% solution in diethyl ether) is added to a solution of 1 mmol of "secondary alcohol" in 5 ml of tetrahydrofuran at room temperature. The reaction solution is heated to reflux for 5 minutes and then a solution of 2.2 mmol of "oxirane" in 1 ml of THF is added. The reaction mixture is heated to reflux for 1-5 hours and poured into saturated aqueous sodium bicarbonate solution, and the mixture is extracted with tert-butyl methyl ether. The combined organic phases are dried over sodium sulphate and evaporated. The title compound is obtained from the residue by flash chromatography (SiO₂ 60F).

### General Method N (N-Tos deprotection II)

0.5 ml of a bluish green sodium naphthalenide stock solution (from 0.04 g of sodium and 0.22 g of naphthalene in 5 ml of dimethoxyethane) is added to a solution of 0.1 mmol of "tosylamide" in 2 ml of dimethoxyethane at -60°C. After 3-6 hours, the reaction mixture is diluted with water and extracted with dichloromethane (2×). The combined organic phases are washed with brine, dried with sodium sulphate and evaporated. The title compound is obtained from the residue by flash chromatography (SiO₂ 60F).

### Example 1

### 4-{4-[1-(3-Fluorophenyl)pyrrolidin-3-yloxy]phenyl}-5-[4-(3-methoxypropyl)-3,4-dihydro-2H-benzo[1,4]oxazin-6-ylmethoxy]piperidin-3-ol

Analogously to method B, 0.373 g of benzyl 4-{4-[1-(3-fluorophenyl)pyrrolidin-3-yloxy]phenyl}-3-hydroxy-5-[4-(3-methoxypropyl)-3,4-dihydro-2h-benzo[1,4]-oxazin-6-ylmethoxy]piperidine-1-carboxylate is used to prepare the title compound.

The starting materials are prepared as follows:
a) Benzyl 4-{4-[1-(3-fluorophenyl)pyrrolidin-3-yloxy]phenyl}-3-hydroxy-5-[4-(3-methoxypropyl)-3,4-dihydro-2h-benzo[1,4]-oxazin-6-ylmethoxy]piperidine-1-carboxylate
   Analogously to method J, 0.590 g of 4-{4-[1-(3-fluorophenyl)pyrrolidin-3-yloxy]phenyl}-3-[4-(3-methoxypropyl)-3,4-dihydro-2H-benzo[1,4]-oxazin-6-ylmethoxy]-5-triisopropylsilanyloxy-piperidine-1-carboxylic acid is reacted. The title compound is obtained as a colourless solid. Rf = 0.14 (1:1 EtOAc - heptane); Rt = 5.57.
b) Benzyl 4-{4-[1-(3-fluorophenyl)pyrrolidin-3-yloxy)phenyl}-3-[4-(3-methoxypropyl)-3,4-dihydro-2H-benzo[1,4]oxazin-6-ylmethoxy]-5-triisopropylsilanyloxypiperidine-1-carboxylate
   Analogously to method K, 0.740 g of benzyl 4-{4-[1-(3-fluorophenyl)pyrrolidin-3-yloxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted. The title compound is obtained as a colourless oil. Rf = 0.45 (1:1 EtOAc - heptane).
c) Benzyl 4-{4-[1-(3-fluorophenyl)pyrrolidin-3-yloxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate
   Analogously to method D, 0.852 g of benzyl 4-{4-[1-(3-fluorophenyl)pyrrolidin-3-yloxy]phenyl}-3-hydroxy-5-triisopropylsilanyloxypiperidine-1-carboxylate and 0.507 g of 6-chloromethyl-4-(3-methoxypropyl)-4H-benz[1,4]oxazin-3-one are reacted. The title compound is obtained as a yellowish oil. Rf = 0.45 (1:2 EtOAc - heptane).
d) Benzyl 4-{4-[1-(3-fluorophenyl)pyrrolidin-3-yloxylphenyl}-3-hydroxy-5-triisopropylsilanyloxypiperidine-1-carboxylate
   Analogously to method I, 0.500 g of benzyl 3-hydroxy-4-(4-hydroxyphenyl)-5-triisopropylsilanyloxypiperidine-1-carboxylate and 0.455 g of 1-(3-fluorophenyl)pyrrolidin-3-yl p-toluene-4-sulphonate are reacted. The title compound is obtained as a colourless oil. Rf = 0.43 (1:1 EtOAc - heptane); Rt = 7.42.
e) Benzyl 3-hydroxy-4-(4-hydroxyphenyl)-5-triisopropylsilanyloxypiperidin-1-carboxylate
   A solution of 3.140 g of 4-(4-hydroxyphenyl)-5-triisopropylsilanyloxypiperidin-3-ol in 90 ml of ethyl acetate is admixed with 90 ml of saturated aqueous sodium hydrogencarbonate solution and 1.57 ml of benzyl chloroformate. The mixture is stirred vigorously for 30 minutes and the phases are then separated. The aqueous phase is extracted with 100 ml of ethyl acetate and the combined organic phases are dried over sodium sulphate and concentrated by evaporation. The title compound is obtained as a colourless solid from the residue by means of flash chromatography (SiO₂ F60). Rf = 0.49 (dichloromethane-methanol-conc. ammonia = 200:20:1); Rt = 5.89.
f) 4-(4-Hydroxyphenyl)-5-triisopropylsilanyloxypiperidin-3-ol
   Analogously to method B, 5.210 g of 4-(4-benzyloxyphenyl)-1-(1-phenylethyl)-5-triisopropylsilanyloxypiperidin-3-ol are reacted. The title compound is obtained as a colourless solid. Rf = 0.19 (dichloromethane-methanol-conc. ammonia = 200:20:1); Rt = 3.80.
g) 4-(4-Benzyloxyphenyl)-1-(1-phenylethyl)-5-triisopropylsilanyloxypiperidin-3-ol
   150 ml of borane-tetrahydrofuran complex (1M in tetrahydrofuran) are added dropwise at 0°C to a solution of 20.00 g of 4-(4-benzyloxyphenyl)-1-(1-phenylethyl)-3-triisopropylsilanyloxy-1,2,3,4-tetrahydropyridine in 280 ml of 1,2-dimethoxyethane. The reaction solution is subsequently stirred at 30 °C over 3 hours. The solution is cooled to room temperature and hydrolysed with 70 ml of water. The hydrolysed solution is stirred for a further 5 minutes and subsequently admixed with 56.00 g of sodium percarbonate, and the suspension is stirred at 50°C over 1 hour. The reaction mixture is poured onto 600 ml of water and extracted with 2 × 500 ml of ethyl acetate. The combined organic phases are washed with 400 ml each of water and brine, and concentrated by evaporation. The title compound is obtained as a yellowish oil from the residue by means of flash chromatography (SiO₂ F60). Rf = 0.23 (1:2 EtOAc - heptane); Rt = 5.75.
h) 4-(4-Benzyloxyphenyl)-1-(1-phenylethyl)-3-triisopropylsilanyloxy-1,2,3,4-tetrahydropyridine
   A suspension of 14.70 g of 4-(4-benzyloxyphenyl)-1-(1-phenylethyl)-1,2,3,4-tetrahydropyridin-3-ol [ 257928-45-3] in 250 ml of dichloromethane is admixed with 6.80 ml of 2,6-lutidine and cooled to 0°C. 12.60 ml of triisopropysilyl trifluoromethanesulphonate are added dropwise and the mixture is stirred at 0°C for a further 1 hour. The reaction solution is poured onto 400 ml of water and the phases are separated. The aqueous phase is extracted with 200 ml of dichloromethane; the combined organic phases are dried over sodium sulphate and concentrated by evaporation. The title compound is obtained as a yellow-brown oil from the residue by means of flash chromatography (SiO₂ F60) Rf = 0.66 (1:2 EtOAc-heptane); Rt = 5.83.
i) 1-(3-Fluorophenyl)pyrrolidin-3-yl p-toluene-4-sulphonate p-Toluenesulphonyl chloride is added in portions to a solution of 0.320 g of
   1-(3-fluorophenyl)-pyrrolidin-3-ol, 0.40 ml of triethylamine and 0.022 g of N,N-dimethylaminopyridine in 15 ml of dichloromethane. The solution is left at room temperature over 24 hours and subsequently poured onto 30 ml of saturated aqueous sodium hydrogencarbonate solution. The mixture is extracted with 2 × 50 ml of tert-butyl methyl ether and the combined organic phases are washed with 30 ml of brine, dried over sodium sulphate and concentrated by evaporation. The title compound is obtained as a light brown solid from the residue by means of flash chromatography (SiO₂ F60). Rf = 0.45 (1:1 EtOAc- heptane); Rt =5.15.
j) 1-(3-Fluorophenyl)pyrrolidin-3-ol
   A 50 ml Schlenk flask is initially charged with 0.800 g of (R)-(-)-3-pyrrolidinol hydrochloride and 1.350 g of sodium tert-butoxide and admixed under argon with 5 ml of degassed toluene. The suspension is stirred at room temperature over 30 minutes. 1.000 g of 3-fluorobromobenzene is added and the mixture is heated to 90°C. A mixture of 0.272 g of tris(dibenzylidene acetone)dipalladium-chloroform complex and 0.334 g of (±)-2,2'-bis(diphenylphosphino)-1,1'-binaphthyl in 5 ml of degassed toluene is added at 90°C and the mixture is stirred at this temperature over 3 hours. The reaction mixture is filtered through Hyflo and the filtrate is admixed with 5 g of silica gel and concentrated by evaporation. The title compound is obtained as a dark brown oil from the residue by means of flash chromatography (SiO₂ F60). Rf = 0.21 (2:3 EtOAc - heptane); Rt = 3.55.
k) 6-Chloromethyl-4-(3-methoxypropyl)-4H-benzo[1,4]oxazin-3-one
   Analogously to method E, 0.37 g of 6-hydroxymethyl-4-(3-methoxypropyl)-4H-benzo[1,4]oxazin-3-one is reacted. The title compound is obtained as a colourless oil. Rf = 0.60 (2:1 EtOAc - heptane). Rt = 4.05.
l) 6-Hydroxymethyl-4-(3-methoxypropyl)-4H-benzof1,41oxazin-3-one
   The suspension of 1.79 g of 6-hydroxymethyl-4H-benzo[1,4]oxazin-3-one, 2.20 ml of 1-chloro-3-methoxypropane, 10g of KF on alumina and 0.033 g of potassium iodide in 150 ml of acetonitrile is stirred at reflux over 72 hours. The mixture is cooled and clarified by filtration, and the filtrate is concentrated by evaporation to dryness. The title compound is obtained from the residue by means of flash chromatography (SiO₂ 60F). Rf = 0.60 (9:1 dichloromethane - methanol); Rt = 2.74.
m) 6-Hydroxymethyl-4H-benzo[1,4]oxazin-3-one
   The mixture of 6.9 g of methyl 3-oxo-3,4-dihydro-2H-benzo[1,4]oxazine-6-carboxylate in 230 ml of tetrahydrofuran is cooled to -40°C. Over 30 minutes, 88.9 ml of diisobutylaluminium hydride (1.5M in toluene) are added dropwise at -40°C. The reaction mixture is stirred at -40°C to -20°C over 1.5 hours and subsequently poured cautiously onto 150 ml of 2N HCl (cold). The organic phase is removed and the water phase is extracted with tetrahydrofuran (5 × 100 ml). The organic phases are washed with brine,(1 × 100 ml), filtered through cotton wool and concentrated by evaporation. The title compound is obtained as beige crystals from the residue by crystallization (from ethanol). Rf = 0.16 (2:1 EtOAc - heptane); Rt = 2.23; m.p.: 186 - 187°C.

According to the process described in Example 1, the following compounds are prepared in an analogous manner:

### Examples

- 8: 4-{4-[3-(3-Fluorophenoxy)propoxy]phenyl}-5-[4-(3-methoxypropyl)-3,4-dihydro-2H-benzo[1,4]oxazin-6-ylmethoxy]piperidin-3-ol
- 9: 5-[4-(3-Methoxypropyl)-3,4-dihydro-2H-benzo[1,4]oxazin-6-ylmethoxy]-4-[4-(3-o-tolyloxypropoxy)phenyl]piperidin-3-ol
- 12: 4-{4-[3-(2-Fluorophenoxy)propoxy]phenyl}-5-[4-(3-methoxypropyl)-3,4-dihydro-2H-benzo[1,4]oxazin-6-ylmethoxy]piperidin-3-ol
- 16: 4-{4-[1-(2-Fluorophenyl)pyrrolidin-3-yloxy]phenyl}-5-[4-(3-methoxypropyl)-3,4-dihydro-2H-benzo[1,4]oxazin-6-ylmethoxy]piperidin-3-ol
- 17: 4-{4-[1-(2,5-Difluorophenyl)pyrrolidin-3-yloxy]phenyl}-5-[4-(3-methoxypropyl)-3,4-dihydro-2H-benzo[1,4]oxazin-6-ylmethoxy]piperidin-3-ol
- 20: 4-{4-[3(R)-(2,5-Difluoro-phenoxy)-butoxyl-phenyl}-5-[4-(3-methoxy-propyl)-3,4-dihydro-2H-benzo[1,4]oxazin-6-ylmethoxy]-piperidin-3-ol

The starting materials are prepared as follows:
a) (R)-Toluene-4-sulphonic acid 3-(2,5-difluoro-phenoxy)-butyl ester
   According to general procedure H and starting from 0.8 g (R)-3-(2,5-difluoro-phenoxy)-butan-1-ol, the title compound is obtained as a yellowish oil. Rf = 0.45 (EtOAc - heptane 1:2); Rt = 5.10.
b) (R)-3-(2,5-Difluoro-phenoxy)-butan-1-ol
   According to general procedure J and starting from 2 g (R)-[3-(2,5-difluoro-phenoxy)-butoxy]-triisopropyl-silane, the title compound is obtained as a yellowish oil. Rf = 0.25 (EtOAc - Heptan 1:2); Rt = 3.73.
c) (R)-[3-(2,5-Difluoro-phenoxy)-butoxyl-triisopropyl-silane
   According to general procedure I, 1.85 g (S)-methanesulphonic acid 1-methyl-3-triisopropylsilanyloxy-propyl ester are reacted with. 0.859 g 2,5-difluorophenol. The title compound is obtained as a yellowish oil. Rf = 0.26 (EtOAc - heptane 1:5); Rt = 7.09.
d) (S)-Methanesulphonic acid 1-methyl-3-triisopropylsilanyloxy-propyl ester
   Methanesulphonylchloride (0.837 g) is added dropwise to a solution of 1.47 g 4-triisopropylsilanyloxy-butan-2-ol and 0.905 g triethylamine in 40 ml of dry tetrahydrofuran cooled to -15°C. The mixture is stirred for 15 minutes at that temperature then for 2 hours at room temperature. It is then poured into 50 ml ice/water and extracted with tert-butyl methyl ether (2 x100 ml). The combined organic phases are washed with 30 ml 1N HCl and 30 ml brine, dried (sodium sulphate) and evaporated to afford the title compound as a colorless oil. Rf = 0.23 (EtOAc - heptane 1:5).
e) (S)-4-Triisopropylsilanyloxy-butan-2-ol
   Triethylamine (1.173 g) is added dropwise to a solution of 2.246 g triisopropylchlorosilane and 1 g (S)-(+)-1,3-butanediol in 15 ml of dry tetrahydrofuran. The mixture is stirred for 48 hours at room temperature, then is diluted with 400 ml of tert-butyl methyl ether and washed respectively with 30 ml 1 N HCl, 50 ml water and 50 ml of brine. The organic phase is dried (sodium sulphate), filtered and evaporated to dryness. The residue is purified by means of flash column chromatography (SiO2 60F) to provide the title compound as a colorless oil. Rf = 0.31 (EtOAc - heptane 1:5).

- 23: 5-[4-(3Methoxypropyl)-3,4-dihydro-2H-benzo[1,4]oxazin-6-ylmethoxyl-4-{4-[2-(2-methyl-benzyloxy)-ethoxyl-phenyl}-piperidin-3-ol

The starting materials are prepared as follows:
a) Toluene-4-sulphonic acid 2-(2-methyl-benzyloxy)-ethyl ester
   The title compound is obtained as a light yellow oil according to method H starting from 2.900 g 2-(2-methyl-benzyloxy)-ethanol. Rf = 0.32 (EtOAc - heptane 1:2); Rt = 4.99.
b) 2-(2-Methyl-benzyloxy)-ethanol
   Dibutyltin oxide (6.100 g) is added to a solution of ethylene glycol (1.500 g) in toluene (250 ml). The reaction mixture is heated to reflux for 24 hours in a Dean-Stark apparatus. The reaction mixture is cooled to 90°C and tetrabutylammonium bromide (1.550 g) and 2-methylbenzyl bromide (6.62 ml) are added. Ca. 50 ml of toluene are distilled off and the remaining reaction mixture is then heated to reflux for 2 hours. The reaction mxture is concentrated under reduced pressure and the residue purified by means of flash column chromatography (SiO₂ 60F) to provide the title compound as a light yellow oil. Rf = 0.25 (EtOAc - heptane 1:1); Rt = 3.20.

### Example 2

### 4-{4-[3-(2-Methoxybenzyloxy)propoxylphenyl)-5-[4-(3-methoxypropyl)-3,4-dihydro-2H-benzo[1,4]oxazin-6-ylmethoxylpiperidin-3-ol

Analogously to method B, 1.000 g of benzyl 3-hydroxy-4-{4-[3-(2-methoxybenzyloxy)-propoxy]phenyl}-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:
a) Benzyl 3-hydroxy-4-{4-[3-(2-methoxybenzyloxy)propoxy]phenyl}-5-[4-(3-methoxypropyl)-3,4-dihydro-2H-benzo[1,4]oxazin-6-ylmethoxylpiperidine-1-carboxylate
   Analogously to method J, 3.100 g of benzyl 4-[4-[3-(2-methoxybenzyloxy)propoxy]phenyl]-5-[4-(3-methoxypropyl)-3,4-dihydro-2H-benzo[1,4]oxazin-6-ylmethoxy]-5-triisopropylsilanyloxy-piperidine-1-carboxylate are reacted. The title compound is obtained as a yellow resin. Rf = 0.25 (3:1 EtOAc - heptane); Rt = 5.50.
b) Benzyl 4-{4-[3-(2-methoxybenzyloxy)propoxy]phenyl}-5-[4-(3-methoxypropyl)-3,4-dihydro-2H-benzo[1,4]oxazin-6-ylmethoxyl-5-triisopropylsilanyloxypiperidine-1-carboxylate
   Analogously to method C, 3.800 g of benzyl 4-{4-[3-(2-methoxybenzyloxy)propoxy]phenyl}-5-[4-(3-methoxypropyl)-3-oxo-3,4-dihydro-2H-benzo[1,4]oxazin-6-ylmethoxy]-5-triisopropylsilanyloxypiperidine-1-carboxylate are reacted. The title compound is obtained as a yellowish oil. Rf = 0.33 (1:1 EtOAc - heptane).
c) Benzyl 4-{4-[3-(2-methoxybenzyloxy)propoxy]phenyl}-5-[4-(3-methoxypropyl)-3-oxo-3,4-dihydro-2H-benzo[1,4]oxazin-6-ylmethoxy]-5-triisopropylsilanyloxypiperidine-1-carboxylate
   Analogously to method D, 3.150 g of benzyl 3-hydroxy-4-{4-[3-(2-methoxybenzyloxy)-propoxy]phenyl}-5-triisopropylsilanyloxypiperidine-1-carboxylate and 1.583 g of 6-chloromethyl-4-(3-methoxypropyl)-4H-benzo[1,4]oxazin-3-one (Example 1k) are reacted. The title compound is obtained as a yellowish oil. Rf = 0.33 (1:1 EtOAc - heptane); Rt = 7.64.
d) Benzyl 3-hydroxy-4-{4-[3-(2-methoxybenzyloxy)propoxy]phenyl}-5-triisopropylsilanyloxypiperidine-1-carboxylate
   Analogously to method 1, 2.500 g of Benzyl 3-hydroxy-4-(4-hydroxyphenyl)-5-triisopropylsilanyloxypiperidine-1-carboxylate (Example 1 e) and 2.017 g of 3-(2-methoxybenzyloxy)propyl p-toluene-4-sulphonate are reacted. The title compound is obtained as a yellow oil. Rf = 0.43 (1:1 EtOAc - heptane); Rt = 7.42.
e) 3-(2-Methoxybenzyloxy)propyl p-toluene-4-sulphonate
   Analogously to method J, 59.00 g of 3-(2-methoxybenzyloxy)propan-1-ol [188879-03-0] are reacted. The title compound is obtained as a colourless solid. Rf = 0.21 (1:4 EtOAc - heptane); Rt = 5.05.

### Example 3

### 4-(4-Methoxyphenyl)-5-[4-(3-methoxypropyl)-3,4-dihydro-2H-benzo[1,4]oxazin-6-ylmethoxy]piperidin-3-ol

Analogously to method B, 0.0352 g of benzyl 3-hydroxy-4-(4-methoxyphenyl)-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:
a) Benzyl 3-hydroxy-4-(4-methoxyphenyl)-5-[4-(3-methoxypropyl)-3,4-dihydro-2H-benzo[1,4]oxazin-6-ylmethoxylpiperidine-1-carboxylate
   Analogously to method J, 0.170 g of benzyl 4-(4-methoxyphenyl)-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a whitish oil. Rf = 0.05 (1:1 EtOAc - heptane); Rt = 4.72.
b) Benzyl 4-(4-methoxyphenyl)-3-[4-(3-methoxypropyl)-3,4-dihydro-2H-benzo[1,4]oxazin-6-ylmethoxy]-5-triisopropylsilanyloxypiperidine-1-carboxylate
   Analogously to method K, 0.200 g of benzyl 4-(4-methoxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a colourless oil. Rf = 0.50 (1:1 EtOAc -heptane); Rt = 5.10.
c) Benzyl 4-(4-methoxyphenyl)-3-[4-(3-methoxypropyl)-3-oxo-3,4-dihydro-2H-benzo[1,4]oxazin-6-ylmethoxyl-5-triisopropylsilanyloxypiperidine-1-carboxylate
   Analogously to Example 1c, 0.375 g of benzyl 3-hydroxy-4-(4-methoxyphenyl)-5-triisopropylsilanyloxypiperidine-1-carboxylate and 0.210 g of 6-chloromethyl-4-(3-methoxypropyl)-4H-benzo[1,4]oxazin-3-one are reacted. The title compound is obtained as a colourless oil. Rf = 0.46 (1:1 EtOAc - heptane); Rt = 7.02.
d) Benzyl 3-hydroxy-4-(4-methoxyphenyl)-5-triisopropylsilanyloxypiperidine-1-carboxylate
   A solution of 0.440 g of benzyl 3-hydroxy-4-(4-hydroxyphenyl)-5-triisopropylsilanyloxypiperidine-1-carboxylate (Example 1e) in 6 ml of acetone is admixed with 0.088 ml of dimethyl sulphate and 0.163 g of potassium carbonate. The suspension is stirred at 65°C for 18 hours. After cooling, the reaction mixture is filtered and concentrated by evaporation. The residue is admixed with 30 ml of water and extracted with 100 ml of tert-butyl methyl ether. The organic phase is washed with 30 ml of brine, dried over sodium sulphate and concentrated by evaporation. The title compound is obtained as a colourless oil from the residue by means of flash chromatography (SiO₂ F60). Rt = 6.47.

### Example 4

### 6-[5-Methoxy-4-(4-methoxyphenyl)piperidin-3-yloxymethyl]-4-(3-methoxypropyl)-3,4-dihydro-2H-benzo[1,4]oxazine

Analogously to method B, 0.0672 g of benzyl 3-methoxy-4-(4-methoxyphenyl)-5-[4-(3-methoxypropyl)-3,4-dihydro-2H-benzo[1,4]oxazin-6-ylmethoxy]piperidine-1-carboxylate is used to prepare the title compound.

The starting material is prepared as follows:
a) Benzyl 3-methoxy-4-(4-methoxyphenyl)-5-[4-(3-methoxypropyl)-3,4-dihydro-2H-benzo[1,4]oxazin-6-ylmethoxy]piperidine-1-carboxylate
   Analogously to method D, 0.0758 g of benzyl 3-hydroxy-4-(4-methoxyphenyl)-5-[4-(3-methoxypropyl)-3,4-dihydro-2H-benzo[1,4]oxazin-6-ylmethoxy]piperidine-1-carboxylate (Example 3a) was reacted. The title compound is obtained as a colourless oil. Rt = 5.29.

### Example 5

### 6-(5-Methoxy-4-{4-[3-(2-methoxybenzyloxy)propoxylphenyl}piperidin-3-yloxymethyl)-4-(3-methoxypropyl)-3,4-dihydro-2H-benzo[1,4]oxazine

Analogously to method B, 0.145 g of benzyl 3-methoxy-4-{4-[3-(2-methoxybenzyloxy)propoxy]phenyl}-5-[4-(3-methoxypropyl)-3,4-dihydro-2H-benzo[1,4]-oxazin-6-ylmethoxy]piperidine-1-carboxylate is used to prepare the title compound.

The starting materials are prepared as follows:
a) Benzyl 3-methoxy-4-{4-[3-(2-methoxybenzyloxy)propoxylphenyl}-5-[4-(3-methoxypropyl)-3,4-dihydro-2H-benzo[1,4]oxazin-6-ylmethoxy]piperidine-1-carboxylate
   A solution of 0.276 g of benzyl 3-hydroxy-4-{4-[3-(2-methoxybenzyloxy)propoxy]phenyl}-5-[4-(3-methoxypropyl)-3,4-dihydro-2H-benzo[1,4]oxazin-6-ylmethoxy]piperidine-1-carboxylate (Example 2a) and 0.070 ml of methyl iodide in 5.0 ml of N,N-dimethylformamide is admixed with stirring at -10°C with 0.019 g of sodium hydride dispersion (60%). The reaction mixture is stirred at -10°C over 1 hour and at room temperature for 18 hours. The mixture is poured onto 1M aqueous sodium hydrogencarbonate solution (50 ml) and extract with tert-butyl methyl ether (2 × 50 ml). The organic phases are washed successively with water (1 × 50 ml) and brine (1 × 60 ml), dried over sodium sulphate and concentrated by evaporation. The title compound is obtained as a colourless oil from the residue by means of flash chromatography (SiO₂ 60F). Rf = 0.37 (2:1 EtOAc - heptane); Rt = 5.92.

### Example 6

### 4-{4-[3-(2-Chlorophenoxy)propoxy]phenyl}-5-[4-(3-methoxypropyl)-3,4-dihydro-2H-benzo[1,4]oxazin-6-ylmethoxy]piperidin-3-ol

0.125 g of benzyl 4-{4-[3-(2-chlorophenoxy)propoxy]phenyl}-3-hydroxy-5-[4-(3-methoxypropyl)-3,4-dihydro-2H-benzo[1,4]oxazin-6-ylmethoxy]piperidine-1-carboxylate are dissolved in 3.8 ml of dioxane. 3.8 ml each of methanol and 40% aqueous potassium hydroxide solution are added and the reaction mixture is stirred in a sealed flask over 14 hours. The reaction mixture is subsequently cooled to room temperature, diluted with 30 ml of water and extracted with ethyl acetate (3 × 60 ml). The combined organic phases are washed with 50 ml of water, dried (sodium sulphate), filtered and concentrated by evaporation. The title compound is obtained from the residue by means of flash chromatography (SiO₂ 60F).

The starting materials are prepared as follows:
a) Benzyl 4-{4-[3-(2-chiorophenoxy)propoxylphenyl}-3-hydroxy-5-[4-(3-methoxypropyl)-3,4-dihydro-2H-benzo[1,4]oxazin-6-ylmethoxylpiperidine-1-carboxylate
   Analogously to method J, 0.386 g of benzyl 4-{4-[3-(2-chlorophenoxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a yellowish oil. Rf = 0.16 (1:1 EtOAc/heptane) ; Rt = 5.64.
b) Benzyl 4-{4-[3-(2-chlorophenoxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate
   Analogously to method K, 0.525 g of benzyl 4-{4-[3-(2-chlorophenoxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a orange oil. Rf = 0.08 (1:3 EtOAc/heptane).
c) Benzyl 4-{4-[3-(2-chlorophenoxy)propoxylphenyl}-3-[4-(3-methoxypropyl)-3-oxo-3,4-dihydro-2H-benzo[1,4]oxazin-6-ylmethoxyl-5-triisopropylsilanyloxypiperidine-1-carboxylate
   Analogously to Example 1 c, 0.460 g of benzyl 4-{4-[3-(2-chlorophenoxy)propoxy]phenyl}-3-hydroxy-5-triisopropylsilanyloxypiperidine-1-carboxylate and 0.213 g of 6-chloromethyl-4-(3-methoxypropyl)-4H-benzo[1,4]oxazin-3-one are reacted. The title compound is obtained as an orange oil. Rf = 0.10 (1:3 EtOAc-heptane); Rt = 7.68.
d) Benzyl 4-{4-[3-(2-chlorophenoxy)propoxy]phenyl}-3-hydroxy-5-triisopropylsilanyloxypiperidine-1-carboxylate
   Analogously to method 1, 0.400 g of benzyl 3-hydroxy-4-(4-hydroxyphenyl)-5-triisopropylsilanyloxypiperidine-1-carboxylate (Example 1e) and 0.241 g of 1-(3-bromopropoxy)-2-chlorobenzene [50912-59-9] are reacted. The title compound is obtained as an orange oil. Rf = 0.10 (1:3 EtOAc-heptane); Rt = 7.00.

### Example 7

### 6-(4-{4-[1-(3-Fluorophenyl)pyrrolidin-3-yloxy]phenyl}-5-methoxypiperidin-3-yloxymethyl-4-(3-methoxypropyl)-3,4-dihydro-2H-benzo[1,4]oxazine

Analogously to Example 5, benzyl 4-{4-[1-(3-fluorophenyl)pyrrolidin-3-yloxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

### Example 10

### 4-{4-[3-(2-Fluorobenzyloxy)propoxy]phenyl}-5-[4-(3-methoxypropyl)-3,4-dihydro-2H-benzo[1,4]oxazin-6-ylmethoxy]piperidin-3-ol

Analogously to method B, 0.144 g of benzyl 4-{4-[3-(2-fluorobenzyloxy)propoxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:
a) Benzyl 4-{4-[3-(2-fluorobenzyloxy)propoxy]phenyl}-3-hydroxy-5-[4-(3-methoxypropyl)-3,4-dihydro-2H-benzo[1,4]oxazin-6-ylmethoxy]piperidine-1-carboxylate
   Analogously to method J, 0.522 g of benzyl 4-{4-[3-(2-fluorobenzyloxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a whitish oil. Rf = 0.15 (1:1 EtOAc-heptane); Rt = 5.51.
b) Benzyl 4-{4-[3-(2-fluorobenzyloxy)propoxy]phenyl}-3-[4-(3-methoxypropyl)-3,4-dihydro-2H-benzo[1,4]oxazin-6-ylmethoxyl-5-triisopropylsilanyloxypiperidine-1-carboxylate
   Analogously to method K, 0.688 g of benzyl 4-{4-[3-(2-fluorobenzyloxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a colourless oil. Rf = 0.49 (1:1 EtOAc/heptane).
c) Benzyl 4-{4-[3-(2-fluorobenzyloxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate
   Analogously to Example 1 c, 0.548 g of benzyl 4-{4-[3-(2-fluorobenzyloxy)propoxy]phenyl}-3-hydroxy-5-triisopropylsilanyloxypiperidine-1-carboxylate and 0.236 g of 6-chloromethyl-4-(3-methoxypropyl)-4H-benzo[1,4]oxazin-3-one are reacted. The title compound is obtained as a yellowish oil. Rt = 7.57.
d) Benzyl 4-{4-[3-(2-fluorobenzyloxy)propoxylphenyl}-3-hydroxy-5-triisopropylsilanyloxypiperidine-1-carboxylate
   Analogously to method 1, 0.400 g of benzyl 3-hydroxy-4-(4-hydroxyphenyl)-5-triisopropylsilanyloxypiperidine-1-carboxylate (Example 1e) and 0.298 g of 3-(2-fluorobenzyloxy)propyl p-toluene-4-sulphonate. The title compound is obtained as a yellowish oil. Rf = 0.54 (1:1 EtOAc-heptane); Rt = 6.89.
e) 3-(2-Fluorobenzyloxy)propyl p-toluene-4-sulphonate
   Analogously to method H, 5.94 g of 3-(2-fluorobenzyloxy)propan-1-ol are reacted. The title compound is obtained as a white solid. Rf = 0.27 (1:4 EtOAc-heptane) ; Rt = 4.99.
f) 3-(2-Fluorobenzyloxy)propan-1-ol
   A solution of 10 g 2-fluorobenzaldehyde, 7.21 g of propanediol and 0.20 g of pyridinium p-tolunesulphonate in 120 ml of hexane is stirred at 90°C in a water separator for 17 hours and then concentrated. The residue is dissolved in 90 ml of toluene and cooled at 0°C. 150 ml of diisobutylaluminium hydride (25% in toluene) are slowly added dropwise and the reaction mixture is stirred at 0°C for 2 hours. A solution of 33 g of citric acid monohydrate in 120 ml of water is added dropwise, followed by 120 ml of 2N HCl. The mixture is stirred at 0°C for another 1 hour, poured onto 150 ml of water and extracted twice with toluene. The combined organic phases are washed with 1 M sodium hydrogencarbonate solution and brine, dried over sodium sulphate, filtered and concentrated by evaporation. The title compound is obtained as a colourless oil from the residue by means of flash chromatography (SiO₂ F60). Rf = 0.39 (2:1 EtOAc-heptane); Rt = 3.22.

### Example 11

### 4-{4-[3-(2,5-Difluorophenoxy)propoxy]phenyl}-5-[4-(3-methoxypropyl)-3,4-dihydro-2H-benzo[1,4]oxazin-6-ylmethoxy]piperidin-3-ol

Analogously to method B, 0.140 g of benzyl 4-{4-[3-(2,5-difluorophenoxy)propoxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:
a) Benzyl 4-{4-[3-(2,5-difluorophenoxy)propoxy]phenyl)-3-hydroxy-5-[4-(3-methoxypropyl)-3,4-dihydro-2H-benzo[1,4]oxazin-6-ylmethoxylpiperidine-1-carboxylate
   Analogously to method J, 0.421 g of benzyl 4-{4-[3-(2,5-difluorophenoxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a whitish oil. Rf = 0.15 (1:1 EtOAc-heptane); Rt = 5.47.
b) Benzyl 4-{4-[3-(2,5-difluorophenoxy)propoxy]phenyl}-3-[4-(3-methoxypropyl)-3,4-dihydro-2H-benzo[1,4]oxazin-6-ylmethoxyl-5-triisopropylsilanyloxypiperidine-1-carboxylate
   Analogously to method K, 0.620 g of benzyl 4-{4-[3-(2,5-difluorophenoxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a colourless oil. Rf = 0.47 (1:1 EtOAc-heptane).
c) Benzyl 4-{4-[3-(2,5-difluorophenoxy)propoxylphenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate
   Analogously to Example 1c, 0.453 g of benzyl 4-{4-[3-(2,5-difluorophenoxy)propoxy]phenyl}-3-hydroxy-5-triisopropylsilanyloxypiperidine-1-carboxylate and 0.209 g of 6-chloromethyl-4-(3-methoxypropyl)-4H-benzo[1,4]oxazin-3-one are reacted. The title compound is obtained as a yellow oil. Rf = 0.47 (1:1 EtOAc-heptane). Rt = 7.26.
d) Benzyl 4-{4-[3-(2,5-difluorophenoxy)propoxylphenyl}-3-hydroxy-5-triisopropylsilanyloxypiperidine-1-carboxylate
   Analogously to method 1, 0.400 g of benzyl 3-hydroxy-4-(4-hydroxyphenyl)-5-triisopropylsilanyloxypiperidine-1-carboxylate (Example 1 e) and 0.236 g of 2-(3-bromopropoxy)-1,4-difluorobenzene are reacted. The title compound is obtained as a colourless oil. Rf = 0.09 (1:3 EtOAc-heptane). Rt = 6.74.
e) 2-(3-Bromopropoxy)-1,4-difluorobenzene
   The mixture of 9.78 g of 2,5-difluorophenol, 150.24 g of 1,3-dibromopropane and 15.585 g of potassium carbonate in 160 ml of acetonitrile is stirred at reflux over 16 hours. The reaction mixture is cooled, diluted with 400 ml of water and extracted twice with 400 ml of tert-butyl methyl ether. The combined organic phases are washed with 400 ml of 1 N NaOH and 300 ml of brine, dried over sodium sulphate, filtered and concentrated by evaporation. The title compound is obtained as a colourless oil from the residue by means of flash chromatography (SiO₂ 60F). Rf = 0.66 (1:1 EtOAc-heptane); Rt = 4.89.

### Example 13

### 4-{4-[2-(3-Fluorobenzyloxy)ethoxylphenyl}-5-[4-(3-methoxypropyl)-3,4-dihydro-2H-benzo[1,4]oxazin-6-ylmethoxylpiperidin-3-ol

Analogously to method B, 0.200 g of benzyl 4-{4-[2-(3-fluorobenzyloxy)ethoxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:
a) Benzyl 4-{4-[2-(3-fluorobenzyloxy)ethoxy]phenyl}-3-hydroxy-5-[4-(3-methoxypropyl)-3,4-dihydro-2H-benzo[1,4]oxazin-6-ylmethoxy]piperidine-1-carboxylate
   Analogously to method J, 0.410 g of benzyl 4-{4-[2-(3-fluorobenzyloxy)ethoxy]phenyl}-3-[4-(3-methoxypropyl)-3,4-dihydro-2H-benzo[1,4]oxazin-6-ylmethoxy]triisopropylsilanyloxypiperidine-1-carboxylate is reacted. The title compound is obtained as a yellowish resin. Rf = 0.13 (2:1 EtOAc - heptane); Rt = 5.31.
b) Benzyl 4-{4-[2-(3-fluorobenzyloxy)ethoxy]phenyl}-3-[4-(3-methoxypropyl)-3,4-dihydro-2H-benzo[1,4]oxazin-6-ylmethoxy]triisopropylsilanyloxypiperidine-1-carboxylate
   Analogously to method K, 0.590 g of butyl 4-{4-[2-(3-fluorobenzyloxy)ethoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted. The title compound is obtained as a yellowish oil. Rf = 0.40 (1:1 EtOAc - heptane).
c) Benzyl 4-{4-[2-(3-fluorobenzyloxy)ethoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate
   Analogously to method D, 0.575 g of benzyl 4-{4-[2-(3-fluorobenzyloxy)ethoxy]phenyl}-3-hydroxy-5-triisopropylsilanyloxypiperidine-1-carboxylate and 0.300 g 6-chloromethyl-4-(3-methoxypropyl)-4H-benz[1,4]oxazin-3-one (Example 1k). The title compound is obtained as a bright yellow oil. Rf = 0.29 (1:1 EtOAc - heptane); Rt = 7.18.
d) Benzyl 4-{4-[2-(3-fluorobenzyloxy)ethoxy]phenyl}-3-hydroxy-5-triisopropylsilanyloxypiperidine-1-carboxylate
   Analogously to method I, 0.500 g of benzyl 3-hydroxy-4-(4-hydroxyphenyl)-5-triisopropylsilanyloxypiperidine-1-carboxylate (Example 1e) and 2.017 g of 2-(3-fluorobenzyloxy)ethyl p-toluene-4-sulphonate are reacted. The title compound is obtained as a yellow oil. Rf = 0.43 (1:1 EtOAc - heptane); Rt = 7.42.
e) 2-(3-Fluorobenzyloxy)ethyl p-toluene-4-sulphonate
   Analogously to method J, 3.010 g of 2-(3-fluorobenzyloxy)ethanol are reacted. The title compound is obtained as a yellowish oil. Rf = 0.32 (1:2 EtOAc - heptane); Rt = 4.82.
f) 2-(3-Fluorobenzyloxy)ethanol
   A solution of 1.47 ml of ethylene glycol in 360 ml of toluene is admixed with 6.650 g of dibutyltin oxide and the reaction solution is subsequently heated to reflux on a water separator over 20 hours. The reaction solution is cooled gently and admixed with 3.380 g of tetrabutylammonium bromide and 10.00 g of 3-fluorobenzyl bromide. 50 ml of toluene are distilled off and the reaction mixture is subsequently heated at reflux over 2 hours. The reaction mixture is concentrated by evaporation and the title compound is obtained as a yellowish liquid from the residue by means of flash chromatography (SiO₂ 60F). Rf = 0.23 (1:1 EtOAc - heptane); Rt = 3.04.

According to the process described in Example 13, the following compounds are prepared in an analogous manner:

### Examples

- 14: 4-{4-[2-(2,5-Difluorobenzyloxy)ethoxy]phenyl}-5-[4-(3-methoxypropyl)-3,4-dihydro-2H-benzo[1,4]oxazin-6-ylmethoxy]piperidin-3-ol
- 15: 4-{4-[2-(2-Fluorobenzyloxy)ethoxy]phenyl}-5-[4-(3-methoxypropyl)-3,4-dihydro-2H-benzo[1,4]oxazin-6-ylmethoxy]piperidin-3-ol
- 24: 4-{4-[3-(Adamantan-1-yloxy)-propoxy]-phenyl]-5-[4-(3-methoxy-propyl)-3,4-dihydro-2H-benzo[1,4]oxazin-6-ylmethoxy]-piperidin-3-ol

The starting materials are prepared as follows:
a) Toluene-4-sulphonic acid 3-(adamantan-1-yloxy)-propyl ester
   The solution of 0.941 g of 3-(adamantan-1-yloxy)-propan-1-ol in 12 ml of dichloromethane is admixed with 0.99 ml triethylamine, 0.061 g 4-dimethylaminopyridine, 1.07 g p-toluenesulphonyl chloride and then stirred for 3 hours at room temperature. The reaction mixture is subsequently poured onto 50 ml of water and extracted twice with 50 ml of dichloromethane. The combined organic phases are washed with 50 ml of brine, dried over sodium sulphate, filtered and concentrated by evaporation. The title compound is obtained as a colourless oil from the residue by means of flash chromatography (SiO₂ 60F). Rf = 0.33 (1:3 EtOAc-heptane); Rt = 5.91.
b) 3-(Adamantan-1-yloxy)-propan-1-ol
   The stirred solution of 1.25 g 1-allyloxy-adamantane in 85 ml of tetrahydrofuran is treated with 1.62 ml borane-methylsulfide comlex at 0°C._The reaction solution is heated to reflux over 1.5 hours, subsequently cooled to 5°C and admixed with 1.61 ml of 2M NaOH and 0.33 ml hydrogen peroxide (30%). The mixture is heated to reflux and stirred for an additional 1.5 hours, subsequently cooled to room temperature and admixed with 85 ml of 1 M potassium carbonate solution. The mixture is extracted with tert-butyl methyl ether (2 × 80 ml) and the combined organic phases are washed with 40 ml of brine, dried over sodium sulphate and concentrated by evaporation. The title compound is obtained as a colourless oil from the residue by means of flash chromatography (SiO₂ 60F). Rf = 0.38 (1:1 EtOAc-heptane).
c) 1-Allyloxy adamantane
   A mixture of 0.434 g sodium hydride and 75 ml of anhydrous tetrahydrofuran is admixed with 1.0 g 1-adamantol and 0.936 ml allylbromide. The reaction mixture is heated to reflux and stirred for additional 18 hours. The reaction mixture is cooled to room temperature, poured onto 150 ml of water and extracted with tert-butyl methyl ether (2 × 150 ml). The combined organic phases are washed with 100 ml of brine, dried over sodium sulphate and concentrated by evaporation The title compound is obtained as a yellow oil from the residue by means of flash chromatography (SiO₂ 60F). Rf = 0.79 (1:2 EtOAc-heptane).

- 25: 5-[4-(3-Methoxy-propyl)-3,4-dihydro-2H-benzo[1,4]oxazin-6-ylmethoxy]-4-{4-[3-(1-methyl-cyclohexyloxy)-propoxyl-phenyl}-piperidin-3-ol

### Example 18

### 6-[5-(2-Methoxyethoxy)-4-(4-methoxyphenyl)piperidin-3-yloxymethyl]-4-(3-methoxypropyl)-3,4,dihydro-2H-benzo[1,4]oxazine

Analogously to method B, 0.027 g of benzyl 3-(2-methoxyethoxy)-4-(4-methoxyphenyl)-5-[4-(3-methoxypropyl)-3,4,dihydro-2H-benzo[1,4]oxazin-6-ylmethoxy]piperidine-1-carboxylate is used to prepare the title compound.

The starting materials are prepared as follows:
a) Benzyl 3-(2-methoxyethoxy)-4-(4-methoxyphenyl)-5-[4-(3-methoxypropyl)-3,4,dihydro-2H-benzo[1,4]oxazin-6-ylmethoxy]piperidine-1-carboxylate
   A solution of 0.156 g of benzyl 3-hydroxy-4-(4-methoxyphenyl)-5-[4-(3-methoxypropyl)-3,4,dihydro-2H-benzo[1,4]oxazin-6-ylmethoxy]piperidine-1-carboxylate (Example 3a) in 4 ml of anhydrous tetrahydrofuran is admixed with 0.028 g of sodium hydride (60% dispersion in paraffin). The reaction mixture is stirred at room temperature for 10 minutes and subsequently heated to reflux. A solution of 0.083 g of 2-methoxyethyl p-toluenesulphonate in 2 ml of tetrahydrofuran is added dropwise over 5 minutes and the reaction mixture is subsequently heated to reflux over 16 hours. The reaction mixture is cooled, admixed with 10 ml of water and extracted with tert-butyl methyl ether (2 × 20 ml). The combined organic phases are washed with 20 ml of brine, dried over sodium sulphate and concentrated by evaporation. The title compound is obtained as a colourless oil from the residue by means of flash chromatography (SiO₂ 60F). Rf = 0.30 (2:1 EtOAc - heptane); Rt = 5.21.

### Example 19

### 6-[5-Cyclohexyloxy-4-(4-methoxy-phenyl)-piperidin-3-yloxymethyl]-4-(3-methoxy-propyl)-3,4-dihydro-2H-benzo[1,4]oxazine

The title compound is prepared according to method C starting from 0.039 g of 3-(cyclohex-2-enyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) 3-(Cyclohex-2-enyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   According to method D, 0.105 g of 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) are reacted with 0.065 g of 3-bromo cyclohexene. The title compound is obtained as a colorless oil. Rf = 0.45 (EtOAc - heptane 2:1); Rt = 5.85.

### Example 21

### 6-[5-Cyclopentyloxy-4-(4-methoxy-phenyl)-piperidin-3-yloxymethyl]-4-(3-methoxy-propyl)-3,4-dihydro-2H-benzo[1,4]oxazine

The title compound was prepared according to method C starting from 0.035 g of 3-(cyclopent-2-enyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) 3-(Cyclopent-2-enyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   A solution of 0.105 g 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) and 0.061 g 2,2,2-trichloro-acetimidic acid cyclopent-2-enyl ester (CAS 748780-85-0) in 6 ml of dichloromethane is cooled to -60°C under argon. Boron trifluoro ethyletherate (0.023 ml) is added dropwise and the reaction mixture is stirred at -60°C to -40°C for 2 hours. The reaction mixture is quenched with saturated aqueous sodium bicarbonate solution and is extracted with dichloromethane (2x). The combined organics are washed with brine, dried (sodium sulphate) and concentrated under reduced pressure. The residue is purified by means of flash column chromatography (SiO₂ 60F) to provide the title compound as a colorless oil. Rf = 0.50 (EtOAc - heptane 2:1); Rt = 5.69.

### Example 22

### 4-{4-[2-(2,5-Difluoro-phenoxy)-ethoxymethyl]-phenyl}-5-[4-(3-methoxy-propyl)-3,4-dihydro-2H-benzo[1,4]oxazin-6-ylmethoxyl-piperidin-3-ol

The title compound is prepared according to method B starting from 0.08 g of 4-{4-[2-(2,5-difluoro-phenoxy)-ethoxymethyl]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester.

The starting materials are prepared as follows:
a) 4-{4-[2-(2,5-Difluoro-phenoxy)-ethoxymethyl]-phenyl}-3-hydroxy-5-[4-(3-methoxy-propyl)-3,4-dihydro-2H-benzo[1,4]oxazin-6-ylmethoxyl-piperidine-1-carboxylic acid benzyl ester
   According to method J, 0.241 g 4-{4-[2-(2,5-difluoro-phenoxy)-ethoxymethyl]-phenyl}-3-[4-(3-methoxy-propyl)-3,4-dihydro-2H-benzo[1,4]oxazin-6-ylmethoxy]-5-triisopropylsilanyloxypiperidine-1-carboxylic acid benzyl ester are reacted to afford the title compound as a colorless oil. Rf = 0.38 (EtOAc - heptane 2:1). Rt = 5.26.
b) 4-{4-[2-(2,5-Difluoro-phenoxy)-ethoxymethyl]-phenyl}-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester
   According to method D, 0.458 g 4-(4-chloromethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester are reacted with 0.159 g 2-(2,5-difluoro-phenoxy)-ethanol. The title compound is obtained as a colorless oil. Rf = 0.45 (EtOAc - heptane 1:2).
c) 4-(4-Chloromethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester
   2.82 g of chlorenamine are added dropwise to a solution of 1 g 4-(4-hydroxymethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester in 20 ml of dry methylene chloride cooled to 0°C. The reaction mixture is stirred for 5 minutes at 0°C and for 18 hours at room temperature. It is then poured into 100 ml water and 100 ml tert-butylmethylether. The mixture is vigorously stirred for 1 hour at room temperature. The organic phase is separated, washed with 75 ml brine, dried over sodium sulphate and evaporated under reduced pressure. The residue is purified by means of flash column chromatography (SiO2 60F) to provide the title compound as a yellow oil. Rf = 0.40 (EtOAc - heptane 1:1.5).
d) 4-(4-Hydroxymethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester
   According to method C, 6.25 g 4-(4-methoxycarbonyl-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester are reacted with 300 ml of BBN-H (4N in tetrahydrofuran) at reflux for 48 hours and then with 5.04 ml ethanolamine. The title compound is obtained as an orange oil. Rf = 0.16 (EtOAc - heptane 1:2).
e) 4-(4-Methoxycarbonyl-phenyl)-3-[4-(3-methoxy-propyl)-3-oxo-3,4-dihydro-2H-benzo[1,4]oxazin-6-ylmethoxyl-5-triisopropylsilanyloxy-piperidine-1-carboxylic acid benzyl ester
   According to method D, 7.97 g 3-hydroxy-4-(4-methoxycarbonyl-phenyl)-5-triisopropylsilanyloxy-piperidine-1-carboxylic acid benzyl ester are reacted with 4.486 g 6-chloromethyl-4-(3-methoxy-propyl)-4H-benzo[1,4]oxazin-3-one. The title compound is obtained as an orange oil. Rf = 0.25 (EtOAc - heptane 1:2); Rt = 6.81.
f) 3-Hydroxy-4-(4-methoxycarbonyl-phenyl)-5-triisopropylsilanyloxy-piperidine-1-carboxylic acid benzyl ester
   An autoclave under argon containing 80 ml N,N-dimethylformamide and 60 ml methanol is loaded with 0.402 g 1,3-bis(diphenylphosphino)propane and 0.217 g palladium(II)acetate. The mixture is stirred for 20 minutes at room temperature and then 11.95 g 3-hydroxy-4-(4-trifluoromethanesulphonyloxy-phenyl)-5-triisopropylsilanyloxy-piperidine-1-carboxylic acid benzyl ester and 5.469 g triethylamine are added. The mixture is stirred for 3 hours under an atmosphere of 5 bars of carbon monoxide at 70°C and then cooled to room temperature. A solution of 0.109 palladium(II)acetate and 0.201 g 1,3-bis(diphenylphosphino)propane in 40 ml N,N-dimethylformamide and 30 ml methanol is added and the mixture is stirred for 3 hours under an atmosphere of 5 bars of carbon monoxide. The reaction mixture is cooled to room temperature, concentrated under reduced pressure by distillation of methanol, poured into 200 ml water and extracted with 2x250 ml tert-butylmethylether. The combined organic phases are washed with 50 ml brine, dried over sodium sulphate and evaporated. The residue is purified by means of flash column chromatography (SiO2 60F) to provide the title compound as a colorless oil. Rf = 0.31 (EtOAc - heptane 2:3). Rt = 6.36.
g) 3-Hydroxy-4-(4-trifluoromethanesulphonyloxy-phenyl)-5-triisopropylsilanyloxy-piperidine-1-carboxylic acid benzyl ester
   Triethylamine (3.05 ml) and 7.66 g N-phenyl-bis(trifluoromethanesulphonylimide) are added to a solution of 10 g 3-hydroxy-4-(4-hydroxy-phenyl)-5-triisopropylsilanyloxy-piperidin-1-carbonic acid benzyl ester (example 1 e) in 80 ml of dry dichloromethane. The mixture is stirred at room temperature for 4 hours and evaporated under reduced pressure. The residue is purified by means of flash column chromatography (SiO2 60F) to provide the title compound as a yellow oil. Rf = 0.17 (EtOAc - heptane 1:3). Rt = 6.60.
h) 2-(2,5-Difluoro-phenoxy)-ethanol
   According to method J, 1.31 g [2-(2,5-difluoro-phenoxy)-ethoxy]-triisopropyl-silane are reacted to afford the title compound as a yellowish oil. Rf = 0.23 (EtOAc - heptane 1:1); Rt = 3.18.
i) [2-(2,5-Difluoro-phenoxy)-ethoxy]-triisopropyl-silane
   A mixture of 0.800 g 2,5-difluorophenol, 1.65 g potassium carbonate and 1.98 g 1-iodo-2-(triisopropylsilyloxy)ethane (CAS 93550-77-7) in 10 ml acetone is stirred at 80°C for 30 hours. The mixture is poured into 75 ml saturated sodium hydrogencarbonate solution and extracted with 2x75 ml tert-butylmethylether. The combined organic phases are washed with 75 ml brine, dried over sodium sulphate and evaporated. The residue is purified by means of flash column chromatography (SiO2 60F) to provide the title compound as a yellowish oil. Rf = 0.75 (EtOAc - heptane 1:10). Rt = 6.67.

According to the process described in Example 22, the following compounds are prepared in an analogous manner:

### Example

### 26 4-{4-[2(S)-(2,5-Difluoro-phenoxy)-propoxymethyl]-phenyl}-5-[4-(3-methoxypropyl)-3,4-dihydro-2H-benzo[1,4]oxazin-6-ylmethoxy]-piperidin-3-ol

A mixture of 0.069 g 4-{4-[2(S)-(2,5-difluoro-phenoxy)-propoxymethyl]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester, 40% aqueous potassium hydroxide solution (2 ml), methanol (2 ml) and dioxane (3 ml) is heated to 100°C for 14 hours in a sealed tube. The reaction mixture is cooled to room temperature, poured into saturated aqueous sodium bicarbonate solution (20 ml) and washed with tert-butylmethylether (2 x 50 ml). The combined organic phases are washed with water (15 ml), brine (10 ml), dried over sodium sulfate and evaporated under reduced pressure. The residue is purified by means of flash column chromatography (SiO₂ 60F) to provide the title compound.

The starting materials are prepared in the following way:
a) (S)-2-(2,5-Difluoro-phenoxy)-propan-1-ol
   According to method K, 1.33 g (S)-2-(2,5-difluoro-phenoxy)-propionic acid ethyl ester are reacted at 60°C for 5 hours to afford the title compound as a colorless oil. Rf = 0.21 (EtOAc - heptane 1:3); Rt = 3.51.
b) (S)-2-(2,5-Difluoro-phenoxy)-propionic acid ethyl ester
   According to method I, 2.00 g ethyl L-(-)-methanesulphonyl lactate and 1.256 g 2,5-difluorophenol are reacted at room temperature for 48 hours to afford the title compound as a colorless oil. Rf = 0.39 (EtOAc - heptane 1:5); Rt = 4.53.

- 35: 4-{4-[2-(2,5-Dichloro-phenoxy)-ethoxymethyl]-phenyl}-5-[4-(3-methoxy-propyl)-3,4-dihydro-2H-benzo[1,4]oxazin-6-ylmethoxy]-piperidin-3-ol

### Example 27

### 4-{4-[2-(2-Chloro-benzyloxy)-ethoxyl-phenyl}-5-[4-(3-methoxy-propyl)-3,4-dihydro-2H-benzo[1,4]oxazin-6-ylmethoxy]-piperidin-3ol

A mixture of 0.125 g 4-{4-[2-(2-chloro-benzyloxy)-ethoxy]phenyl}-3-hydroxy-5-[4-(3-methoxypropyl)-3,4-dihydro-2H-benzo[1,4]oxazin-6-ylmethoxy]-piperidine-1-carboxylic acid benzyl ester, 40% aqueous potassium hydroxide solution (8 ml) and methanol (8 ml) is heated to 105°C for 6 hours in a sealed tube. The reaction mixture is cooled to room temperature and diluted with water (40 ml) and ethyl acetate (40 ml). The layers are separated and the aqueous layer is extracted once with ethyl acetate. The combined organics are dried over sodium sulphate and concentrated. The residue is purified by means of flash column chromatography (SiO₂ 60F) to provide the title compound.

The starting materials are prepared analogous to example 1 starting from 2-(2-chlorobenzyloxy)-ethanol (CAS 1199-30-0).

### Example 28

### Dimethyl-carbamic acid 4-(4-methoxy-phenyl)-5-[4-(3-methoxy-propyl)-3,4-dihydro-2H-benzo[1,4]oxazin-6-ylmethoxy]-piperidin-3-yl ester

The title compound was prepared according to method C starting from 0.029 g of 3-dimethylcarbamoyloxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) 3-Dimethylcarbamoyloxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   Sodium hydride (0.007 g) is added to a solution of 0.105 g 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) in 2 ml of tetrahydrofuran. The mixture is stirred for 30 minutes at room temperature. N,N-Dimethylcarbamoyl chloride (0.029 ml) is added and the mixture is heated to reflux for 2 hours. Additional sodium hydride (0.007 g) and N,N-dimethylcarbamoyl chloride (0.010 ml) are added and the mixture heated to reflux for one more hour. The reaction mixture is quenched with water and is extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless oil. Rf = 0.38 (EtOAc - heptane 3:1); Rt = 5.26.

### Example 29

### 4-{4-[1-(3-Fluoro-phenyl)-pyrrolidin-3(R)-yloxyl-phenyl}-5-[4-(3-methoxy-propyl)-3,4-dihydro-2H-benzo[1,4]-oxazin-6-ylmethoxy]-piperidin-3-ol

The title compound was prepared according to method C starting from 0.022 g of 4-{4-[1-(3-fluor-phenyl)-pyrrolidin-3-yloxy]-phenyl}-3(R)-hydroxy-5-[4-(3-methoxy-propyl)-3,4-dihydro-2H-benzo[1,4]-oxazin-6-ylmethoxy]-piperidine-1-carboxylic acid benzyl ester.

The starting material is prepared as follows:
a) 4-{4-[1-(3-Fluor-phenyl)-pyrrolidin-3-yloxyl-phenyl}-3(R)-hydroxy-5-[4-(3-methoxypropyl)-3,4-dihydro-2H-benzo[1,4]-oxazin-6-ylmethoxy]-piperidine-1-carboxylic acid benzyl ester
   A solution of 0.025 ml diisopropylazodicarboxylate in 1 ml of tetrahydrofuran is added dropwise to a solution of 0.045 g 4-{4-[1-(3-fluor-phenyl)-pyrrolidin-3-yloxy]-phenyl}-3-hydroxy-5-[4-(3-methoxy-propyl)-3,4-dihydro-2H-benzo[1,4]-oxazin-6-ylmethoxy]-piperidine-1-carboxylic acid benzyl ester (example 1a), 0.015 g benzoic acid and 0.032 g triphenylphosphine in 1 ml of tetrahydrofuran The reaction solution is stirred for 3 hours at room temperature and is then concentrated under reduced pressure. The residue is purified through a short pad of SiO₂. The crude material is then dissolved in 2.5 ml of methanol/tetrahydrofuran 4:1. Potassium carbonate (0.034 g) is added to the solution and the mixture is stirred for 16 hours at room temperature. The reaction mixture is quenched with water and extracted with tert-butyl methyl ether (2x). The combined organics are washed with brine, dried (sodium sulphate) and concentrated under reduced pressure. The title compound is obtained as a colorless glass and can be used without further purification. Rf = 0.51 (EtOAc - heptane 2:1); Rt = 5.59.

### Example 30

### Acetic acid 4-(4-methoxy-phenyl)-5-[4-(3-methoxy-propyl)-3,4-dihydro-2H-benzo[1,4]oxazin-6-ylmethoxy]-piperidin-3-yl ester

The title compound is prepared according to method C starting from 0.038 g of 3-acetoxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) 3-Acetoxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   Acetic anhydride (0.018 ml) is added to a solution of 0.051 g 3-hydroxy-4-(4-methoxyphenyl)-5-[4-(3-methoxy-propyl)-3,4-dihydro-2H-benzo[1,4]oxazin-6-ylmethoxy]-piperidine-1-carboxylic acid benzyl ester (example 3a), 0.002 g N,N-dimethylaminopyridine and 0.036 ml triethylamine in 2 ml of dichloromethane The mixture is stirred for 24 hours at room temperature. The reaction mixture is quenched with water and extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless oil. Rf = 0.22 (EtOAc - heptane 1:1); Rt = 5.31.

### Example 31

### 4-{4-[1-(3-Fluoro-phenyl)pyrrolidine-yloxy]-phenyl}-5-[4-(3-methoxy-propyl)-3,4-dihydro-2H-benzo[1,4]oxazin-6-ylmethoxy]-piperidin-3(R)-ylamine

The title compound is prepared according to method C starting from 0.106 g of 3(R)-azido-4-{4-[1-(3-fluoro-phenyl)-pyrrolidin-3-yloxy]-phenyl} -5-[4-(3-methoxy-propyl)-3,4-dihydro-2H-benzo[1,4]-oxazin-6-ylmethoxy]-piperidine-1-carboxylic acid benzyl ester.

The starting materials are prepared as follows:
a) 3(R)-Azido-4-{4-[1-(3-fluoro-phenyl)-pyrrolidin-3-yloxyl-phenyl} -5-[4-(3-methoxy-propyl)-3,4-dihydro-2H-benzo[1,4]-oxazin-6-ylmethoxy]-piperidine-1-carboxylic acid benzyl ester
   A mixture of 0.174 g 4-{4-[1-(3-fluoro-phenyl)-pyrrolidin-3-yloxy]-phenyl}-3-methanesulphonyloxy-5-[4-(3-methoxy-propyl)-3,4-dihydro-2H-benzo[1,4]-oxazin-6-ylmethoxy]-piperidine-1-carboxylic acid benzyl ester and 0.141 g sodium azide in 4 ml of dimethylformamide is heated to 100°C for 24 hours. The reaction mixture is poured on water and extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless wax. Rf = 0.39 (EtOAc - heptane 1:1); Rt = 5.98.
b) 4-{4-[1-(3-Fluoro-phenyl)-pyrrolidin-3-yloxy]-phenyl}-3-methanesulphonyloxy-5-[4-(3-methoxy-propyl)-3,4-dihydro-2H-benzo[1,4]-oxazin-6-ylmethoxyl-piperidine-1-carboxylic acid benzyl ester
   Methanesulphonyl chloride (0.040 ml) is added to a solution of 0.261 g 4-{4-[1-(3-Fluorphenyl)-pyrrolidin-3-yloxy]-phenyl}-3-hydroxy-5-[4-(3-methoxy-propyl)-3,4-dihydro-2H-benzo[1,4]-oxazin-6-ylmethoxy]-piperidine-1-carboxylic acid benzyl ester (example 1 a), 0.090 ml triethylamine and 0.005 g N,N-dimethylaminopyridine in 1 ml of dichloromethane. The reaction mixture is stirred for 1 hour at room temperature and is then poured on saturated aqueous sodium bicarbonate solution and is extracted with tert-butyl methyl ether (2x). The combined organics are washed with brine, dried (sodium sulphate) and concentrated under reduced pressure. The title compound is obtained as beige foam and can be used for the next step without purification. Rt = 5.76.

### Example 32

### 2,2-Dimethyl-propionic acid 4-(4-methoxy-phenyl)-5-f4-(3-methoxy-propyl)-3,4-dihydro-2H-benzo[1,4]oxazin-6-ylmethoxyl-piperidin-3-yl ester

The title compound is prepared according to method C starting from 0.042 g of 3-(2,2-dimethyl-propionyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) 3-(2,2-Dimethyl-propionyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   Pivaloyl chloride (0.020 ml) is added to a solution of 0.052 g 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) and 0.003 g N,N-dimethylaminopyridine in 2 ml of pyridine. The mixture is stirred for 4 hours at reflux and then quenched with saturated aqueous sodium bicarbonate solution and is extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless oil. Rf = 0.31 (EtOAc - Heptan 1:1); Rt = 5.89.

According to the process described in Example 32, the following compound is prepared in an analogous manner:

### Example

- 33: lsobutyric acid 4-(4-methoxy-phenyl)-5-[4-(3-methoxy-propyl)-3,4-dihydro-2H-benzo[1,4]oxazin-6-ylmethoxy]-piperidin-3-y] ester

### Example 34

### 4-{4-[3-(2,5-Difluoro-phenyl)-propoxymethyl]-phenyl}-5-[4-(3-methoxy-propyl)-3,4-dihydro-2H-benzo[1,4]oxazin-6-ylmethoxy]-piperidin-3-ol

The title compound is prepared according to example 26 starting from 0.160 g of 4-{4-[3-(2,5-difluoro-phenyl)-propoxymethyl]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester.

The starting materials are prepared analogous to example 22 starting from 3-(2,5-difluorophenyl)-propan-1-ol.

The starting material is prepared as follows:
a) 3-(2,5-Difluoro-phenyl)-propan-1-ol
   According to method K, 2.10 g 3-(2,5-difluorophenyl)propionic acid (CAS: 130408-15-0) are reacted to afford the title compound as a colorless oil. Rf = 0.21 (EtOAc - heptane 1:3); Rt = 3.54.

### Example 36

### 6-[4-(4-Methoxy-phenyl)-5-phenoxy-piperidin-3-xloxymethyl]-4-(3-methoxy-propyl)-3,4-dihydro-2H-benzo[1,4]oxazine

The title compound is prepared according to method C starting from 0.023 g of 3-(4-chloro-phenoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) 3-(4-Chloro-phenoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   Sodium hydride (0.012 g) is added to a cooled (0°C) solution of 0.050 g 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) in 2 ml of N,N-dimethylformamide. The mixture is stirred for 15 minutes at 0°C and 1-chloro-4-fluorobenzene (0.013 ml) is added. The mixture is heated to 70°C for 2 h. The reaction is quenched with saturated aqueous sodium bicarbonate solution and is extracted with tert-butyl methyl ether (2x). The combined organics are washed with brine, dried (sodium sulphate) and concentrated under reduced pressure. The residue is dissolved in ethyl acetate (10 ml). Saturated aqueous sodium carbonate solution(1 0 ml) is added and the mixture cooled to 0°C. Benzyl chloroformate (0.015 ml) is added and the reaction mixture is then stirred vigorously for 30 minutes. The layers are separated and the aqueous layer is extracted with ethyl acetate (1x). The combined organics are washed with brine, dried over sodium sulphate and concentrated. The residue is purified by means of flash column chromatography (SiO₂ 60F) to provide the title compound as a colorless wax. Rf = 0.12 (EtOAc - heptane 1:2); Rt = 5.94.

### Example 37

### 4-{4-[1-(3-Fluoro-phenyl)pyrrolidine-yloxy]-phenyl}-5-[4-(3-methoxy-propyl)-3,4-dihydro-2H-benzo[1,4]oxazin-6-ylmethoxy]-piperidin-3(S)-ylamine

The title compound is prepared analogous to example 31 starting from 4-{4-[1-(3-fluor-phenyl)-pyrrolidin-3-yloxy]-phenyl}-3(R)-hydroxy-5-[4-(3-methoxy-propyl)-3,4-dihydro-2H-benzo[1,4]-oxazin-6-ylmethoxy]-piperidine-1-carboxylic acid benzyl ester (example 29a).

### Example 38

### {4-{4-[1-(3-Fluoro-phenyl)-pyrrolidin-yloxy]-phenyl}-5-[4-(3-methoxy-propyl)-3,4-dihydro-2H-benzo[1,4]oxazin-6-ylmethoxy]-piperidin-3-ylmethyl-amine

The title compound is prepared according to method C starting from 0.046 g of 3-(benzyloxycarbonyl-methyl-amino)-4-{4-[1-(3-fluoro-phenyl)-pyrrolidin-3-yloxy]-phenyl} -5-[4-(3-methoxy-propyl)-3,4-dihydro-2H-benzo[1,4]-oxazin-6-ylmethoxy]-piperidine-1-carboxylic acid benzyl ester.

The starting materials are prepared as follows:
a) 3-(Benzyloxycarbonyl-methyl-amino)-4-{4-[1-(3-fluoro-phenyl)-pyrrolidin-3-yloxy]-phenyl} -5-[4-(3-methoxy-propyl)-3,4-dihydro-2H-benzo[1,4]-oxazin-6-ylmethoxy]-piperidine-1-carboxylic acid benzyl ester
   Sodium hydride (0.011 g) is added to a cooled (0°C) solution of 0.087 g 3-benzyloxycarbonylamino-4-{4-[1-(3-fluoro-phenyl)-pyrrolidin-3-yloxy]-phenyl} -5-[4-(3-methoxy-propyl)-3,4-dihydro-2H-benzo[1,4]-oxazin-6-ylmethoxy]-piperidine-1-carboxylic acid benzyl ester in 2 ml of N,N-dimethylformamide. The mixture is stirred for 15 minutes at 0°C and then methyl iodide (0.023 ml) is added. The mixture is stirred at room temperature for 16 hours. The reaction is quenched with saturated aqueous sodium bicarbonate solution and is extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless wax. Rf = 0.15 (EtOAc - heptane 1:1); Rt = 6.09.
b) 3-Benzyloxycarbonylamino-4-{4-[1-(3-fluoro-phenyl)-pyrrolidin-3-yloxy]-phenyl}-5-[4-(3-methoxy-propyl)-3,4-dihydro-2H-benzo[1,4]-oxazin-6-ylmethoxy]-piperidine-1-carboxylic acid benzyl ester
   Saturated aqueous sodium carbonate solution (3 ml) is added to a solution of 0.090 g 4-{4-[1-(3-fluoro-phenyl)pyrrolidin-yloxy]-phenyl}-5-[4-(3-methoxy-propyl)-3,4-dihydro-2H-benzo[1,4]oxazin-6-ylmethoxy]-piperidin-3(S)-ylamine (example 37) in ethyl acetate (3 ml) and the mixture cooled to 0°C. Benzyl chloroformate (0.056 ml) is added and the reaction mixture is then stirred vigorously for 30 min. The layers are separated and the aqueous layer is extracted with ethyl acetate (1x). The combined organics are washed with brine, dried over sodium sulphate and concentrated. The residue is purified by means of flash column chromatography (SiO₂ 60F) to provide the title compound as a colorless wax. Rf = 0.23 (EtOAc - heptane 1:1); Rt = 5.88.

### Example 39

### (2-{4-{4-[1-(3-Fluoro-phenyl)-pyrrolidin-3-yloxyl-phenyl}-5-[4-(3-methoxy-propyl)-3,4-dihydro-2H-benzo[1,4]oxazin-6-ylmethoxy]-piperidin-3-yloxy}-ethyl)-methyl-amine

A suspension of 0.500 g N-{2-[4-{4-[1-(3-fluoro-phenyl)-pyrrolidin-3-yloxy]-phenyl}-5-[4-(3-methoxy-propyl)-3,4-dihydro-2H-benzo[1,4]-oxazin-6-ylmethoxy]-1-(toluene-4-sulfonyl)-piperidin-3yloxy]-ethyl}-4,N-dimethyl-benzenesulfonamide and 0.341 g sodium dihydrogenphosphate monohydrate in 14 ml of methanol/tetrahydrofuran 6:1 is treated portion wise with 3.200 g of Na/Hg over a period of 2 hours. After the last portion is added the reaction mixture is stirred for 16 hours at room temperature. The mixture is filtered over Hyflo and the filtrate is concentrated under reduced pressure. The residue is taken up in a 2:1 mixture of dichloromethane/saturated aqueous sodium bicarbonate solution. The layers are separated and the organic layer is washed with water, dried over sodium sulphate and evaporated. The residue is purified by means of flash column chromatography (SiO₂ 60F).

The starting material are prepared as follows:
a) N-{2-[4-{4-[1-(3-Fluoro-phenyl)-pyrrolidin-3-yloxy]-phenyl}-5-[4-(3-methoxy-propyl)-3,4-dihydro-2H-benzo[1,4]-oxazin-6-ylmethoxy]-1-(toluene-4-sulfonyl)-piperidin-3yloxyl-ethyl}-4,N-dimethyl-benzenesulfonamide
   Sodium hydride (0.306 g) is added to a solution of 1.430 g 4-{4-[1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-ol in 10 ml of dry tetrahydrofuran. The mixture is warmed to 50°C and a solution of 4.770 g toluene-4-sulfonic acid 2-[methyl-(toluene-4-sulfonyl)-amino]-ethyl ester (CAS 3559-06-6) in 10 ml of tetrahydrofuran is added. The reaction solution is stirred at 50°C for 20 hours. The solution is cooled to room temperature, diluted with tert-butyl methyl ether and washed with saturated aqueous sodium bicarbonate solution and brine, dried (sodium sulphate) and concentrated under reduced pressure. The residue is purified by means of flash column chromatography (SiO₂ 60F) to provide the title compound as a colorless foam. Rf = 0.47 (EtOAc - Heptan 2:1); Rt = 5.95.
b) 4-{4-[1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-ol
   Saturated aqueous sodium carbonate solution (25 ml) is added to a solution of 1.280 g 4-{4-[1-(3-fluoro-phenyl)-pyrrolidin-3-yloxy]-phenyl}-5-[4-(3-methoxy-propyl)-3,4-dihydro-2H-benzo[1,4]-oxazin-6-ylmethoxy]-piperidin-3-ol_(example 1)_in ethyl acetate_(25 ml) and the mixture cooled to 0°C. 4-Toluenesulfonyl chloride (0.460 g) is added and the reaction mixture is then stirred vigorously for 1 hour. The layers are separated and the aqueous layer is extracted with ethyl acetate (1x). The combined organics are washed with brine, dried over sodium sulphate and concentrated. The residue is purified by means of flash column chromatography (SiO₂ 60F) to provide the title compound as a colorless foam. Rf = 0.16 (EtOAc - heptane 1:1); Rt = 5.40.

### Example 40

### 4-(4-Isobutyl-phenyl)-5-[4-(3-methoxy-propyl)-3,4-dihydro-2H-benzo[1,4]oxazin-6-ylmethoxyl-piperidin-3-ol

To a stirred solution of 102.5 mg 3-hydroxy-4-{4-[1-(2-methoxy-acetoxy)-2-methyl-propyl]-phenyl}-5-[4-(3-methoxy-propyl)-3,4-dihydro-2H-benzo[1,4]oxazin-6-ylmethoxy]-piperidine-1-carboxylic acid benzyl ester (diastereoisomeric mixture) in 4 ml ethanol are added 10µl ethanolamine and Pd/C (10%, Engelhard). The mixture is hydrogenated for 14 hours at 0°C. Then the reaction mixture is filtered through a pad of celite and evaporated under reduced pressure. The residue is purified by means of flash column chromatography (SiO₂ 60F) to provide the title compound as a colorless oil. Rf = 0.22 (dichloromethane-methanol-ammonia 200:20:1); Rt = 4.28.

The starting materials are prepared as follows:
a) 3-Hydroxy-4-{4-[1-(2-methoxy-acetoxy)-2-methyl-propyl]-phenyl}-5-[4-(3-methoxypropyl)-3,4-dihydro-2H-benzo[1,4]oxazin-6-ylmethoxy]-piperidine-1-carboxylic acid benzyl ester (diastereoisomeric mixture)
   To a stirred solution of 182.3 mg 4-{4-[1-(2-methoxy-acetoxy)-2-methyl-propyl]-phenyl}-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (diastereoisomeric mixture) in 1.5 ml tetrahydrofuran is added 0.58 ml tetrabutylammoniumfluoride (1M in tetrahydrofuran). The reaction mixture is stirred for 2 hours at room temperature. The reaction mixture is quenched with water and extracted with ethyl acetate (2x). The combined organics were washed with brine, dried (sodium sulphate) and concentrated under reduced pressure. The residue is purified by means of flash column chromatography (SiO₂ 60F) to provide the title compound as a white foam. Rf = 0.18 (EtOAc - heptane 2:1); Rt = 5.11.
b) 4-{4-[1-(2-Methoxy-acetoxy)-2-methyl-propyl]-phenyl}-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (diastereoisomeric mixture)
   To a stirred solution of 194 mg 4-[4-(1-Hydroxy-2-methyl-propyl)-phenyl]-3-[4-(3-methoxypropyl)-3,4-dihydro-2H-benzo[1,4]oxazin-6-ylmethoxy]-5-triisopropylsilanyloxy-piperidine-1-carboxylic acid benzyl ester (diastereoisomeric mixture) in 3.5 ml toluene are added at 0°C 53 µl pyridine, 3.1 mg 4-dimethylaminopyridine and 56 µl methoxyacetyl chloride. The solution is allowed to warm to room temperature and is stirred for 2h. The reaction mixture is quenched with water and extracted with ethyl acetate (2x). The combined organics are washed with brine, dried (sodium sulphate) and concentrated under reduced pressure. The residue is purified by means of flash column chromatography (SiO₂ 60F) to provide the title compound as a light yellow oil. Rf = 0.26 (EtOAc - heptane 2:3).
c) 4-[4-(1-Hydroxy-2-methyl-propyl)-phenyl]-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (diastereoisomeric mixture)
   To a stirred solution of 320 mg 4-(4-Formyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester in 5 ml tetrahydrofuran are added at 0°C 0.49 ml isopropylmagnesium bromide (1M in tetrahydrofuran). After stirring for 1 hour, the reaction mixture is allowed to warm to room temperature. The reaction mixture is quenched with 1M HCl and extracted with ethyl acetate (2x). The combined organics are washed with brine, dried (sodium sulphate) and concentrated under reduced pressure. The residue is purified by means of flash column chromatography (SiO₂ 60F) to provide the title compound as a yellow oil. Rf = 0.27 (EtOAc - heptane 1:2).
d) 4-(4-Formyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester
   To a stirred solution of 230 mg o-iodoxy benzoic acid (IBX) in 3 ml dimethylsulfoxide is added a solution of 500 mg 4-(4-hydroxymethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (example 22) in 3 ml dimethylsulfoxide at room temperature. The reaction mixture is stirred for 30 minutes and then 0.5N NaOH is added. The resulting mixture is extracted with with tert-butyl methyl ether (2x) and the combined organics are washed with brine, dried (sodium sulphate) and concentrated under reduced pressure. The residue is purified by means of flash column chromatography (SiO₂ 60F) to provide the title compound as a yellow oil. Rf = 0.54 (EtOAc - heptane 1:1); Rt = 6.09.

### Example 41

### 6-{4-{4-[1-(3-Fluoro-phenyl)-pyrrolidin-3-yloxy]-phenyl}-5-[2-(1H-tetrazol-5-yl)-ethoxy]-piperidin-3-yloxymethyl}-4-(3-methoxy-propyl)-3,4-dihydro-2H-benzo[1,4]oxazine

The title compound is prepared according to example 39 starting from 0.600 g 6-[4-{4-[1-(3-fluoro-phenyl)-pyrrolidin-3-yloxy]-phenyl}-5-[2-(H-tetrazol-5-yl)-ethoxy]-1-(toluene-4-sulfonyl)-piperidin-3-yloxymethyl]-4-(3-methoxy-propyl)-3,4-dihydro-2H-benzo[1,4]oxazine.

The starting materials are prepared as follows:
a) 6-[4-{4-[1-(3-Fluoro-phenyl)-pyrrolidin-3-yloxy]-phenyl}-5-[2-(1H-tetrazol-5-yl)-ethoxyl-1-(toluene-4-sulfonyl)-piperidin-3-yloxymethyl]-4-(3-methoxy-propyl)-3,4-dihydro-2H-benzo[1,4]oxazine
   Dibutyltin oxide (0.0686 g) is added to a solution of 1.20 g 3-[4-{4-[1-(3-Fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-yloxy]-propionitrile and 2.36 g trimethylsilylazide in 15 ml dry toluene. The mixture is stirred at 100°C for 19 hours and then cooled to room temperature, diluted with 100 ml ethyl acetate and washed with 30 ml 1 N HCl. The aqueous phase is extracted with 100 ml ethyl acetate (2x). The combined organic phases are washed with 50 ml water and 50 ml brine, dried over sodium sulphate and evaporated under reduced pressure. The residue is purified by means of flash column chromatography (SiO₂ 60F) to provide the title compound as a colorless oil. Rf = 0.10 (EtOAc); Rt = 5.19.
b) 3-[4-{4-[1-(3-Fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-yloxy]-propionitrile
   0.378 g 2,3,4,6,7,8,9,10-Octahydro-pyrimido[1,2-a]azepine (DBU) is added to a solution of 1.85 g 4-{4-[1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-ol (example 39b) and 0.658 g acrylonitrile in 12 ml dry acetonitrile in a sealed tube protected from light. The mixture is stirred for 48 hours at 50°C and then cooled to room temperature. 0.658 g acrylonitrile and 0.378 g DBU are added and the reaction mixture is stirred for 24 hours at 50°C. This process is repeated once, then the mixture is evaporated under reduced pressure. The residue is purified by means of flash column chromatography (SiO₂ 60F) to provide the title compound as a colorless oil. Rf = 0.33 (EtOAc - heptane 1:1); Rt = 5.74.

### Example 42

### 4(S)-[4-(3-Ethoxy-2(S)-methylpropoxymethyl)phenyl]-5(R)-[4-(3-methoxypropyl)-3,4-dihydro-2H-benzo[1,4]oxazin-6-ylmethoxy]piperidin-3(S)-ol

The title compound is prepared in analogy to method L from 0.44 g of (3S,4S,5R)-4-[4-((S)-3-ethoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol.

The starting materials are prepared as follows:
a) (3S,4S,5R)-4-[4-((S)-3-Ethoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   0.62 g of 6-[(3R,4R,5S)-4-[4-((S)-3-ethoxy-2-methylpropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine are reacted in analogy to method J. The title compound is obtained as a yellowish resin. Rf = 0.33 (EtOAc/heptane 2:1); Rt = 5.35 (gradient I).
b) 6-[(3R,4R,5S)-4-[4-((S)-3-Ethoxy-2-methylpropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.119 g of sodium hydride dispersion (60%) is added to a mixture of 0.92 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine and 0.30 g of (R)-3-ethoxy-2-methylpropan-1-ol in 20 ml of N,N-dimethylformamide at 0°C. After stirring for 22 hours, the reaction mixture is mixed with saturated sodium bicarbonate solution and extracted with tert-butyl methyl ether (3x). The combined organic phases are washed with brine, dried with sodium sulphate and evaporated. The title compound is obtained as a colourless oil from the residue by flash chromatography (SiO₂ 60F). Rf = 0.31 (EtOAc/heptane 1:2).
c) 6-[(3R,4R,5S)-4-(4-Chloromethylphenyl)-1-(toluene-4-sulphonyl)-5-triisopropylsilanyloxy-piperidin-3-yloxymethyl]-4-(3-methoxypropyl)-3,4-dihydro-2H-benzo[1,4]oxazine
   0.633 ml of methanesulphonyl chloride is added to a mixture of 5.28 g of {4-[(3R,4R,5S)-3-[4-(3-methoxypropyl)-3,4-dihydro-2H-benzo[1,4]oxazin-6-ylmethoxy]-1-(toluene-4-sulphonyl)-5-triisopropylsilanyloxypiperidin-4-yl]phenyl}methanol, 1.48 ml of triethylamine and 0.185 g of tetrabutylammonium chloride in 100 ml of dichloromethane at room temperature. After 66 hours, the reaction mixture is diluted with tert-butyl methyl ether and washed successively with water and brine, dried with sodium sulphate and evaporated. The title compound is obtained as a yellowish resin from the residue. Rf = 0.26 (EtOAc/heptane 1:2).
d) {4-[(3R,4R,5S)-3-[4-(3-Methoxypropyl)-3,4-dihydro-2H-benzo[1,4]oxazin-6-ylmethoxy]-1-(toluene-4-sulphonyl)-5-triisopropylsilanyloxypiperidin-4-yl]-phenyl}methanol
   1.43 g of toluenesulphonyl chloride are added to a mixture of 4.16 g of (4-{(3R,4R,5S)-3-[4-(3-methoxypropyl)-3,4-dihydro-2H-benzo[1,4]oxazin-6-ylmethoxy]-5-triisopropylsilanyloxy-piperidin-4-yl}phenyl)methanol in 125 ml of ethyl acetate and 125 ml of 2N sodium carbonate solution at room temperature. After 14 hours, the phases are separated and the aqueous phase is extracted with ethyl acetate. The combined organic phases are washed with brine, dried with sodium sulphate and evaporated. The title compound is obtained as an orange resin. Rf = 0.30 (EtOAc/heptane 1:1); Rt = 29.47 (II).
e) (4-{(3R,4R,5S)-3-[4-(3-Methoxypropyl)-3,4-dihydro-2H-benzo[1,4]oxazin-6-ylmethoxyl-5-triisopropylsilanyloxypiperidin-4-yl}phenyl)methanol
   5.0 g of benzyl (3R,4R,5S)-4-(4-hydroxymethylphenyl)-3-[4-(3-methoxypropyl)-3,4-dihydro-2H-benzo[1,4]oxazin-6-ylmethoxy]-5-triisopropylsilanyloxypiperidine-1-carboxylate are reacted in analogy to method B. The title compound is obtained as an orange resin. Rt = 4.66 (gradient I).
f) Benzyl (3R,4R,5S)-4-(4-hydroxymethylphenyl)-3-[4-(3-methoxypropyl)-3,4-dihydro-2H-benzo[1,4]oxazin-6-ylmethoxy]-5-triisopropylsilanyloxypiperidine-1-carboxylate
   27.47 g of benzyl (3R,4R,5S)-4-(4-carboxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate are reacted in analogy to method K. The title compound is obtained as an orange resin. Rf = 0.14 (EtOAc/heptane 1:2).
g) Benzyl (3R,4R,5S)-4-(4-carboxyphenyl)-3-[4-(3-methoxypropyl)-3-oxo-3,4-dihydro-2H-benzo[1,4]oxazin-6-ylmethoxyl-5-triisopropylsilanyloxypiperidine-1-carboxylate
   A mixture of 28.85 g of benzyl (3R,4R,5S)-4-(4-methoxycarbonylphenyl)-3-[4-(3-methoxypropyl)-3-oxo-3,4-dihydro-2H-benzo[1,4]oxazin-6-ylmethoxy]-5-triisopropylsilanyloxy-piperidine-1-carboxylate in 250 ml of tetrahydrofuran and 144.5 ml 1N NaOH is stirred at 80°C for 48 hours. The reaction mixture is cooled, mixed with 100 ml of 2N HCl and extracted with tert-butyl methyl ether (3 × 750 ml). The combined organic phases are evaporated, stripped with toluene, taken up in ethyl acetate, dried with sodium sulphate, filtered and evaporated, and the crude title compound is obtained as a yellowish resin. Rt = 6.31 (gradient I).
h) Benzyl (3R,4R,5S)-4-(4-methoxycarbonylphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate
   36.80 g of benzyl (3R,4R,5S)-3-hydroxy-4-(4-methoxycarbonylphenyl)-5-triisopropylsilanyl-oxypiperidine-1-carboxylate and 22.60 g of 6-chloromethyl-4-(3-methoxypropyl)-4H-benzo[1,4]oxazin-3-one are reacted in analogy to method D. The title compound is obtained as a yellowish oil. Rf = 0.14 (EtOAc/heptane 1:2); Rt = 6.86 (gradient I).
i) Benzyl (3R,4R,5S)-3-hydroxy-4-(4-methoxycarbonylphenyl)-5-triisopropylsilanyloxy-piperidine-1-carboxylate
   160 ml of N,N-dimethylformamide, 116 ml of methanol, 1.08 g of diphenylphosphinopropane and 0.582 g of palladium(II) acetate are introduced into an autoclave under argon. The reaction mixture is stirred at room temperature for 20 minutes. Then 66.73 g of benzyl (3R,4R,5S)-3-hydroxy-4-(4-trifluoromethanesulphonyloxyphenyl)-5-triisopropylsilanyloxy-piperidine-1-carboxylate and 16 ml of triethylamine are added, and the autoclave is loaded with 5 bar of carbon monoxide. The reaction mixture is then stirred under a pressure of 5 bar at 70°C for 3 hours. The reaction mixture is subsequently cooled, and a solution of palladium(II) acetate (0.293 g) and diphenylphosphinopropane (0.539 g) in 90 ml of DMF and 65 ml of methanol is added. The reaction mixture is then stirred under 5 bar of carbon monoxide at 70°C for a further 3 hours. The reaction solution is cooled and stirred with 580 ml of water and 180 ml of tert-butyl methyl ether. The phases are separated and the aqueous phase is extracted twice more with 180 ml of tert-butyl methyl ether. The organic phases are combined and evaporated to dryness. The title compound is obtained as an orange oil from the residue by flash chromatography (SiO₂ 60F). Rf = 0.19 (EtOAc/heptane 1:2).
j) Benzyl (3R,4R,5S)-3-hydroxy-4-(4-trifluoromethanesulphonyloxyphenyl)-5-triisopropylsilanyloxypiperidine-1-carboxylate
   40.55 g of N-phenyl(trifluoromethanesulphonimide) and then 16.2 ml of triethylamine are added to a solution of 52.95 g of benzyl (3R,4R,5S)-3-hydroxy-4-(4-hydroxyphenyl)-5-triisopropylsilanyloxypiperidine-1-carboxylate at room temperature. After 3.5 hours, the reaction mixture is mixed with 150 g of SiO₂ and evaporated. The title compound is obtained as a yellow oil from the residue by flash chromatography (SiO₂ 60F). Rf = 0.30 (EtOAc/heptane 1:2); Rt = 6.51 (gradient I).
k) Benzyl (3R,4R,5S)-3-hydroxy-4-(4-hydroxyphenyl)-5-triisopropylsilanyloxypiperidine-1-carboxylate
   A solution of 63.0 g of (3R,4R,5S)-4-(4-hydroxyphenyl)-5-triisopropylsilanyloxypiperidin-3-ol in 800 ml of ethyl acetate is mixed with 800 ml of saturated sodium bicarbonate solution. The two-phase mixture is cooled to 0°C and, after slow addition of 31.1 g of benzyl chloroformate, stirred for 2 hours. The reaction mixture is extracted with ethyl acetate/tetrahydrofuran. The organic phases are evaporated, and the title compound is obtained as a white foam by crystallization (EtOAc/heptane) from the residue. Rf = 0.38 (EtOAc/heptane 1:1); Rt = 5.77 (gradient I).
l) (3R,4R,5S)-4-(4-Hydroxyphenyl)-5-triisopropylsilanyloxypiperidin-3-ol
   5.210 g of (3R,4R,5S)-4-(4-benzyloxyphenyl)-1-((R)-1-phenylethyl)-5-triisopropylsilanyloxy-piperidin-3-ol are reacted in analogy to method B. The title compound is obtained as a colourless solid. Rf = 0.19 (dichloromethane/methanol/25% conc. ammonia = 200:20:1); Rt = 3.80 (gradient I).
m) (3R,4R,5S)-4-(4-Benzyloxyphenyl)-1-((R)-1-phenylethyl)-5-triisopropylsilanyloxy-piperidin-3-ol
   150 ml of borane-tetrahydrofuran complex (1M in tetrahydrofuran) are added dropwise to a solution of 20.00 g of (S)-4-(-4-benzyloxyphenyl)-1-((R)-1-phenylethyl)-3-triisopropyl silanyloxy-1,2,3,4-tetrahydropyridine in 280 ml of 1,2-dimethoxyethane at 0°C. The reaction solution is then stirred at 30°C for 3 hours. The solution is cooled to room temperature and hydrolysed with 70 ml of water. The hydrolysed solution is stirred for 5 minutes and then 56.00 g of sodium percarbonate are added, and the suspension is stirred at 50°C for 1 hour. The reaction mixture is poured into 600 ml of water and extracted with ethyl acetate (2x). The combined organic phases are washed with 400 ml each of water and brine and evaporated. The title compound is obtained as a yellowish oil from the residue by flash chromatography (SiO₂ F60). Rf = 0.23 (EtOAc/heptane 1:2); Rt = 5.75 (gradient I).
n) (S)-4-(4-Benzyloxyphenyl)-1-((R)-1-phenylethyl)-3-triisopropylsilanyloxy-1,2,3,6-tetrahydropyridine
   A suspension of 14.70 g of 4-(4-benzyloxyphenyl)-1-(1(R)-phenylethyl)-1 ,2,3,6-tetrahydropyridin-3(S)-ol [257928-45-3] in 250 ml of dichloromethane is mixed with 6.80 ml of 2,6-lutidine and cooled to 0°C. 12.60 ml of triisopropylsilyl trifluoromethanesulphonate are added dropwise, and the reaction mixture is stirred at 0°C for 1 hour. The reaction solution is poured into 400 ml of water, and the phases are separated. The aqueous phase is back-extracted with 200 ml of dichloromethane, and the combined organic phases are dried with sodium sulphate and evaporated. The title compound is obtained as a yellowish brown oil from the residue by flash chromatography (SiO₂ F60). Rf = 0.66 (EtOAc/heptane 1:2); Rt = 5.83 (gradient I).
o) 6-Chloromethyl-4-(3-methoxypropyl)-4H-benzo[1,4]oxazin-3-one
   0.37 g of 6-hydroxymethyl-4-(3-methoxypropyl)-4H-benzo[1,4]oxazin-3-one is reacted in analogy to method E. The title compound is obtained as a colourless oil. Rf = 0.60 (EtOAc/heptane 2:1); Rt = 4.05 (gradient I).
p) 6-Hydroxymethyl-4-(3-methoxypropyl)-4H-benzo[1,4]oxazin-3-one
   A suspension of 1.79g of 6-hydroxymethyl-4H-benzo[1,4]oxazin-3-one, 2.20 ml of 1-chloro-3-methoxypropane, 10 g of potassium fluoride on alumina and 0.033 g of potassium iodide in 150 ml of acetonitrile is stirred under reflux for 72 hours. The reaction mixture is cooled and clarified by filtration, and the filtrate is evaporated to dryness. The title compound is obtained as a yellow oil from the residue by flash chromatography (SiO₂ 60F). Rf = 0.60 (dichloromethane/methanol 9:1); Rt = 2.74 (gradient I).
q) 6-Hydroxymethyl-4H-benzo[1,4]oxazin-3-one
   A mixture of 6.9 g of methyl 3-oxo-3,4-dihydro-2H-benzo[1,4]oxazine-6-carboxylate [202195-67-3] and 230 ml of tetrahydrofuran is cooled to -40°C. 88.9 ml of diisobutylaluminium hydride (1.5M in toluene) are added dropwise over the course of 30 minutes at -40°C. The reaction mixture is stirred at -40°C to -20°C for 1.5 hours and then cautiously poured into 150 ml of 2N HCI (cold). The organic phase is separated off and the aqueous phase is extracted with tetrahydrofuran (5 × 100 ml). The organic phases are washed with brine (1 × 100 ml), filtered through cotton wool and evaporated. The title compound is obtained as beige crystals from the residue by crystallization (from ethanol). Rf = 0.16 (EtOAc/heptane 2:1); Rt = 2.23 (gradient I); m.p.: 186-187°C.

The following compounds are prepared in an analogous manner to the process described in Example 42:

### Examples

- 45: 5(R)-[4-(3-Methoxypropyl)-3,4-dihydro-2H-benzo[1,4]oxazin-6-ylmethoxy]-4(S)-[4-(tetrahydropyran-4-ylmethoxymethyl)phenyl]piperidin-3(S)-ol
- 79: (3S,4S,5R)-4-(4-Cyclopropylmethoxymethylphenyl)-5-[4-(3-methoxypropyl)-3,4-dihydro-2H-benzo[1,4]oxazin-6-ylmethoxy]piperidin-3-ol

### Example 43

### (3S,4S,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxylpiperidin-3-ol

The title compound is prepared in analogy to method L from 0.35 g of (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol.

The starting materials are prepared as follows:
a) (3S,4S,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   1.42 g of 6-[(3R,4R,5S)-4-[4-((S)-3-methoxy-2-methylpropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine are reacted in analogy to method J. The title compound is obtained as a yellowish resin. Rf = 0.24 (EtOAc/heptane 2:1); Rt = 5.13 (gradient I).
b) 6-[(3R,4R,5S)-4-[4-((S)-3-Methoxy-2-methylpropoxymethyl)-phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   2 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and 0.551 g of (R)-3-methoxy-2-methylpropan-1-ol are reacted in analogy to Example 42b. The title compound is obtained as a yellowish resin. Rf = 0.26 (EtOAc/heptane 1:2).
c) (R)-3-Methoxy-2-methylpropan-1-ol
   3.03 g of triisopropyl-(3-methoxy-2(S)-methylpropoxy)silane are reacted in analogy to method J. The title compound is obtained as a yellowish liquid. Rf = 0.15 (EtOAc/heptane 1:4).
d) Triisopropyl-((S)-3-methoxy-2-methylpropoxy)silane
   3.09 g of sodium hydride (60% dispersion in oil) are added to a solution of 9.55 g of (S)-2-methyl-3-triisopropylsilanyloxypropan-1-ol [256643-28-4] and 7.3 ml of methyl iodide in 70 ml of N,N-dimethylformamide at 0°C. After 60 hours at room temperature, the reaction mixture is diluted with tert-butyl methyl ether and washed successively with water and brine, dried with sodium sulphate and evaporated. The title compound is obtained as a yellow oil from the residue by flash chromatography (SiO₂ 60F). Rf = 0.41 (EtOAc/heptane 1:10).

### Example 44

### (2-{(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]piperidin-3-yloxy}methyl)methylamine

The title compound is prepared from 0.28 g of N-{2-[(3S,4R,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]ethyl}-N-methylbenzenesulphonamide in analogy to method L.

The starting material is prepared as follows:
a) N-{2-[(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-yloxy]ethyl}-N-methylbenzenesulphonamide
   0.104 g of sodium hydride dispersion (60%) is added to a solution of 0.38 g of (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol and 0.80 g of 2-[methyl(toluene-4-sulphonyl)amino]ethyl toluene-4-sulphonate in 6 ml of tetrahydrofuran at room temperature, and the mixture is then heated to 45°C. 2-[methyl(toluene-4-sulphonyl)-amino]ethyl toluene-4-sulphonate and sodium hydride dispersion (60%) are again added after 1 and 2 hours. After 3 hours, the reaction mixture is diluted with tert-butyl methyl ether and washed successively with 1:1 water/saturated aqueous sodium bicarbonate solution and brine, dried with sodium sulphate and evaporated. The title compound is obtained as a colourless oil from the residue by flash chromatography (SiO₂ 60F) . Rf = 0.36 (EtOAc/heptane 2:1); Rt = 5.96 (gradient I).
b) (3S,4S,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxyl-1-(toluene-4-sulphonyl)piperidin-3-ol
   0.62 g of 6-[(3R,4R,5S)-4-[4-((S)-3-methoxy-2-methylpropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine are reacted in analogy to method J. The title compound is obtained as a yellowish resin. Rf = 0.24 (EtOAc/heptane 2:1); Rt = 5.13 (gradient I).
c) 6-[(3R,4R,5S)-4-[4-((S)-3-Methoxy-2-methylpropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.92 g of 6-[(3R,4R,5S)-4-(4-chloromethylphenyl)-1-(toluene-4-sulphonyl)-5-triisopropylsilanyl-oxypiperidin-3-yloxymethyl]-4-(3-methoxypropyl)-3,4-dihydro-2H-benzo[1,4]oxazine (Example 42c) and 0.30 g of (R)-3-methoxy-2-methylpropan-1-ol (Example 43c) are reacted in analogy to Example 42b. The title compound is obtained as an orange oil. Rf = 0.26 (EtOAc/heptane 1:2).

### Example 46

### (3S,4S,5R)-5-[4-(3-Methoxypropyl)-3,4-dihydro-2H-benzo[1,4]oxazin-6-ylmethoxy]-4-[4-((R)-2-methyl-3-methylsulphanylpropoxymethyl)phenyl]piperidin-3-ol

The title compound is obtained from (3S,4S,5R)-5-[4-(3-methoxypropyl)-3,4-dihydro-2H-benzo[1,4]oxazin-6-ylmethoxy]-4-[4-((R)-2-methyl-3-methylsulphanylpropoxymethyl)phenyl]-1-(toluene-4-sulphonyl)piperidin-3-ol in analogy to method N.

The starting materials are prepared as follows:
a) (3S,4S,5R)-5-[4-(3-Methoxypropyl)-3,4-dihydro-2H-benzo[1,4]oxazin-6-ylmethoxy]-4-[4-((R)-2-methyl-3-methylsulphanylpropoxymethyl)phenyl]-1-(toluene-4-sulphonyl)piperidin-3-ol
   6-[(3R,4R,5S)-4-(4-Chloromethylphenyl)-1-(toluene-4-sulphonyl)-5-triisopropylsilanyloxy-piperidin-3-yloxymethyl]-4-(3-methoxypropyl)-3,4-dihydro-2H-benzo[1,4]oxazine (Example 42c) and (R)-2-methyl-3-methylsulphanylpropan-1-ol are reacted in analogy to Example 42b. The title compound is obtained as a yellow resin. Rf = 0.26 (EtOAc/heptane 1:1); Rt = 5.45 (gradient I).
b) (R)-2-Methyl-3-methylsulphanylpropan-1-ol
   2.55 g of triisopropyl-((R)-2-methyl-3-methylsulphanylpropoxy)silane are reacted in analogy to method J. The title compound is obtained as a yellowish oil. Rf = 0.14 (EtOAc/heptane 1:4).
c) Triisopropyl-((R)-2-methyl-3-methylsulphanylpropoxy)silane
   A solution of 2.06 g of sodium methanethiolate in 15 ml of ethanol is added dropwise to a solution of 6.05 g of ((R)-3-bromo-2-methylpropoxy)triisopropylsilane in 60 ml of tetrahydrofuran at room temperature. After 19 hours, the reaction mixture is diluted with diethyl ether and washed successively with water and brine, dried with sodium sulphate and evaporated. The crude title compound is obtained as yellowish oil from the residue. Rf = 0.18 (heptane).
d) ((R)-3-Bromo-2-methylpropoxy)triisopropylsilane
   1.51 g of imidazole and 4.26 ml of chlorotriisopropylsilane are added to a solution of 3.15 g of (R)-3-bromo-2-methylpropan-1-ol [93381-28-3] in 50 ml of dichloromethane at 0°C. After 18 hours at room temperature, the reaction mixture is quenched with 200 ml of 0.1 N HCl and extracted with diethyl ether (2×) - the combined organic phases are washed with water and brine, dried with sodium sulphate and evaporated. The crude title compound is obtained as a colourless liquid from the residue. Rf = 0.75 (EtOAc/heptane 1:10).

### Example 47

### (3S,4S,5R)-4-[4-((2R,3S)-3-Methoxy-2-methylbutoxymethyl)phenyl]-5-[4-(3-methoxypropyl)-3,4-dihydro-2H-benzo[1,4]oxazin-6-ylmethoxylpiperidin-3-ol

The title compound is prepared from 0.18 g of (3S,4S,5R)-4-[4-((2R,3S)-3-methoxy-2-methylbutoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol in analogy to method L.

The starting materials are prepared as follows:
a) (3S,4S,5R)-4-[4-((2R,3S)-3-Methoxy-2-methylbutoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-ol
   0.282 g of 6-[(3R,4R,5S)-4-[4-((2R,3S)-3-methoxy-2-methylbutoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine is reacted in analogy to method J. The title compound is obtained as a colourless oil. Rf = 0.23 (EtOAc/heptane 2:1); Rt = 5.25 (gradient I).
b) 6-[(3R,4R,5S)-4-[4-((2R,3S)-3-Methoxy-2-methylbutoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.027 g of sodium hydride (60% dispersion in oil) is added to a solution of 0.278 g of (2S,3R)-4-{4-[(3R,4R,5S)-3-[4-(3-methoxypropyl)-3,4-dihydro-2H-benzo[1,4]oxazin-6-ylmethoxy]-1-(toluene-4-sulphonyl)-5-triisopropylsilanyloxypiperidin-4-yl]-benzyloxy}-3-methylbutan-2-ol and 0.237 g of methyl iodide in 5 ml of tetrahydrofuran at 0°C. After 6 hours at room temperature, the reaction mixture is diluted with 230 ml of tert-butyl methyl ether and washed successively with 70 ml of saturated aqueous sodium bicarbonate solution, 30 ml of water and 50 ml of brine, dried with sodium sulphate and evaporated. The title compound is obtained as a yellowish oil from the residue by flash chromatography (SiO₂ 60F). Rf = 0.54 (EtOAc/heptane 1:1).
c) (2S,3R)-4-{4-[(3R,4R,5S)-3-[4-(3-Methoxypropyl)-3,4-dihydro-2H-benzo[1,4]oxazin-6-ylmethoxy]-1-(toluene-4-sulphonyl)-5-triisopropylsilanyloxypiperidin-4-yl]benzyloxy}-3-methylbutan-2-ol
   1.04 g of magnesium bromide diethyl etherate complex are added to a solution of 1.46 g of 4-(3-methoxypropyl)-6-[(3R,4R,5S)-4-{4-[(2R,3S)-2-methyl-3-(tetrahydropyran-2-yloxy)-butoxymethyl]phenyl}-1-(toluene-4-sulphonyl)-5-triisopropylsilanyloxypiperidin-3-yloxymethyl]-3,4-dihydro-2H-benzo[1,4]oxazine in 24 ml of diethyl ether. After 2 hours, a further 0.5 g of magnesium complex is added. The reaction mixture is stirred vigorously at room temperature for 20 hours and is then quenched at 0°C successively with 20 ml of saturated aqueous sodium bicarbonate solution and 50 ml of water. The mixture is extracted with 300 ml of ethyl acetate. The organic phase is washed successively with 40 ml of water and 40 ml of brine, dried with sodium sulphate and evaporated. The title compound is obtained as a colourless oil from the residue by flash chromatography (SiO₂ 60F). Rf = 0.22 (EtOAc/heptane 1:1).
d) 4-(3-Methoxypropyl)-6-[(3R,4R,5S)-4-[4-[(2R,3S)-2-methyl-3-(tetrahydropyran-2-yloxy)-butoxymethyl]phenyl}-1-(toluene-4-sulphonyl)-5-triisopropylsilanyloxypiperidin-3-yloxymethyl]-3,4-dihydro-2H-benzo[1,4]oxazine
   1.50 g of 6-[(3R,4R,5S)-4-(4-chloromethylphenyl)-1-(toluene-4-sulphonyl)-5-triisopropyl-silanyloxypiperidin-3-yloxymethyl]-4-(3-methoxypropyl)-3,4-dihydro-2H-benzo[1,4]oxazine (Example 42c) and 0.549 g of (2R,3S)-2-methyl-3-(tetrahydropyran-2-yloxy)butan-1-ol are reacted in analogy to Example 42b. The title compound is obtained as a colourless oil. Rf = 0.21 (EtOAc/heptane 1:2).
e) (2R,3S)-2-Methyl-3-(tetrahydropyran-2-yloxy)butan-1-ol
   A solution of 2.29 g of methyl (2S,3S)-2-methyl-3-(tetrahydropyran-2-yloxy)butane-carboxylate in 15 ml of diethyl ether is added dropwise to a suspension of 0.804 g of lithium aluminium hydride in 20 ml of diethyl ether. The reaction solution is then stirred at 0°C for 2 hours. The solution is hydrolysed at 0°C successively with 1.3 ml of water and with 1.3 ml of 1 N sodium hydroxide solution. The hydrolysed solution is stirred at 0°C for 1 hour and then filtered through Celite and concentrated. The title compound is obtained as a colourless oil from the residue by flash chromatography (SiO₂ F60). Rf = 0.20 and 0.11 (diastereomers of the protective group) (EtOAc/heptane 1:2).
f) Methyl (2S,3S)-2-methyl-3-(tetrahydropyran-2-yloxy)butanecarboxylate
   4.455 g of 3,4-2H-dihydropyran and 0.027 g of pyridinium p-toluenesulphonate are successively added to a solution of 1.40 g of methyl (2S,3S)-3-hydroxy-2-methylbutane-carboxylate [66767-60-0] in 50 ml of dichloromethane. After 15 hours, the reaction mixture is concentrated. The residue is taken up in 50 ml of diethyl ether, and the white precipitate is filtered off. The filtrate is evaporated and the crude title compound is obtained as a colourless oil. Rf = 0.50 (EtOAc/heptane 1:2).

The following compound is prepared in an analogous manner to the process described in Example 47:

### Example

- 48: (3S,4S,5R)-4-[4-((2R,3S)-3-Ethoxy-2-methylbutoxymethyl)phenyl]-5-[4-(3-methoxypropyl)-3,4-dihydro-2H-benzo[1,4]oxazin-6-ylmethoxy]piperidin-3-ol

### Example 49

### 6-{(3R,4S,5S)-4-[4-((S)-3-Methoxy-2-methylpropoxymethyl)pheny]-5-[2-(1H-tetrazol-5-yl)ethoxy]piperidin-3-yloxymethyl}-4-(3-methoxypropyl)-3,4-dihydro-2H-benzo[1,4]oxazine

The title compound is prepared from 0.286 g of 6-[(3R,4S,5S)-4-[4-((S)-3-methoxy-2-methylpropoxymethyl)phenyl]-5-[2-(1 H-tetrazol-5-yl)ethoxy]-1-(toluene-4-sulphonyl)piperidin-3-yloxymethyl]-4-(3-methoxypropyl)-3,4-dihydro-2H-benzo[1,4]oxazine in analogy to method L.

The starting materials are prepared as follows:
a) 6-[(3R,4S,5S)-4-[4-((S)-3-Methoxy-2-methylpropoxymethyl)phenyl]-5-[2-(1H-tetrazol-5-yl)ethoxy]-1-(toluene-4-sulphonyl)piperidin-3-yloxymethyl]-4-(3-methoxypropyl)-3,4-dihydro-2H-benzo[1,4]oxazine
   0.033 g of dibutyltin oxide is added to a solution of 0.345 g of 3-[(3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propionitrile and 1.76 g of trimethylsilyl azide in 7 ml of toluene. After 14 hours at 100°C, the reaction solution is quenched with 10 ml of 1 N HCI at room temperature. The mixture is extracted three times with 70 ml of ethyl acetate. The combined organic phases are washed with brine, dried with sodium sulphate and evaporated. The title compound is obtained as a brown oil from the residue by flash chromatography (SiO₂ 60F). Rf = 0.29 (EtOAc/methanol 10: 1); Rt = 4.93 (gradient I).
b) 3-[(3S,4S,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-yloxy]propionitrile
   0.365 g of acrylonitrile is added to a solution of 0.47 g of (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 43a) and 0.105 g of 2,3,4,6,7,8,9,10-octahydropyrimido[1,2-a]azepine (DBU) in 3.5 ml of acetonitrile. After 18 hours at 50°C, 0.105 g of DBU and 0.365 g of acrylonitrile are again added to the reaction solution. After 62 hours, the reaction mixture is evaporated. The title compound is obtained as a colourless oil from the residue by flash chromatography (SiO₂ 60F). Rf = 0.22 (EtOAc/heptane 1:1); Rt = 5.43 (gradient I).

### Example 50

### (S)-1-Methoxy-3-{(3S,4R,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.28 g of (S)-1-methoxy-3-[(3S,4R,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting material is prepared as follows:
a) (S)-1-Methoxy-3-[(3S,4R,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol
   0.335 g of (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 43a) and 0.097 g of R-(-)-glycidyl methyl ether [64491-70-9] are reacted in analogy to method M. The title compound is obtained as a cloudy oil. Rf = 0.28 (EtOAc/heptane 2:1); Rt = 5.25 (gradient I).

The following compound is prepared in an analogous manner to the process described in Example 50:

### Example

- 151: (R)-1-Methoxy-3-{(3S,4R,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]piperidin-3-yloxy}propan-2-ol

### Example 52

### (R)-1-{(3S,4R,5R)-4-(4-Cyclopropylmethoxymethylphenyl)-5-[4-(3-methoxypropyl)-3,4-dihydro-2H-benzo[1,4]oxazin-6-ylmethoxylpiperidin-3-yloxy}-3-methoxypropan-2-ol

The title compound is prepared from 0.455 g of (R)-1-[(3S,4R,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-yloxy]-3-methoxypropan-2-ol in analogy to method L.

The starting materials are prepared as follows:
a) (R)-1-[(3S,4R,5R)-4-(4-Cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-yloxy]-3-methoxypropan-2-ol
   1.0 g of (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol and 0.290 g of S-(+)-glycidyl methyl ether [64491-68-5] are reacted in analogy to method M. The title compound is obtained as a beige oil. Rf = 0.32 (EtOAc/heptane 2:1); Rt = 5.25 (gradient I).
b) (3S,4S,5R)-4-(4-Cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol
   5.0 g of 6-[(3R,4R,5S)-4-(4-cyclopropylmethoxymethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine are reacted in analogy to method J. The title compound is obtained as a white foam. Rf = 0.18 (EtOAc/heptane 1:1); Rt = 5.14 (gradient I).
c) 6-[(3R,4R,5S)-4-(4-Cyclopropylmethoxymethylphenyl)-1-(toluene-4-sulphonyl)-5-triisopropylsilanyloxypiperidin-3-yloxymethyl]-4-(3-methoxypropyl)-3,4-dihydro-2H-benzo-[1,4]oxazine
   0.544 g of sodium hydride (60% dispersion in oil) is added to a stirred solution of 0.90 g of cyclopropylmethanol in 6 ml of N,N-dimethylformamide at -10°C. After 10 minutes, a solution of 5.30 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) in 8 ml of tetrahydrofuran is added, and the mixture is then stirred at 0°C for 3 hours. The reaction mixture is poured into 1 M sodium bicarbonate solution (150 ml) and extracted with tert-butyl methyl ether (2 × 150 ml). The organic phases are washed with water (150 ml) and brine (150 ml), dried with sodium sulphate and evaporated. The title compound is obtained as a yellow oil from the residue by flash chromatography (SiO₂ 60F). Rf = 0.46 (EtOAc/heptane 1:1).

The following compound is prepared in an analogous manner to the process described in Example 52:

### Example

- 53: (S)-1-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-3-methoxypropan-2-ol

### Example 54

### (R)-1-{(3S,4R,5R)-4-(4-Ethylphenyl)-5-[4-(3-methoxypropyl)-3,4-dihydro-2H-benzo-[1,4]oxazin-6-ylmethoxy]piperidin-3-yloxy}-3-methoxypropan-2-ol

The title compound is prepared from 0.11 g of (R)-1-[(3S,4R,5R)-4-(4-ethylphenyl)-5-[4-(3-methoxypropyl)-3,4-dihydro-2H-benzo[1,4]oxazin-6-ylmethoxy]-1-(toluene-4-sulphonyl)-piperidin-3-yloxy]-3-methoxypropan-2-ol in analogy to method L.

The starting materials are prepared as follows:
a) (R)-1-[(3S,4R,5R)-4-(4-Ethylphenyl)-5-[4-(3-methoxypropyl)-3,4-dihydro-2H-benzo-[1,4]oxazin-6-ylmethoxy]-1-(toluene-4-sulphonyl)piperidin-3-yloxy]-3-methoxypropan-2-ol
   0.18 g of (3S,4S,5R)-4-(4-ethylphenyl)-5-[4-(3-methoxypropyl)-3,4-dihydro-2H-benzo-[1,4]oxazin-6-ylmethoxy]-1-(toluene-4-sulphonyl)piperidin-3-ol and 0.058 g of S-(+)-glycidyl methyl ether [64491-68-5] are reacted in analogy to method M. The title compound is obtained as a colourless oil. Rf = 0.17 (EtOAc/heptane 1:1); Rt = 5.30 (gradient I).
b) (3S,4S,5R)-4-(4-Ethylphenyl)-5-[4-(3-methoxypropyl)-3,4-dihydro-2H-benzo[1,4]oxazin-6-ylmethoxy]-1-(toluene-4-sulphonyl)piperidin-3-ol
   0.233 g of 6-[(3R,4R,5S)-4-(4-ethylphenyl)-1-(toluene-4-sulphonyl)-5-triisopropylsilanyloxy-piperidin-3-yloxymethyl]-4-(3-methoxypropyl)-3,4-dihydro-2H-benzo[1,4]oxazine is reacted in analogy to method J. The title compound is obtained as a colourless resin. Rf = 0.32 (EtOAc/heptane 1:1); Rt = 5.20 (gradient I).
c) 6-[(3R,4R,5S)-4-(4-Ethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.274 g of 6-[(3R,4R,5S)-4-(4-ethylphenyl)-1-(toluene-4-sulphonyl)-5-triisopropylsilanyloxypiperidin-3-yloxymethyl]-4-(3-methoxypropyl)-4H-benzo[1,4]oxazin-3-one is reacted in analogy to method K. The title compound is obtained as a colourless oil. Rf = 0.34 (EtOAc/heptane 1:2).
d) 6-[(3R,4R,5S)-4-(4-Ethylphenyl)-1-(toluene-4-sulphonyl)-5-triisopropylsilanyloxypiperidin-3-yloxymethyl]-4-(3-methoxypropyl)-4H-benzo[1,4]oxazin-3-one
   0.483 g of (3R,4R,5S)-4-(4-ethylphenyl)-1-(toluene-4-sulphonyl)-5-triisopropylsilanyloxypiperidin-3-ol and 0.282 g of 6-bromomethyl-4-(3-methoxypropyl)-4H-benzo[1,4]oxazin-3-one are reacted in analogy to method D. The title compound is obtained as a colourless oil. Rf = 0.38 (EtOAc/heptane 1:2).
e) (3R,4R,5S)-4-(4-Ethylphenyl)-1-(toluene-4-sulphonyl)-5-triisopropylsilanyloxypiperidin-3-ol
   0.49 g of (3R,4R,5S)-4-(4-ethylphenyl)-5-triisopropylsilanyloxypiperidin-3-ol and 0.223 g of toluenesulphonyl chloride are reacted in analogy to Example 42d. The title compound is obtained as a colourless oil. Rf = 0.09 (EtOAc/heptane 1:10); Rt = 6.85 (gradient I).
f) (3R,4R,5S)-4-(4-Ethylphenyl)-5-triisopropylsilanyloxypiperidin-3-ol
   0.62 g of benzyl (3R,4R,5S)-3-hydroxy-5-triisopropylsilanyloxy-4-(4-vinylphenyl)piperidine-1-carboxylate in 10 ml of methanol are reacted in analogy to method B. The title compound is obtained as a colourless oil. Rt = 5.24 (gradient I).
g) Benzyl (3R,4R,5S)-3-hydroxy-5-triisopropylsilanyloxy-4-(4-vinylphenyl)-piperidine-1-carboxylate
   0.639 g of 2-vinyl-4,4,5,5-tetramethyl-1,3,2-dioxaborolane and 0.409 g of potassium carbonate are successively added to a solution of 1.246 g of benzyl (3R,4R,5S)-3-hydroxy-4-(4-trifluoromethanesulphonyloxyphenyl)-5-triisopropylsilanyloxypiperidine-1-carboxylate (Example 42j) in 10 ml of dioxane in a Schlenk tube. The mixture is briefly degassed and 0.184 g of tetrakis-(triphenylphosphine)palladium(0) complex is also added. After 14 hours at 85°C, a further 0.32 g of 2-vinyl-4,4,5,5-tetramethyl-1,3,2-dioxaborolane and 0.09 g of Pd(0) complex are added at room temperature. After 24 hours at 95°C, the reaction mixture is cooled at room temperature, diluted with 200 ml of tert-butyl methyl ether and washed successively with 40 ml of water and 20 ml of brine. The organic phase is dried with sodium sulphate and evaporated. The title compound is obtained as a brown oil from the residue by flash chromatography (SiO₂ 60F). Rf = 0.12 (EtOAc/heptane 1:4); Rt = 6.54 (gradient I).

### Example 55

### (R)-1-Methoxy-3-[(3S,4R,5R)-5-[4-(3-methoxypropyl)-3,4-dihydro-2H-benzo[1,4]oxazin-6-ylmethoxy]-4-(4-propylphenyl)piperidin-3-yloxy]propan-2-ol

The title compound is prepared from 0.117 g of benzyl (3S,4R,5R)-3-((R)-2-hydroxy-3-methoxypropoxy)-5-[4-(3-methoxypropyl)-3,4-dihydro-2H-benzo[1,4]oxazin-6-ylmethoxy]-4-(4-propylphenyl)piperidine-1-carboxylate in analogy to method B.

The starting materials are prepared as follows:
a) Benzyl (3S,4R,5R)-3-((R)-2-hydroxy-3-methoxypropoxy)-5-[4-(3-methoxypropyl)-3,4-dihydro-2H-benzo[1,4]oxazin-6-ylmethoxy]-4-(4-propylphenyl)piperidine-1-carboxylate
   0.299 g of benzyl (3S,4S,5R)-3-hydroxy-5-[4-(3-methoxypropyl)-3,4-dihydro-2H-benzo-[1,4]oxazin-6-ylmethoxy]-4-(4-propylphenyl)piperidine-1-carboxylate and 0.101 g of (S)-(+)-glycidyl methyl ether [64491-68-5] are reacted in analogy to method M. The title compound is obtained as a colourless oil. Rf = 0.21 (EtOAc/heptane 1:1); Rt = 5.50 (gradient I).
b) Benzyl (3S,4S,5R)-3-hydroxy-5-[4-(3-methoxypropyl)-3,4-dihydro-2H-benzo[1,4]oxazin-6-ylmethoxy]-4-(4-propylphenyl)piperidine-1-carboxylate
   0.423 g of benzyl (3R,4R,5S)-3-[4-(3-methoxypropyl)-3,4-dihydro-2H-benzo[1,4]oxazin-6-ylmethoxy]-4-(4-propylphenyl)-5-triisopropylsilanyloxypiperidine-1-carboxylate is reacted in analogy to method J. The title compound is obtained as a colourless oil. Rf = 0.20 (EtOAc/heptane 1:2); Rt = 5.38 (gradient I).
c) Benzyl (3R,4R,5S)-3-[4-(3-methoxypropyl)-3,4-dihydro-2H-benzo[1,4]oxazin-6-yl-methoxy]-4-(4-propylphenyl)-5-triisopropylsilanyloxypiperidine-1-carboxylate
   1.3 ml of propylmagnesium bromide (2N solution in THF) are added to a solution of 1.75 g of benzyl (3R,4R,5S)-3-[4-(3-methoxypropyl)-3,4-dihydro-2H-benzo[1,4]oxazin-6-ylmethoxy]-4-(4-trifluoromethanesulphonyloxyphenyl)-5-triisopropylsilanyloxypiperidine-1-carboxylate, 0.037 g of iron(III) acetylacetonate and 5 ml of N-methylpyrrolidon in 50 ml of tetrahydrofuran in a Schlenk tube. After 1 hour at room temperature, a further 0.037 g of iron(III) complex and 1.3 ml of propylmagnesium bromide are added. After 48 hours, the reaction mixture is diluted with tert-butyl methyl ether and quenched with 1 N aqueous HCl. The organic phase is separated and washed successively with water and brine, dried with sodium sulphate and evaporated. The title compound is obtained as a yellowish resin from the residue by flash chromatography (SiO₂60F). Rf = 0.13 (EtOAc/heptane 1:10).
d) Benzyl (3R,4R,5S)-3-[4-(3-methoxypropyl)-3,4-dihydro-2H-benzo[1,4]oxazin-6-ylmethoxy]-4-(4-trifluoromethanesulphonyloxyphenyl)-5-triisopropylsilanyloxypiperidine-1-carboxylate
   6.53 g of benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate and 3.41 g of N-phenyltrifluoromethanesulphonimide in analogy to Example 42j. The title compound is obtained as a reddish oil. Rf = 0.61 (EtOAc/heptane 1:1).
e) Benzyl (3R,4R,5S)-4-(4-hydroxyphenyl)-3-[4-(3-methoxypropyl)-3,4-dihydro-2H-benzo-[1,4]oxazin-6-ylmethoxy]-5-triisopropylsilanyloxypiperidine-1-carboxylate
   10.2 g of benzyl (3R,4R,5S)-4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate are reacted in analogy to method K. The title compound is obtained as a colourless oil. Rf = 0.36 (EtOAc/heptane 1:1).
f) Benzyl (3R,4R,5S)-4-(4-Hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate
   1.45 g of tetrakis(tirphenylphosphine)palladium(0) complex are added to a mixture of 12.58 g of benzyl (3R,4R,5S)-4-(4-allyloxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate and 6.33 g of potassium carbonate in 100 ml of methanol. After 5 hours at room temperature, the reaction mixture is filtered and the filtrate is evaporated. The title compound is obtained as a yellowish resin from the residue by flash chromatography (SiO₂ 60F). Rf = 0.25 (EtOAc/heptane 1:1); Rt = 6.39 (gradient I).
g) Benzyl (3R,4R,5S)-4-(4-allyloxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate
   10.1 g of benzyl (3R,4R,5S)-4-(4-allyloxyphenyl)-3-hydroxy-5-triisopropylsilanyloxypiperidine-1-carboxylate and 5.55 g of 6-chloromethyl-4-(3-methoxypropyl)-4H-benzo[1,4]oxazin-3-one (Example 420) are reacted in analogy to method D. The title compound is obtained as a yellowish oil. Rf = 0.43 (EtOAc/heptane 1:1); Rt = 7.13 (gradient I).
h) Benzyl (3R,4R,5S)-4-(4-allyloxyphenyl)-3-hydroxy-5-triisopropylsilanyloxypiperidine-1-carboxylate
   76.2 g of benzyl (3R,4R,5S)-3-hydroxy-4-(4-hydroxyphenyl)-5-triisopropylsilanyloxypiperidine-1-carboxylate (Example 42k) and 30.22 g of allyl bromide are reacted at 60°C in analogy to method F. The title compound is obtained as a yellowish resin. Rf = 0.33 (EtOAc/heptane 1:2); Rt = 6.59 (gradient I).

The following compound is prepared in an analogous manner to the process described in Example 55:

### Example

- 56: (R)-1-{(3S,4R,5R)-4-(4-Butylphenyl)-5-[4-(3-methoxypropyl)-3,4-dihydro-2H-benzo[1,4]oxazin-6-ylmethoxy]piperidin-3-yloxy}-3-methoxypropan-2-ol

### Example 57

### (R)-1-{(3S,4R,5R)-4-(4-Ethoxymethylphenyl)-5-[4-(3-methoxypropyl)-3,4-dihydro-2H-benzo[1,4]oxazin-6-ylmethoxy]piperidin-3-yloxy}-3-methoxypropan-2-ol

The title compound is prepared from 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and ethanol in analogy to the process described in Example 50 and Example 43.

### Example 58

### (R)-1-Methoxy-3-{(3S,4R,5R)-4-(4-methoxymethylphenyl)-5-[4-(3-methoxypropyl)-3,4-dihydro-2H-benzo[1,4]oxazin-6-ylmethoxy]piperidin-3-yloxy}propan-2-ol

The title compound is prepared from 0.28 g of (R)-1-methoxy-3-[(3S,4R,5R)-4-(4-methoxymethylphenyl)-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting materials are prepared as follows:
a) (R)-1-Methoxy-3-[(3S,4R,5R)-4-(4-methoxymethylphenyl)-5-[4-(3-methoxypropyl)-3,4-dihydro-2H-benzo[1,4]oxazin-6-ylmethoxy]-1-(toluene-4-sulphonyl)piperidin-3-yloxy]-propan-2-ol
   0.370 g of (3S,4S,5R)-4-(4-methoxymethylphenyl)-5-[4-(3-methoxypropyl)-3,4-dihydro-2H-benzo[1,4]oxazin-6-ylmethoxy]-1-(toluene-4-sulphonyl)piperidin-3-ol and 0.119 g of S-(+)-glycidyl methyl ether [64491-68-5] are reacted in analogy to method M. The title compound is obtained as a colourless resin. Rf = 0.06 (EtOAc/heptane 1:1); Rt = 4.84 (gradient I).
b) (3S,4S,5R)-4-(4-Methoxymethylphenyl)-5-[4-(3-methoxypropyl)-3,4-dihydro-2H-benzo[1,4]oxazin-6-ylmethoxy]-1-(toluene-4-sulphonyl)piperidin-3-ol
   The title compound is prepared from 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and methanol in analogy to the process described in Example 52. The title compound is obtained as a grey resin. Rf = 0.11 (EtOAc/heptane 1:1); Rt = 4.74 (gradient I).

### Example 59

### (R)-1-Methoxy-3-{(3S,4R,5R)-4-[4-(2-methoxyethoxymethyl)phenyl]-5-{4-(3-methoxypropyl)-3,4-dihydro-2H-benzo[1,4]oxazin-6-ylmethoxylpiperidin-3-yloxy}propan-2-ol

The title compound is prepared from 0.17 g of (R)-1-methoxy-3-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting materials are prepared as follows:
a) (R)-1-Methoxy-3-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol
   0.260 g of (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol and 0.078 g of S-(+)-glycidyl methyl ether [64491-68-5] are reacted in analogy to method M. The title compound is obtained as a colourless resin. Rf = 0.23 (EtOAc/heptane 4:1); Rt = 4.76 (gradient I).
b) (3S,4S,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   0.380 g of 6-[(3R,4R,5S)-4-[4-(2-methoxyethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine is reacted in analogy to method J. The title compound is obtained as a yellow oil. Rf = 0.35 (EtOAc/heptane 4:1); Rt = 4.62 (gradient I).
c) 6-[(3R,4R,5S)-4-[4-(2-Methoxyethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.026 g of sodium hydride (60% dispersion in oil) is added to a solution of 0.40 g of {4-[(3R,4R,5S)-3-[4-(3-methoxypropyl)-3,4-dihydro-2H-benzo[1,4]oxazin-6-ylmethoxy]-1-(toluene-4-sulphonyl)-5-triisopropylsilanyloxypiperidin-4-yl]phenyl}methanol (Example 42d), 0.11 g of 2-bromoethyl methyl ether and 0.19 g of tetrabutylammonium iodide in 2 ml of N,N-dimethylformamide at -5°C and stirred at room temperature for 18 hours. The reaction mixture is poured into ice-water and extracted with tert-butyl methyl ether. The organic phases are washed with water and brine, dried with sodium sulphate and evaporated. The title compound is obtained as a yellow oil from the residue by flash chromatography (SiO₂ 60F). Rf = 0.4 (EtOAc/heptane 1:1).

The following compounds are prepared in an analogous manner to the process described in Example 59:

### Examples

- 111: (S)-1-Methoxy-3-{(3S,4R,5R)-4-[4-(3-methoxypropoxymethyl)phenyl]-5-[4-(3-methoxypropyl)-3,4-dihydro-2H-benzo[1,4]oxazin-6-ylmethoxy]piperidin-3-yloxy}-propan-2-ol

Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a).
- 112: (R)-1-Methoxy-3-{(3S,4R,5R)-4-[4-(3-methoxypropoxymethyl)phenyl]-5-[4-(3-methoxypropyl)-3,4-dihydro-2H-benzo[1,4]oxazin-6-ylmethoxy]piperidin-3-yloxy}-propan-2-ol

Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a).

### Example 60

### (R)-1-Methoxy-3-{(3S,4R,5R)-4-[4-(2-methoxyethyl)phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.08 g of (R)-1-methoxy-3-[(3S,4R,5R)-4-[4-(2-methoxyethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting materials are prepared as follows:
a) (R)-1-Methoxy-3-[(3S,4R,5R)-4-[4-(2-methoxyethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol
   0.106 g of (3S,4S,5R)-4-[4-(2-methoxyethyl)phenyl]-5-[4-(3-methoxypropyl)-3,4-dihydro-2H-benzo[1,4]oxazin-6-ylmethoxy]-1-(toluene-4-sulphonyl)piperidin-3-ol and 0.032 g of (S)-(+)-glycidyl methyl ether [64491-68-5] are reacted in analogy to method M. The title compound is obtained as a colourless oil. Rf = 0.17 (EtOAc/heptane 2:1); Rt = 4.88 (gradient I).
b) (3S,4S,5R)-4-[4-(2-Methoxyethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   0.155 g of 6-[(3R,4R,5S)-4-[4-(2-methoxyethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine is reacted in analogy to method J. The title compound is obtained as a colourless resin. Rf = 0.11 (EtOAc/heptane 1:1); Rt = 4.79 (gradient I).
c) 6-[(3R,4R,5S)-4-[4-(2-Methoxyethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.032 g of sodium hydride (60% dispersion in oil) is added to a solution of 0.261 g of 2-{4-[(3R,4R,5S)-3-[4-(3-methoxypropyl)-3,4-dihydro-2H-benzo[1,4]oxazin-6-ylmethoxy]-1-(toluene-4-sulphonyl)-5-triisopropylsilanyloxypiperidine-4-yl]phenyl}ethanol and 0.232 ml of methyl iodide in 1 ml of N,N-dimethylformamide and 3 ml of tetrahydrofuran. After 6 hours at room temperature, the reaction mixture is diluted with 250 ml of tert-butyl methyl ether and washed successively with 50 ml of saturated sodium bicarbonate solution, 50 ml of water and 30 ml of brine, dried with sodium sulphate and evaporated. The title compound is obtained as a yellow oil from the residue by flash chromatography (SiO₂ 60F). Rf = 0.60 (EtOAc/heptane 1:1).
d) 2-{4-[(3R,4R,5S)-3-[4-(3-Methoxypropyl)-3,4-dihydro-2H-benzo[1,4]oxazin-6-ylmethoxy]-1-(toluene-4-sulphonyl)-5-triisopropylsilanyloxypiperidin-4-yl]phenyl}ethanol
   2.6 ml of diisobutylaluminium hydride (1N solution in dichloromethane) are added dropwise to a solution of 1.45 g of {4-[(3R,4R,5S)-3-[4-(3-methoxypropyl)-3,4-dihydro-2H-benzo[1,4]oxazin-6-ylmethoxy]-1-(toluene-4-sulphonyl)-5-triisopropylsilanyloxypiperidin-4-yl]phenyl}acetonitrile in 15 ml of dichloromethane at -78°C. After 30 minutes at -78°C, the reaction mixture is stirred at room temperature for 2 hours and then quenched successively with 1 N aqueous ammonium chloride solution and with 1 N aqueous HCl (pH 2). The mixture is extracted twice with 100 ml of tert-butyl methyl ether. The combined organic phases are washed with 30 ml of water and then 20 ml of brine, dried with sodium sulphate and evaporated. The residue is dissolved in 20 ml of tetrahydrofuran and, at 0°C, 2.88 ml of borane-THF complex (1M solution in tetrahydrofuran) are added. After 2 hours, 50 ml of methanol are cautiously added at 0°C, and the mixture is evaporated. The title compound is obtained as a yellowish oil from the residue by flash chromatography (SiO₂ 60F). Rf = 0.21 (EtOAc/heptane 1:1).
e) {4-[(3R,4R,5S)-3-[4-(3-Methoxypropyl)-3,4-dihydro-2H-benzo[1,4]oxazin-6-ylmethoxy]-1-(toluene-4-sulphonyl)-5-triisopropylsilanyloxypiperidin-4-yl]phenyl}acetonitrile
   0.072 g of tetrabutylammonium cyanide, 0.069 g of 18-crown-6 and 0.258 g of potassium cyanide are added to a solution of 2.0 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) in 20 ml of tetrahydrofuran and 3 ml of acetonitrile. After 2 hours at 50°C, the reaction mixture is diluted at room temperature with 250 ml of tert-butyl methyl ether. The mixture is washed successively with 20 ml of saturated sodium bicarbonate solution, 20 ml of water and 20 ml of brine, dried with sodium sulphate and evaporated. The title compound is obtained as a colourless oil from the residue by flash chromatography (SiO₂ 60F). Rf = 0.25 (EtOAc/heptane 1:2).

### Example 61

### 6-[(3R,4S,5S)-4-[4-(2-Methoxyethoxymethyl)phenyl]-5-(3-methoxypropoxy)piperidin-3-yloxymethyl]-4-(3-methoxypropyl)-3,4-dihydro-2H-benzo[1,4]oxazine

The title compound is prepared from 0.281 mmol of 6-[(3R,4S,5S)-4-[4-(2-methoxyethoxymethyl)phenyl]-5-(3-methoxypropoxy)-1-(toluene-4-sulphonyl)piperidin-3-yloxymethyl]-4-(3-methoxypropyl)-3,4-dihydro-2H-benzo[1,4]oxazine in analogy to method L.

The starting material is prepared as follows:
a) 6-[(3R,4S,5S)-4-[4-(2-Methoxyethoxymethyl)phenyl]-5-(3-methoxypropoxy)-1-(toluene-4-sulphonyl)piperidin-3-yloxymethyl]-4-(3-methoxypropyl)-3,4-dihydro-2H-benzo[1,4]oxazine
   0.83 mmol of sodium hydride (60% dispersion in oil) is added to a solution of 0.55 mmol of (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b), 0.69 mmol of 1-bromo-3-methoxypropane and 0.055 mmol of sodium iodide in 2 ml of N,N-dimethylformamide at -5°C, and the mixture is stirred at room temperature for 4 hours. the reaction mixture is poured into ice-water and extracted with tert-butyl methyl ether (3×). The combined organic phases are washed with water and brine, dried with sodium sulphate and evaporated. The title compound is obtained as a yellow oil from the residue by flash chromatography (SiO₂ 60F). Rf = 0.26 (EtOAc/heptane 3:1); Rt = 5.30 (gradient I).

### Example 62

### 6-[(3R,4S,5S)-4-[4-((S)-3-Methoxy-2-methylpropoxymethyl)phenyl]-5-(3-methoxypropoxy)-piperidin-3-yloxymethyl]-4-(3-methoxypropyl)-3,4-dihydro-2H-benzo[1,4]oxazine

The title compound is prepared from 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) in analogy to the process described in Example 61 and Example 43.

### Example 63

### 6-[(3R,4S,5S)-4-(4-Cyclopropylmethoxymethylphenyl)-5-(3-methoxypropoxy)piperidin-3-yloxymethyl]-4-(3-methoxypropyl)-3,4-dihydro-2H-benzo[1,4]oxazine

The title compound is prepared from 0.247 g of 6-[(3R,4S,5S)-4-(4-cyclopropylmethoxymethylphenyl)-5-(3-methoxypropoxy)-1-(toluene-4-sulphonyl)piperidin-3-yloxymethyl]-4-(3-methoxypropyl)-3,4-dihydro-2H-benzo[1,4]oxazine in analogy to method L.

The starting material is prepared as follows:
a) 6-[(3R,4S,5S)-4-(4-Cyclopropylmethoxymethylphenyl)-5-(3-methoxypropoxy)-1-(toluene-4-sulphonyl)piperidin-3-yloxymethyl]-4-(3-methoxypropyl)-3,4-dihydro-2H-benzo[1,4]oxazine
   31 mg of sodium hydride (60% dispersion in oil) are added to a solution of 0.35 g of (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 52b), 0.133 g 1-bromo-3-methoxypropane and 8 mg of sodium iodide in 2 ml of N,N-dimethylformamide at -5°C, and the mixture is stirred at room temperature for 4 hours. The reaction mixture is poured into ice-water and extracted with tert-butyl methyl ether. The organic phases are washed with water and brine, dried with sodium sulphate and evaporated. The title compound is obtained as a colourless oil from the residue by flash chromatography (SiO₂ 60F). Rf = 0.23 (EtOAc/heptane 3:1); Rt = 5.73 (gradient I).

### Example 64

### (S)-1-Methoxy-3-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.208 g of (S)-1-methoxy-3-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting material is prepared as follows:
a) (S)-1-Methoxy-3-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-yloxy]propan-2-ol
   0.20 g of (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b) and 0.060 g of (R)-(-)-glycidyl methyl ether [64491-70-9] are reacted in analogy to method M. The title compound is obtained as a yellow oil. Rf = 0.05 (EtOAc/heptane 2:1); Rt = 4.76 (gradient I).

### Example 65

### 6-[(3R,4R,5S)-4-[4-(2-Methoxyethoxymethyl)phenyl]-5-(2-[1,2,4]triazol-1-yl-ethoxy)piperidin-3-yloxymethyl]-4-(3-methoxypropyl)-3,4-dihydro-2H-benzo[1,4]oxazine

The title compound is prepared from 0.180 g of benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(2-[1,2,4]triazol-1-yl-ethoxy)piperidine-1-carboxylate in analogy to method B.

The starting materials are prepared as follows:
a) Benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(2-[1,2,4]triazol-1-yl-ethoxy)piperidine-1-carboxylate
   0.146 g of 1,2,4-triazole sodium salt [41253-21-8] is added to a solution of 0.240 g of benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-[2-(toluene-4-sulphonyloxy)ethoxy]piperidine-1-carboxylate in 6 ml of N,N-dimethylformamide at 0°C, and the mixture is then stirred at room temperature for 4 hours. The reaction mixture is poured into ice-water and extracted with tert-butyl methyl ether. The organic phases are washed with water and brine, dried with sodium sulphate and evaporated. The title compound is obtained as a colourless oil from the residue by flash chromatography (SiO₂ 60F). Rf = 0.40 (dichloromethane/methanol/25% conc. ammonia = 200:20:1); Rt = 4.49 (gradient I).
b) (3R,4R,5S)-4-[4-(2-Methoxyethoxymethyl)phenyl]-3-[4-(3-methoxypropyl)-3,4-dihydro-2H-benzo[1,4]oxazin-6-ylmethoxy]-5-[2-(toluene-4-sulphonyloxy)ethoxy]piperidine-1-carboxylate
   0.815 g of benzyl (3S,4R,5R)-3-(2-hydroxyethoxy)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate is reacted in analogy to method H. The title compound is obtained as a yellowish oil. Rf = 0.16 (EtOAc/heptane 2:1); Rt = 5.51 (gradient I).
c) Benzyl (3S,4R,5R)-3-(2-Hydroxyethoxy)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate
   1.14 g of benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(2-triisopropylsilanyloxyethoxy)piperidine-1-carboxylate are reacted in analogy to method J. The title compound is obtained as a yellowish oil. Rf = 0.38 (EtOAc/heptane 2:1); Rt = 4.63 (gradient I).
d) Benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(2-triisopropylsilanyloxyethoxy)piperidine-1-carboxylate
   0.165 g of sodium hydride (60% dispersion in oil) is added to a solution of 1.65 g of benzyl (3S,4S,5R)-3-hydroxy-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate at 0°C, and the mixture is stirred for 30 minutes. 1.11 g of (2-iodoethoxy)triisopropylsilane are added to the resulting solution, and it is then stirred at room temperature for 14 hours. The reaction mixture is poured into ice-water and extracted with tert-butyl methyl ether. The organic phases are washed with water and brine, dried with sodium sulphate and evaporated. The title compound is obtained as a colourless oil from the residue by flash chromatography (SiO₂ 60F). Rf = 0.39 (EtOAc/heptane 2:1).
e) Benzyl (3S,4S,5R)-3-hydroxy-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate
   Benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-triisopropylsilanyloxypiperidine-1-carboxylate is reacted in analogy to method J. The title compound is obtained as a yellowish resin. Rf = 0.30 (EtOAc/heptane 2:1); Rt = 4.63 (gradient I).
f) 2-Methoxyethyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-triisopropylsilanyloxypiperidine-1-carboxylate and Benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-triisopropylsilanyloxypiperidine-1-carboxylate
   The two title compounds are obtained from 4.650 g of benzyl (3R,4R,5S)-4-(4-chloromethylphenyl)-3-[4-(3-methoxypropyl)-3,4-dihydro-2H-benzo[1,4]oxazin-6-ylmethoxy]-5-triisopropylsilanyloxypiperidine-1-carboxylate in analogy to method D. 2-Methoxyethyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-triisopropylsilanyloxypiperidine-1-carboxylate:
   Yellowish resin; Rf = 0.26 (EtOAc/heptane 1:1); Rt = 29.90 (gradient II).
   Benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-triisopropylsilanyloxypiperidine-1-carboxylate: Yellowish resin; Rf = 0.36 (EtOAc/heptane 1:1); Rt = 31.96 (gradient II).
g) Benzyl (3R,4R,5S)-4-(4-chloromethylphenyl)-3-[4-(3-methoxypropyl)-3,4-dihydro-2H-benzo[1,4]oxazin-6-ylmethoxy]-5-triisopropylsilanyloxypiperidine-1-carboxylate
   A solution of 5.430 g of benzyl (3R,4R,5S)-4-(4-hydroxyethylphenyl)-3-[4-(3-methoxypropyl)-3,4-dihydro-2H-benzo[1,4]oxazin-6-ylmethoxy]-5-triisopropylsilanyloxypiperidine-1-carboxylate (Example 42f) in 100 ml of dichloromethane is cooled to 0°C, and 12.12 ml of 1-chloro-N,N,2-trimethylpropenylamine are added dropwise. The reaction solution is warmed to 20°C over 16 hours, tert-butyl methyl ether and water are added, and the phases are separated. The organic phase is washed with brine, dried (sodium sulphate) and evaporated. The title compound is obtained as a yellowish oil from the residue by flash chromatography (SiO₂ 60F). Rf = 0.39 (EtOAc/heptane 1:2).

### Example 66

### (2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}ethyl)dimethylamine

The title compound is prepared from 0.215 g of benzyl (3S,4R,5R)-3-(2-dimethylaminoethoxy)-4-[4-(2-methoxyethoxymethyl)phenyl]-5-[4-(3-methoxypropyl)-3,4-dihydro-2H-benzo-[1,4]oxazin-6-ylmethoxy]piperidine-1-carboxylate in analogy to method B.

The starting material is prepared as follows:
a) Benzyl (3S,4R,5R)-3-(2-dimethylaminoethoxy)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate
   A solution of 0.290 g of benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-[2-(toluene-4-sulphonyloxy)ethoxy]piperidine-1-carboxylate (Example 65a), 0.24 ml of triethylamine and 3.13 ml of dimethylamine (33% in ethanol) is stirred at room temperature for 3 hours. The reaction mixture is then poured into ice-water and extracted with tert-butyl methyl ether. The organic phases are washed with water and brine, dried with sodium sulphate and evaporated. The title compound is obtained as a yellowish oil from the residue by flash chromatography (SiO₂ 60F). Rf = 0.17 (dichloromethane/methanol/25% conc. ammonia = 200:20:1); Rt = 4.33 (gradient I).

### Example 67

### 6-[(3R,4S,5S)-4-[4-(2-Methoxyethoxymethyl)phenyl]-5-(3-[1,2,4]triazol-1-yl-propoxy)-piperidin-3-yloxymethyl]-4-(3-methoxypropyl)-3,4-dihydro-2H-benzo[1,4]oxazine

The title compound is prepared from 0.062 g of 6-[(3R,4S,5S)-4-[4-(2-methoxyethoxymethyl)phenyl]-1-(toluene-4-sulphonyl)-5-(3-[1,2,4]triazol-1-yl-propoxy)piperidin-3-yloxymethyl]-4-(3-methoxypropyl)-3,4-dihydro-2H-benzo[1,4]oxazine in analogy to method L.

The starting materials are prepared as follows:
a) 6-[(3R,4S,5S)-4-[4-2-Methoxyethoxymethyl)phenyl]-1-(toluene-4-sulphonyl)-5-(3-[1,2,4]-triazol-1-yl-propoxy)piperidin-3-yloxymethyl]-4-3-methoxypropyl)-3,4-dihydro-2H-benzo-[1,4]oxazine
   The title compound is obtained as a yellowish oil from 0.099 g of 3-[(3S,4S,5R)-4-[4-(2-methoxyethoxymethyl)phenyl]-5-[4-(3-methoxypropyl)-3,4-dihydro-2H-benzo[1,4]oxazin-6-yl-methoxy]-1-(toluene-4-sulphonyl)piperidin-3-yloxy]propyl toluene-4-sulphonate in analogy to Example 65a. Rf = 0.19 (dichloromethane/methanol 95:5); Rt = 4.70 (gradient 1).
b) 3-[(3S,4S,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propyl toluene-4-sulphonate
   The title compound is obtained as a colourless oil from 0.107 g of 3-[(3S,4S,5R)-4-[4-2-methoxyethoxymethyl)phenyl]-5-[4-(3-methoxypropyl)-3,4-dihydro-2H-benzo[1,4]oxazin-6-yl-methoxy]-1-(toluene-4-sulphonyl)piperidin-3-yloxy]propan-1-ol in analogy to method H. Rf = 0.34 (EtOAc/heptane 3:1); Rt = 5.63 (gradient I).
c) 3-[(3S,4S,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-1-ol
   The title compound is obtained as a colourless oil from 0.177 g of 3-[(3R,4S,5S)-4-[4-(2-methoxyethoxymethyl)phenyl]-1-(toluene-4-sulphonyl)-5-(3-triisopropylsilanyloxypropoxy)-piperidin-3yloxymethyl]-4-(3-methoxypropyl)-3,4-dihydro-2H-benzo[1,4]oxazine in analogy to method J. Rf = 0.07 (EtOAc/heptane 4:1); Rt = 4.75 (gradient I).
d) 3-[(3R,4S,5S)-4-[4-2-Methoxyethoxymethyl)phenyl]-1-(toluene-4-sulphonyl)-5-(3-triisopropylsilanyloxypropoxy)piperidin-3-yloxymethyl]-4-(3-methoxypropyl)-3,4-dihydro-2H-benzo[1,4]oxazine
   0.030 g of sodium hydride (60% dispersion in oil) is added to a solution of 0.324 g of (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b) in 3 ml of N,N-dimethylformamide at 0°C. The reaction mixture is stirred at room temperature for 30 minutes and then 0.008 g of sodium iodide and 0.221 g of (3-bromopropoxy)triisopropylsilane [215650-24-1] are added. The reaction mixture is stirred at room temperature for 2 hours. The reaction mixture is poured into saturated aqueous sodium bicarbonate solution, and the mixture is extracted with tert-butyl methyl ether. The combined organic extracts are washed with brine, dried with sodium sulphate and evaporated. The title compound is obtained as a yellowish oil from the residue by flash chromatography (SiO₂ 60F). Rf = 0.49 (EtOAc/heptane 2:1); Rt = 32.67 (gradient II).

### Example 68

### (R)-1-{(3S,4R,5R )-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy]propan-2-ol

The title compound is obtained from 0.262 g of benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate in analogy to method B.

The starting material is prepared as follows:
a) Benzyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate
   0.043 g of sodium hydride (60% dispersion in oil) is added to a solution of 0.507 g of benzyl (3S,4S,5R)-3-hydroxy-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate (Example 65e) in 5 ml of tetrahydrofuran. The mixture is stirred at 40°C for 45 minutes. A solution of 0.354 g of (R)-1-oxiranylmethyl toluene-4-sulphonate [113826-06-5] in 3 ml tetrahydrofuran is added, and the mixture is heated at 50°C for 3 hours. The reaction mixture is poured into saturated aqueous sodium bicarbonate solution, and the mixture is extracted with tert-butyl methyl ether. The combined organic extracts are washed with brine, dried over sodium sulphate and evaporated. The title compound is obtained as a colourless oil from the residue by flash chromatography (SiO₂ 60F). Rf = 0.24 (EtOAc/heptane 2:1); Rt = 5.25 (gradient I).

The following compounds are prepared in an analogous manner to the process described in Example 68:

### Examples

- 70: (S)-1-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}propan-2-ol
- 86: (R)-1-{(3S,4R,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}propan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a). Deprotection of the protective group on the nitrogen (last stage of the synthesis) is carried out in analogy to method L.
- 113: (S)-1-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}propan-2-ol
Starting from (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 52b). Deprotection of the protective group on the nitrogen (last stage of the synthesis) is carried out in analogy to method L.
- 114: (S)-1-{(3S,4R,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}propan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a). Deprotection of the protective group on the nitrogen (last stage of the synthesis) is carried out in analogy to method L.
- 117: (S)-1-{(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]piperidin-3-yloxy}propan-2-ol
Starting from (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 43a). Deprotection of the protective group on the nitrogen (last stage of the synthesis) is carried out in analogy to method L.
- 149: (R)-1-{(3S,4R,5R)-4-[4-(1-Methoxymethylcyclopropylmethoxymethyl)phenyl]-5-[4-(3-methoxypropyl)-3,4-dihydro-2H-benzo[1,4]oxazin-6-ylmethoxy]piperidin-3-yloxy}-propan-2-ol
Starting from (3S,4S,5R)-4-[4-(1-methoxymethylcyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol. Deprotection of the protective group on the nitrogen (last stage of the synthesis) is carried out in analogy to method L.

The starting materials are prepared as follows:
a) (3S,4S,5R)-4-[4-(1-Methoxymethylcyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   The title compound is identified on the basis of the Rf from 0.5 mmol of 6-[(3R,4R,5S)-4-[4-(1-methoxymethylcyclopropylmethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo-[1,4]oxazine in analogy to method J.
b) 6-[(3R,4R,5S)-4-[4-(1-Methoxymethylcyclopropylmethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   The title compound is identified on the basis of the Rf from 1 mmol of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and (1-methoxymethylcyclopropyl)methanol [338455-22-4] in analogy to Example 42b.

- 150: (R)-1-{(3S,4R,5R)-4-[4-(1-Methoxycyclopropylmethoxymethyl)phenyl]-5-[4-(3-methoxypropyl)-3,4-dihydro-2H-benzo[1,4]oxazin-6-ylmethoxy]piperidin-3-yloxy}propan-2-ol
Starting from (3S,4S,5R)-4-[4-(1-methoxycyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol. Deprotection of the protective group on the nitrogen (last stage of the synthesis) is carried out in analogy to method L.

The starting materials are prepared as follows:
a) (3S,4S,5R)-4-[4-(1-Methoxycyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   The title compound is prepared in analogy to the process described in Example 149 from 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and (1-methoxycyclopropyl)methanol and identified on the basis of the Rf.
b) (1-Methoxycyclopropyl)methanol
   The title compound is identified on the basis of the Rf from 2 mmol of methyl 1-methoxycyclopropanecarboxylate in analogy to Example 67e.

### Example 69

### 2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-1-pyrrolidin-1-ylethanone

The title compound is prepared from 0.121 g of 2-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]-1-pyrrolidin-1-ylethanone in analogy to method L.

The starting materials are prepared as follows:
a) 2-[(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]-1-pyrrolidin-1-ylethanone
   0.194 ml of propylphosphonic anhydride [68957-94-8, T3P] (50% in ethyl acetate) is added to a solution of 0.196 g of [(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yl-oxy] acetic acid, 0.024 g of pyrrolidine and 0.193 ml of triethylamine in 2 ml of dichloromethane at 0°C, and the mixture is stirred at room temperature for 16 hours. The reaction mixture is diluted with dichloromethane, and 0.1 M aqueous HCl is added. The phases are separated and the aqueous phase is extracted twice more with dichloromethane. The combined organic phases are washed with brine, dried with sodium sulphate and evaporated. The title compound is obtained as a yellow oil from the residue by flash chromatography (SiO₂ 60F). Rf = 0.17 (EtOAc); Rt = 4.86 (gradient I).
b) [(3S,4R,5R)-4-[4-(2-Methoxyethoxymethyl)phenyl]-5-[4-(3-methoxypropyl)-3,4-dihydro-2H-benzo[1,4]oxazin-6-ylmethoxyl]-1-(toluene-4-sulphonyl)piperidin-3-yloxy] acetic acid
   4 ml of a 1.5M aqueous lithium hydroxide solution are added to a solution of 0.24 g of methyl [(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy] acetate in 4 ml of tetrahydrofuran, and the mixture is stirred at room temperature for 5 hours. 2M aqueous HCl is added to the reaction mixture until the pH is 2. The resulting mixture is extracted twice with 80 ml of ethyl acetate each time. The combined organic phases are washed with brine, dried with sodium sulphate and evaporated. The title compound is obtained without further purification as a yellow oil. Rt = 4.67 (gradient I).
c) Methyl [(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy] acetate
   0.02 g of sodium hydride (60% dispersion in oil) is added to a solution of 0.25 g of (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b), 0.241 g of methyl bromoacetate and 5.7 mg of sodium iodide in 3 ml of N,N-dimethylformamide at room temperature, and the mixture is stirred at room temperature for 3 hours. The reaction mixture is diluted with ethyl acetate and poured into 0.1 M aqueous HCl. The resulting mixture is extracted three times with ethyl acetate. The combined organic phases are washed with brine, dried with sodium sulphate and evaporated. The title compound is obtained as a yellow oil from the residue by flash chromatography (SiO₂ 60F). Rt = 5.11 (gradient I).

The following compounds are prepared in an analogous manner to the process described in Example 69:

### Examples

- 72: N,N-Diethyl-2-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-yloxy}acetamide
- 73: N-Ethyl-2-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-yloxy}-N-methylacetamide
- 74: 2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-N-methyl-N-propylacetamide
- 75: 2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-N-propylacetamide
- 76: 2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-N,N-dimethylacetamide
- 102: 2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-1-piperidin-1-ylethanone
- 103: 2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-1-((R)-2-methylpyrrolidin-1-yl)ethanone

The following compounds are prepared in analogous manner to the process described in Example 69 starting from (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 52b):

### Examples

- 83: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-N,N-dimethylacetamide
- 84: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-1-pyrrolidin-1-ylethanone
- 93: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-N-propylacetamide
- 95: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-N,N-diethylacetamide
- 96: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-N-ethyl-N-methylacetamide
- 99: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-1-((R)-2-methylpyrrolidin-1-yl)ethanone
- 100: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-1-piperidin-1-ylethanone
- 101: 2-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}-1-morpholin-4-ylethanone

The following compounds are prepared in an analogous manner to the process described in Example 69 starting from (3S,4S,5R)-4-(4-methoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 58b):

### Examples

- 90: 2-{(3S,4R,5R)-4-(4-Methoxymethylphenyl)-5-[4-(3-methoxypropyl)-3,4-dihydro-2H-benzo[1,4]oxazin-6-ylmethoxy]piperidin-3-yloxy}-1-((R)-2-methylpiperidin-1-yl)ethanone
- 91: 1-((2S,6R)-2,6-Dimethylpiperidin-1-yl)-2-{(3S,4R,5R)-4-(4-methoxymethylphenyl)-5-[4-(3-methoxypropyl)-3,4-dihydro-2H-benzo[1,4]oxazin-6-ylmethoxy]piperidin-3-yl-oxy}ethanone
- 92: 2-{(3S,4R,5R)-4-(4-Methoxymethylphenyl)-5-[4-(3-methoxypropyl)-3,4-dihydro-2H-benzo[1,4]oxazin-6-ylmethoxy]piperidin-3-yloxy}-1-piperidin-1-ylethanone
- 94: 2-{(3S,4R,5R)-4-(4-Methoxymethylphenyl)-5-[4-(3-methoxypropyl)-3,4-dihydro-2H-benzo[1,4]oxazin-6-ylmethoxy]piperidin-3-yloxy}-1-pyrrolidin-1-ylethanone
- 97: 2-{(3S,4R,5R)-4-(4-Methoxymethylphenyl)-5-[4-(3-methoxypropyl)-3,4-dihydro-2H-benzo[1,4]oxazin-6-ylmethoxy]piperidin-3-yloxy}-1-((R)-3-methylmorpholin-4-yl)-ethanone
- 98: 1-((3S,5R)-3,5-Dimethylmorpholin-4-yl)-2-{(3S,4R,5R)-4-(4-methoxymethylphenyl)-5-[4-(3-methoxypropyl)-3,4-dihydro-2H-benzo[1,4]oxazin-6-ylmethoxy]piperidin-3-yloxy}ethanone
- 110: N-Ethyl-2-{(3S,4R,5R)-4-(4-methoxymethylphenyl)-5-[4-(3-methoxypropyl)-3,4-dihydro-2H-benzo[1,4]oxazin-6-ylmethoxy]piperidin-3-yloxy}-N-methylacetamide

The following compounds are prepared in an analogous manner to the process described in Example 69 starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a):

### Examples

- 127: 2-{(3S,4R,5R)-4-[4-(3-Methoxypropoxymethyl)phenyl-5-[4-(3-methoxypropyl)-3,4-dihydro-2H-benzo[1,4]oxazin-6-ylmethoxy]piperidin-3-yloxy}-1-pyrrolidin-1-ylethanone
- 128: N,N-Diethyl-2-{(3S,4R,5R)-4-[4-(3-methoxypropoxymethyl)phenyl]-5-[4-(3-methoxypropyl)-3,4-dihydro-2H-benzo[1,4]oxazin-6-ylmethoxy]piperidin-3-yloxy}acetamide
- 129: N-Ethyl-2-{(3S,4R,5R)-4-[4-(3-methoxypropoxymethyl)phenyl]-5-[4-(3-methoxypropyl)-3,4-dihydro-2H-benzo[1,4]oxazin-6-ylmethoxy]piperidin-3-yloxy}-N-methylacetamide

### Example 71

### 6-[(3R,4R,5S)-4-[4-(2-Methoxyethoxymethyl)phenyl]-5-(3-methyl-3H-imidazol-4-ylmethoxy)-piperidin-3-yloxymethyl]-4-(3-methoxypropyl)-3,4-dihydro-2H-benzo[1,4]oxazine

2-Methoxyethyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(3-methyl-3H-imidazol-4-ylmethoxy)-piperidine-1-carboxylate (0.150 g) is dissolved in 1:1 methanol/dioxane (4 ml), and 2 ml of 40% aqueous potassium hydroxide solution are added to the solution. The mixture is heated in a closed flask at 80°C for 4 hours. The reaction solution is poured into water and extracted with tert-butyl methyl ether. The combined organic extracts are washed with brine, dried over sodium sulphate and concentrated. The title compound is obtained from the residue by flash chromatography (SiO₂ 60F).

The starting material is prepared as follows:
a) 2-Methoxyethyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(3-methyl-3H-imidazol-4-yl-methoxy)piperidine-1-carboxylate
   0.086 g of sodium hydride (60% dispersion in oil) is added to a solution of 0.430 g of 2-methoxyethyl (3S,4S,5R)-3-hydroxy-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate (Example 65e) and 0.241 g of 5-chloromethyl-1-methyl-1 H-imidazole hydrochloride [90773-41-4] in 4 ml of N,N-dimethylformamide at 0°C. 0.027 g of tetrabutylammonium iodide is added, and the reaction mixture is stirred at room temperature for 18 hours. The reaction mixture is poured into saturated aqueous sodium bicarbonate solution, and the mixture is extracted with tert-butyl methyl ether. The combined organic extracts are washed with brine, dried over sodium sulphate and evaporated. The title compound is obtained as a yellowish oil from the residue by flash chromatography (SiO₂ 60F). Rf = 0.11 (dichloromethane/methanol 95:5); Rt = 3.79 (gradient I).

### Example 77

### (R)-1-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}butan-2-ol

The title compound is obtained from 0.726 g of (R)-1-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-toluene-3-sulphony)piperidin-3-yloxy]butan-2-ol in analogy to method L.

The starting material is prepared as follows:
a) (R)-1-[(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-toluene-3-sulphony)piperidin-3-yloxy]-butan-2-ol
   0.015 g of copper(I) cyanide is taken up in 10 ml of dry tetrahydrofuran under argon in a heat-dried Schlenk tube. The suspension is cooled to -78°C, and 0.429 ml of methylmagnesium bromide solution (35% in diethyl ether) is added dropwise. A solution of 0.815 g of 6-[(3R,4R,5S)-4-[4-(2-methoxyethoxymethyl)phenyl]-5-((R)-1-oxiranylmethoxy)-1-(toluene-4-sulphonyl)piperidinyloxymethyl]-4-(3-methoxypropyl)-3,4-dihydro-2H-benzo[1,4]oxazine in 5 ml of dry tetrahydrofuran is added, and the reaction mixture is stirred at -78°C for 30 minutes and then thawed to 20°C over 16 hours. The reaction mixture is poured into saturated aqueous ammonium chloride solution and adjusted to pH 10 with 25% ammonium hydroxide solution. The mixture is extracted with diethyl ether, and the combined organic extracts are washed with brine, dried with sodium sulphate and evaporated. The title compound is obtained as a yellow resin from the residue by flash chromatography (SiO₂ 60F). Rf = 0.14 (EtOAc/heptane 2:1); Rt = 5.06 (gradient I).
b) 6-[(3R,4R,5S)-4-[4-(2-Methoxyethoxymethyl)phenyl]-5-((R)-1-oxiranylmethoxy)-1-(toluene-4-sulphonyl)piperidinyloxymethyl]-4-(3-methoxypropyl)-3,4-dihydro-2H-benzo[1,4]oxazine
   The title compound is obtained as a colourless oil from (3S,4S,5R)-4-[4-(2-methoxyethoxymethyl)phenyl]-5-[4-(3-methoxypropyl)-3,4-dihydro-2H-benzo[1,4]oxazin-6-yloxymethyl]-1-(toluene-4-sulphonyl)piperidin-3-ol (Example 59b) in analogy to Example 68a. Rf = 0.13 (EtOAc/heptane 3:1); Rt = 5.09 (gradient I).

The following compounds are prepared in an analogous manner to the process described in Example 77:

### Examples

- 115: (S)-1-{(3S,4R,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a).
- 116: (S)-1-{(3S,4R,5R)-4-[4-(3-Methoxypropoxymethyl)phenyl]-5-[4-(3-methoxypropyl)-3,4-dihydro-2H-benzo[1,4]oxazin-6-ylmethoxylpiperidin-3-yloxy}pentan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a) using ethylmagnesium bromide solution.
- 118: (R)-1-{(3S,4R,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a).

- 123: (R)-1-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 52b).
- 124: (S)-1-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 52b).
- 130: (R)-1-{(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 43a).
- 131: (S)-1-{(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 43a).
- 133: (R)-1-{(3S,4R,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}pentan-2-ol
Starting from (3S,4S,5R)-4-[4-(3-methoxypropoxymethyl)phenyl]-5-[4-(3-methoxy-propyl)-3,4-dihydro-2H-benzo[1,4]oxazin-6-ylmethoxy]-1-(toluene-4-sulphonyl)piperidin-3-ol (Example 85a) using ethylmagnesium bromide solution.
- 135: (R)-1-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}pentan-2-ol
Starting from (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b) using ethylmagnesium bromide solution.
- 136: (S)-1-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxylpiperidin-3-yloxy}pentan-2-ol
Starting from (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b) using ethylmagnesium bromide solution.

- 147: (R)-1-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b).

- 148: (S)-1-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b).

### Example 78

### (3S,4S,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ol

The title compound is prepared from 14.64 g of (3S,4S,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b) in analogy to method L.

### Example 80

### (R)-1-{(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]piperidin-3-yloxy}propan-2-ol

The title compound is prepared from 215 mg (R)-1-[(3S,4R,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting materials are prepared as follows:
a) (R)-1-[(3S,4R,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol
   42 mg of sodium borohydride are added to a solution of 275 mg of 6-[(3R,4R,5S)-4-[4-((S)-3-methoxy-2-methylpropoxymethyl)phenyl]-5-((R)-1-oxiranylmethoxy)-1-(toluene-4-sulphonyl)piperidin-3-yloxymethyl]-4-(3-methoxypropyl)-3,4-dihydro-2H-benzo[1,4]oxazine in 5 ml of ethanol and 0.25 ml of tetrahydrofuran. After 21 hours at 45°C, the reaction mixture is diluted with tert-butyl methyl ether. The mixture is washed successively with saturated ammonium chloride solution, water and brine. The combined aqueous phases are back-extracted with dichloromethane (1×). The combined organic phases are dried with sodium sulphate and evaporated. The title compound is obtained as a yellowish resin from the residue by flash chromatography (SiO₂ 60F). Rf = 0.08 (EtOAc/heptane 1:1); Rt = 5.34 (gradient I).
b) 6-[(3R,4R,5S)-4-[4-((S)-3-Methoxy-2-methylpropoxymethyl)phenyl]-5-((R)-1-oxiranylmethoxy)-1-(toluene-4-sulphonyl)piperidin-3-yloxymethyl]-4-(3-methoxypropyl)-3,4-dihydro-2H-benzo[1,4]oxazine
   396 mg of (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 43a) and 334 mg of (R)-1-oxiranymethyl toluene-4-sulphonate [113826-06-5] are reacted in analogy to Example 68a. The title compound is obtained as a colourless resin. Rf = 0.05 (EtOAc/heptane 1:2); Rt = 5.49 (gradient I).

### Example 81

### (R)-1-{(3S,4R,5R)-5-[4-(3-Methoxypropyl)-3,4-dihydro-2H-benzo[1,4]oxazin-6-ylmethoxyl-4-[4-((R)-2-methyl-3-methylsulphanylpropoxymethyl)phenyl]piperidin-3-yloxy}propan-2-ol

The title compound is prepared from 565 mg of (R)-1-[(3S,4R,5R)-5-[4-(3-methoxypropyl)-3,4-dihydro-2H-benzo[1,4]oxazin-6-ylmethoxy]-4-[4-((R)-2-methyl-3-methylsulphanylpropoxymethyl)phenyl]-1-(toluene-4-sulphonyl)piperidin-3-yloxy]propan-2-ol in analogy to method N.

The starting materials are prepared as follows:
a) (R)-1-[(3S,4R,5R)-5-[4-(3-Methoxypropyl)-3,4-dihydro-2H-benzo[1,4]oxazin-6-yl-methoxy]-4-[4-((R)-2-methyl-3-methylsulphanylpropoxymethyl)phenyl]-1-(toluene-4-sulphonyl)piperidin-3-yloxy]propan-2-ol
   670 mg of 4-(3-methoxypropyl)-6-[(3R,4R,5S)-4-[4-((R)-2-methyl-3-methylsulphanylpropoxymethyl)phenyl]-5-((R)-1-oxiranylmethoxy)-1-(toluene-4-sulphonyl)piperidin-3-yloxymethyl]-3,4-dihydro-2H-benzo[1,4]oxazine are reacted in analogy to Example 80a. The title compound is obtained as a yellowish resin. Rf = 0.09 (EtOAc/heptane 1:1); Rt = 5.63 (gradient I).
b) 4-(3-Methoxypropyl)-6-[(3R,4R,5S)-4-[4-((R)-2-methyl-3-methylsulphanylpropoxymethyl)phenyl]-5-((R)-1-oxiranylmethoxy)-1-(toluene-4-sulphonyl)piperidin-3-yloxymethyl]-3,4-dihydro-2H-benzo[1,4]oxazine
   848 mg of (3S,4S,5R)-5-[4-(3-methoxypropyl)-3,4-dihydro-2H-benzo[1,4]oxazin-6-yl-methoxy]-4-[4-((R)-2-methyl-3-methylsulphanylpropoxymethyl)phenyl]-1-(toluene-4-sulphonyl)piperidin-3-ol (Example 46a) and 699 mg of (R)-1-oxiranylmethyl toluene-4-sulphonate [113826-06-5] are reacted in analogy to Example 68a. The title compound is obtained as a colourless resin. Rf = 0.10 (EtOAc/heptane 1:2); Rt = 5.79 (gradient I).

### Example 82

### (R)-1-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}propan-2-ol

The title compound is prepared from 210 mg of (R)-1-[(3S,4R,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method L.

The starting materials are prepared as follows:
a) (R)-1-[(3S,4R,5R)-4-(4-Cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-yloxy]-propan-2-ol
   480 mg of 6-[(3R,4R,5S)-4-(4-cyclopropylmethoxymethylphenyl)-5-((R)-1-oxiranylmethoxy)-1-(toluene-4-sulphonyl)piperidin-3-yloxymethyl]-4-(3-methoxypropyl)-3,4-dihydro-2H-benzo-[1,4]oxazine are reacted in analogy to Example 80a. The title compound is obtained as a cloudy colourless oil. Rf = 0.20 (EtOAc/heptane 3:1); Rt = 5.27 (gradient I).
b) 6-[(3R,4R,5S)-4-(4-Cyclopropylmethoxymethylphenyl)-5-((R)-1-oxiranylmethoxy)-1-(toluene-4-sulphonyl)piperidin-3-yloxymethyl]-4-(3-methoxypropyl)-3,4-dihydro-2H-benzo[1,4]oxazine
   370 mg of (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example52b) and 256 mg of (R)-1-oxiranylmethyl toluene-4-sulphonate [113826-06-5] are reacted in analogy to Example 68a. The title compound is obtained as a yellow oil. Rf = 0.50 (EtOAc/heptane 3:1); Rt = 5.47 (gradient I).

### Example 85

### (3S,4S,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-ol

The title compound is prepared from 342 mg of (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol in analogy to method L.

The starting materials are prepared as follows:
a) (3S,4S,5R)-4-[4-(3-Methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   1.18 g of 6-[(3R,4R,5S)-4-[4-(3-methoxypropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo-[1,4]oxazine are reacted in analogy to method J. The title compound is obtained as a yellow oil. Rf = 0.3 (EtOAc/heptane 2:1); Rt = 4.85 (gradient I).
b) 6-[(3R,4R,5S)-4-[4-(3-Methoxypropoxymethyl)phenyl]-1-(toluene-4-sulphonyl)-5-triisopropylsilanyloxypiperidin-3-yloxymethyl]-4-(3-methoxypropyl)-3,4-dihydro-2H-benzo-[1,4]oxazine
   2 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and 0.48 g of 3-methoxy-1-propanol are reacted in analogy to Example 42b. The title compound is obtained as a yellow oil. Rf = 0.5 (EtOAc/heptane 1:1); Rt = 29.43 (II).

### Example 87

### (3S,4S,5R)-5-[4-(3-Methoxypropyl)-3,4-dihydro-2H-benzo[1,4]oxazin-6-ylmethoxy]-4-[4-(2-methylsulphanylethoxymethyl)phenyl]piperidin-3-ol

The title compound is prepared from 0.20 mmol of (3S,4S,5R)-5-[4-(3-methoxypropyl)-3,4-dihydro-2H-benzo[1,4]oxazin-6-ylmethoxy]-4-[4-(2-methylsulphanylethoxymethyl)phenyl]-1-(toluene-4-sulphonyl)piperidin-3-ol in analogy to method N.

The starting material is prepared as follows:
a) (3S,4S,5R)-5-[4-(3-Methoxypropyl)-3,4-dihydro-2H-benzo[1,4]oxazin-6-ylmethoxy]-4-[4-(2-methylsulphanylethoxymethyl)phenyl]-1-(toluene-4-sulphonyl)piperidin-3-ol
   0.8 mmol of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and 1.0 mmol of 2-methylsulphanylethanol are reacted in analogy to Example 42b. The title compound is obtained as a yellow oil. Rf = 0.18 (EtOAc/heptane 1:1); Rt = 5.06 (gradient I).

The following compound is prepared in an analogous manner to the process described in Example 87.

Example
- 88: (3S,4S,5R)-4-[4-(2-Methoxyethylsulphanylmethyl)phenyl]-5-[4-(3-methoxy-propyl)-3,4-dihydro-2H-benzo[1,4]oxazin-6-ylmethoxy]piperidin-3-ol
Starting from (3S,4S,5R)-4-[4-(2-methoxyethylsulphanylmethyl)phenyl]-5-[4-(3-methoxypropyl)-3,4-dihydro-2H-benzo[1,4]oxazin-6-ylmethoxy]-1-(toluene-4-sulphonyl)piperidin-3-ol.

The starting materials are prepared as follows:
a) (3S,4S,5R)-4-[4-(2-Methoxyethylsulphanylmethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   0.75 mmol of 6-[(3R,4R,5S)-4-[4-(2-methoxyethylsulphanylmethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine are reacted in analogy to method J. The title compound is obtained as a yellow oil. Rf = 0.20 (EtOAc/heptane 1:1 ); Rt = 4.92 (gradient I).
b) 6-[(3R,4R,5S)-4-[4-(2-Methoxyethylsulphanylmethyl)-phenyl]-1-(toluene-4-sulphonyl)-5-triisopropylsilanyloxypiperidin-3-yloxymethyl]-4-(3-methoxypropyl)-3,4-dihydro-2H-benzo-[1,4]oxazine
   1.0 mmol of 2-{4-[(3R,4R,5S)-3-[4-(3-methoxypropyl)-3,4-dihydro-2H-benzo[1,4]oxazin-6-yl-methoxy]-1-(toluene-4-sulphonyl)-5-triisopropylsilanyloxypiperidin-4-yl]benzylsulphanyl}ethanol and 1.5 mmol of methyl iodide are reacted in analogy to method D. The title compound is obtained as a yellow oil. Rf = 0.50 (EtOAc/heptane 1:1); Rt = 32.09 (gradient II).
c) 2-{4-[(3R,4R,5S)-3-[4-(3-Methoxypropyl)-3,4-dihydro-2H-benzo[1,4]oxazin-6-ylmethoxy]-1-(toluene-4-sulphonyl)-5-triisopropylsilanyloxypiperidin-4-yl]benzylsulphanyl}ethanol
   A mixture of 2 mmol of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo-[1,4]oxazine (Example 42c), 2 mmol of 2-mercaptoethanol and 3 mmol of potassium carbonate in 8 ml of N,N-dimethylformamide is stirred at room temperature for 4 hours. The reaction mixture is diluted with water and extracted with tert-butyl methyl ether (3x). The combined organic phases are washed with water, dried with sodium sulphate and evaporated. The title compound is obtained as a yellow oil from the residue by flash chromatography (SiO₂ 60F). Rf = 0.50 (EtOAc/heptane 1:1); Rt = 22.92 (gradient II).

### Example 89

### (R)-1-{(3S,4R,5R)-5-[4-(3-Methoxypropyl)-3,4-dihydro-2H-benzo[1,4]oxazin-6-ylmethoxy]-4-[4-(2-methylsulphanylethoxymethyl)phenyl]piperidin-3-yloxy}propan-2-ol

60.1 mg of lithium aluminium hydride are added to a solution of 263 mg of (R)-1-[(3S,4R,5R)-5-[4-(3-methoxypropyl)-3,4-dihydro-2H-benzo[1,4]oxazin-6-ylmethoxy]-4-[4-(2-methylsulphanylethoxymethyl)phenyl]-1-(toluene-4-sulphonyl)piperidin-3-yloxy]propan-2-ol in 4 ml of tetrahydrofuran. The suspension is heated at 50°C for 26 hours, cooled to room temperature and, after cautious addition successively of 20 drops of water, 20 drops of 4N NaOH and 60 drops of water, stirred for 30 minutes. It is filtered through Hyflow and evaporated. The title compound is obtained from the residue by flash chromatography (SiO₂ 60F).

The starting materials are prepared as follows:
a) (R)-1-[(3S,4R,5R)-5-[4-(3-Methoxypropyl)-3,4-dihydro-2H-benzo[1,4]oxazin-6-ylmethoxy]-4-[4-(2-methylsulphanylethoxymethyl)phenyl]-1-(toluene-4-sulphonyl)piperidin-3-yloxy]propan-2-ol
   The title compound is obtained as a pale brown oil from (3S,4S,5R)-5-[4-(3-methoxypropyl)-3,4-dihydro-2H-benzo[1,4]oxazin-6-ylmethoxy]-4-[4-(2-methylsulphanylethoxymethyl)phenyl]-1-(toluene-4-sulphonyl)piperidin-3-ol (Example 87a) in analogy to Example 80a-b. Rf = 0.25 (EtOAc/heptane 3:1); Rt = 5.17 (gradient I).

The following compound is prepared in an analogous manner to the process described in Example 89:

### Example

- 104: (R)-1-{(3S,4R,5R)-4-[4-(2-Methoxyethylsulphanylmethyl)phenyl]-5-[4-(3-methoxypropyl)-3,4-dihydro-2H-benzo[1,4]oxazin-6-ylmethoxy]piperidin-3-yloxy}propan-2-ol
Starting from (3S,4S,5R)-4-[4-(2-methoxyethylsulphanylmethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 87a).

### Example 105

### Isopropyl{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-ylmethyl}amine

The title compound is prepared from isopropyl[(3R,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-3-ylmethyl]amine in analogy to method L.

The starting materials are prepared as follows:
a) Isopropyl[(3R,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-3-ylmethyl]amine
   A solution of 0.50 mmol of (3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl toluene-4-sulphonate and 1.0 mmol of isopropylamine in 4 ml of 1-methylpyrrolidin-2-one (NMP) is stirred at 85°C for 8 hours. The reaction mixture is cooled to room temperature, diluted with water and extracted with dichloromethane (3x). The combined organic phases are washed with brine, dried with sodium sulphate and evaporated. The title compound is identified on the basis of the Rf from the residue by flash chromatography (SiO₂ 60F).
b) (3S,4R,5R)-4-[4-(2-Methoxyethoxymethyl)phenyl]-5-[4-(3-methoxypropyl)-3,4-dihydro-2H-benzo[1,4]oxazin-6-ylmethoxyl-1-(toluene-4-sulphonyl)piperidin-3-ylmethyl toluene-4-sulphonate
   The title compound is identified on the basis of the Rf from 1 mmol of [(3S,4R,5R)-4-[4-(2-methoxyethoxymethyl)phenyl]-5-[4-(3-methoxypropyl)-3,4-dihydro-2H-benzo-[1,4]oxazin-6-ylmethoxy]-1-(toluene-4-sulphonyl)piperidin-3-yl]methanol in analogy to method H.
c) [(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yl]methanol
   The title compound is identified on the basis of the Rf from benzyl (3R,4R,5S)-3-hydroxy-4-(4-hydroxyphenyl)-5-triisopropylsilanyloxymethylpiperidine-1-carboxylate in analogy to the process described in Example 42a-j.
d) Benzyl (3R,4R,5S)-3-hydroxy-4-(4-hydroxyphenyl)-5-triisopropylsilanyloxymethyl-piperidine-1-carboxylate
   1.09 g of imidazole and 0.68 g of triisopropylchlorosilane are added to a solution of 1.76 g of benzyl (3R,4R,5S)-3-hydroxy-5-hydroxymethyl-4-(4-hydroxyphenyl)piperidine-1-carboxylate in 40 ml N,N-dimethylformamide at room temperature. After 16 hours, the reaction mixture is diluted with 1 N HCl and extracted with tert-butyl methyl ether (3x). The combined organic phases are dried with sodium sulphate and evaporated. The title compound is identified on the basis of the Rf from the residue by flash chromatography (SiO₂ 60F).
e) Benzyl (3R,4R,5S)-3-hydroxy-5-hydroxymethyl-4-(4-hydroxyphenyl)piperidine-1-carboxylate
   3.30 ml of benzyl chloroformate are slowly added to a solution of 5.58 g (3R,4R,5S)-5-hydroxymethyl-4-(4-hydroxyphenyl)piperidin-3-ol hydrobromide [303043-56-3] in 100 ml of saturated sodium bicarbonate solution and 100 ml of ethyl acetate at 0°C, and the mixture is stirred for 5 hours. The reaction mixture is extracted with ethyl acetate/tetrahydrofuran (2x). The combined organic phases are evaporated and the title compound is identified on the basis of the Rf from the residue.

The following compounds are prepared in an analogous manner to the process described in Example 105:

### Examples

- 106: tert-Butyl{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-ylmethyl}amine
- 107: {(3R,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}(2-methoxvethyl)amine
- 108: 6-{(3R,4R,5S)-4-[4-(2-Methoxyethoxymethyl)phenyl]-5-morpholin-4-ylmethylpiperidin-3-yloxymethyl}-4-(3-methoxypropyl)-3,4-dihydro-2H-benzo[1,4]oxazine

### Example 109

### N-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}acetamide

The title compound is prepared from N-[(3R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl]acetamide in analogy to method L.

The starting materials are prepared as follows:
a) N-[(3R,4R,5R)-4-[4-(2-Methoxyethoxymethyl)phenyl]-5-[4-(3-methoxy-propyl)-3,4-dihydro-2H-benzo[1,4]oxazin-6-ylmethoxy]-1-(toluene-4-sulphonyl)piperidin-3-ylmethyl]-acetamide
   5 mmol of triethylamine and 1 mmol of propylphosphonic anhydride [68957-94-8, T3P] (50% in ethyl acetate) are successively added to a solution of 1 mmol of C-[(3R,4R,5R)-4-[4-(2-methoxyethoxymethyl)phenyl]-5-[4-(3-methoxypropyl)-3,4-dihydro-2H-benzo-[1,4]oxazin-6-ylmethoxy]-1-(toluene-4-sulphonyl)piperidin-3-yl]methylamine and 1 mmol of acetic acid in 20 ml of dichloromethane at room temperature. After 12 hours, the reaction mixture is diluted with dichloromethane and washed successively with 1 N HCl and brine, dried with sodium sulphate and evaporated. The title compound is obtained as a yellow oil from the residue by flash chromatography (SiO₂ 60F). Rf = 0.44 (dichloromethane/methanol/25% conc. ammonia 200:20:1); Rt =4.52 (gradient I)..
b) C-[(3R,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yl]-methylamine
   A solution of 0.5 mmol of 6-[(3R,4R,5R)-5-azidomethyl-4-[4-(2-methoxyethoxymethyl)-phenyl]-1-(toluene-4-sulphonyl)piperidin-3-yloxymethyl]-4-(3-methoxypropyl)-3,4-dihydro-2H-benzo[1,4]oxazine in 15 ml of tetrahydrofuran is hydrogenated in the presence of 50 mg of 10% Pd/C (moist) at room temperature for 6 hours. The reaction mixture is clarified by filtration and the filtrate is evaporated. The title compound is obtained as a yellow oil from the residue by flash chromatography (SiO₂ 60F). Rf = 0.30 (dichloromethane/methanol/25% conc. ammonia 200:20:1); Rt =4.25 (gradient I).
c) 6-[(3R,4R,5R)-5-Azidomethyl-4-[4-(2-methoxyethoxymethyl)phenyl]-1-(toluene-4-sulphonyl)piperidin-3-yloxymethyl]-4-(3-methoxypropyl)-3,4-dihydro-2H-benzo-[1,4]oxazine
   A solution of 0.50 mmol of (3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl toluene-4-sulphonate (Example 105b) and 0.75 mmol of sodium azide in 5 ml of N,N-dimethylformamide is stirred at room temperature for 24 hours. The reaction mixture is diluted with water and extracted with tert-butyl methyl ether (3x). The combined organic phases are dried with sodium sulphate and evaporated. The title compound is obtained as a yellow oil from the residue by flash chromatography (SiO₂ 60F). Rf = 0.33 (EtOAc/heptane 3:1); Rt = 5.42 (gradient I).

The following compounds are prepared in an analogous manner to the process described in Example 109:

### Examples

- 138: N-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}pentanamide
- 140: N-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}-2-(tetrahydropyran-4-yl)acetamide
Using (tetrahydropyran-4-yl)acetic acid [85064-61-5]
- 141: N-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-ylmethyl}tetrahydropyran-4-carboxamide
Using tetrahydropyran-4-carboxylic acid [5337-03-1]
- 142: 2-Cyclopentyl-N-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-ymethyl}acetamide
Using cyclopentylacetic acid [1123-00-8]
- 143: N-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-ylmethyl}-(meso-1S,5R,6R)-3-oxabicyclo[3.1.0]hexane-6-carboxamide
Using (meso-1S,5R,6R)-3-oxabicyclo[3.1.0]hexane-6-carboxylic acid [55780-88-6]
- 144: N-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}-2-(meso-1R,5S,6S)-3-oxabicyclo[3.1.0]hex-6-ylacetamide
Using (meso-1 R,5S,6S)-(3-oxabicyclo[3.1.0]hex-6-yl)acetic acid

The starting materials are prepared as follows:
a) (meso-1R,5S,6S)-(3-Oxabicyclo[3.1.0]hex-6-yl)acetic acid
   3 mmol of triethylamine and 0.5 mmol of silver trifluoroacetate are added to a solution of 1 mmol of 1-diazo-3-(meso-1R,5S,6S)-3-oxabicyclo[3.1.0]hex-6-ylpropan-2-one in 70 ml of tetrahydrofuran/water 10:1 at -15°C. The reaction mixture is warmed to room temperature and stirred at room temperature for 2 hours. It is diluted with tert-butyl methyl ether, washed with 1M HCl and brine, dried with sodium sulphate and evaporated. The title compound is identified on the basis of the Rf from the residue by flash chromatography (SiO₂ 60F).
b) 1-Diazo-3-(meso-1R,5S,6S)-3-oxabicyclo[3.1.0]hex-6-yl-propan-2-one
   1.2 mmol of triethylamine and 1 mmol of ethyl chloroformate are added to a solution of 1 mmol of (meso-1S,5R,6R)-3-oxabicyclo[3.1.0]hexane-6-carboxylic acid [55780-88-6] in 60 ml of tetrahydrofuran at -15°C. The reaction mixture is warmed to -5°C and stirred at this temperature for 1 hour. It is cooled to -30°C, and 2.5 mmol of a diazomethane solution in ether are added and the mixture is stirred overnight. It is diluted with tert-butyl methyl ether, washed with saturated aqueous sodium bicarbonate solution and brine, dried with sodium sulphate and evaporated. The title compound is identified on the basis of the Rf from the residue by flash chromatography (SiO₂ 60F).

- 145: N-{(3S,4R,5R)-4-[4-(2-Methoxyethoxymethyl)-phenyl]-5-[4-(3-methoxypropyl)-3,4-dihydro-2H-benzo[1,4]oxazin-6-ylmethoxy]piperidin-3-ylmethyl}-4-methoxycyclohexanecarboxamide
Using 4-methoxycyclohexanecarboxylic acid [99183-14-9]
- 162: N-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}cyclopentanecarboxamide
Using cyclopentancarboxylic acid [3400-45-1]
- 163: 2-Ethyl-N-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-ylmethyl}butyramide
Using 2-ethylbutyric acid [88-09-5]

### Example 119

### (S)-4-{(3S,4S,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}butan-2-ol

The title compound is prepared from (S)-4-[(3S,4S,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-3-yloxy]butan-2-ol in analogy to method L.

The starting materials are prepared as follows:
a) (S)-4-[(3S,4S,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]butan-2-ol
   The title compound is obtained as a colourless wax from 1.04 g of 6-[(3R,4S,5S)-4-[4-(2-methoxyethoxymethyl)phenyl]-1-(toluene-4-sulphonyl)-5-((S)-3-triisopropylsilanyloxybutoxy)-piperidin-3-yloxymethyl]-4-(3-methoxypropyl)-3,4-dihydro-2H-benzo[1,4]oxazine in analogy to method J. Rf = 0.07 (EtOAc/heptane 3:1).
b) 6-[(3R,4S,5S)-4-[4-(2-Methoxyethoxymethyl)phenyl]-1-(toluene-4-sulphonyl)-5-((S)-3-triisopropylsilanyloxybutoxy)piperidin-3-yloxymethyl]-4-(3-methoxypropyl)-3,4-dihydro-2H-benzo[1,4]oxazine
   140 mg of sodium hydride (60% dispersion in oil) are added to a solution of 1.04 g of (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59b) in 8 ml of DMF at 0°C, and the mixture is stirred for 1 hour. It is then cooled to -5°C, and 1.27 g of (S)-triisopropylsilanyloxybutyl toluene-4-sulphonate are added. The reaction mixture is stirred at 60°C for 3 hours and then cooled to room temperature. It is subsequently diluted with tert-butyl methyl ether and poured into ice-water. The resulting mixture is extracted three times with tert-butyl methyl ether. The combined organic phases are washed with brine, dried with sodium sulphate and evaporated. The title compound is obtained as a colourless oil from the residue by flash chromatography (SiO₂ 60F). Rf = 0.28 (EtOAc/heptane 1:1).
c) (S)-3-triisopropylsilanyloxybutyl toluene-4-sulphonate
   7.33 ml of lutidine are added to a solution of 10 g of (S)-3-hydroxybutyl toluene-4-sulphonate [82614-88-4] in 100 ml of dichloromethane at 0°C. 12.49 ml of triisopropylsilyl trifluoromethanesulphonate are added dropwise, and the mixture is stirred at 0°C for 1 hour. It is quenched with 0.5M HCl and extracted with dichloromethane (2x). The combined organic phases are washed with brine, dried with sodium sulphate and evaporated. The title compound is obtained as a colourless liquid from the residue by flash chromatography (SiO₂ 60F). Rf = 0.72 (EtOAc/heptane 1:1); Rt = 6.64 (gradient I).

The following compounds are prepared in an analogous manner to the process described in Example 119:

### Examples

- 120: (R)-4-{(3S,4S,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}butan-2-ol
Using tert-butyl ((R)-3-iodo-1-methylpropoxy)dimethylsilane [109715-47-1]
- 121: (R)-4-{(3S,4S,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a) using tert-butyl ((R)-3-iodo-1-methylpropoxy)dimethylsilane [109715-47-1]
- 122: (S)-4-{(3S,4S,5R)-4-[4-(3-Methoxypropoxymethyl)phenyl]-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-[4-(3-methoxypropoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 85a).
- 125: (S)-4-{(3S,4S,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 52b).
- 126: (R)-4-{(3S,4S,5R)-4-(4-Cyclopropylmethoxymethylphenyl)-5-[4-(3-methoxypropyl)-3,4-dihydro-2H-benzo[1,4]oxazin-6-ylmethoxy]piperidin-3-yloxy}butan-2-ol
Starting from (3S,4S,5R)-4-(4-cyclopropylmethoxymethylphenyl)-5-[4-(3-methoxypropyl)-3,4-dihydro-2H-benzo[1,4]oxazin-6-ylmethoxy]-1-(toluene-4-sulphonyl)piperidin-3-ol (Example 43b) using tert-butyl ((R)-3-iodo-1-methylpropoxy)dimethylsilane [109715-47-1].
- 132: (R)-4-{(3S,4S,5R)-4-[4-((S)-3-Methoxy-2-methylpropoxymethyl)phenyl]-5-4-(3-methoxypropyl)-3,4-dihydro-2H-benzo[1,4]oxazin-6-ylmethoxy]piperidin-3-yloxy}-butan-2-ol
Starting from (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-ol (Example 43a) using tert-butyl((R)-3-iodo-1-methylpropoxy)dimethylsilane [109715-47-1].
- 134: (S)-4-{(3S,4S,5R)-4-[4-((S)-3-Methoxy-2-methylpropoxymethyl)phenyl]-5-[4-(3-methoxypropyl)-3,4-dihydro-2H-benzo[1,4]oxazin-6-ylmethoxy]piperidin-3-yloxy}-butan-2-ol
Starting from (3S,4S,5R)-4-[4-((S)-3-methoxy-2-methylpropoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-ol (Example 43a).

### Example 137

### N-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-ylmethyl}acetamide

The title compound is prepared by the process described in Example 105 and 109

The following compound is prepared in an analogous manner to the process described in Example 137:

### Example

- 139: N-{(3S,4R,5R)-4-(4-Cyclopropylmethoxymethylphenyl)-5-[4-(3-methoxypropyl)-3,4-dihydro-2H-benzo[1,4]oxazin-6-ylmethoxy]piperidin-3-ylmethyl}pentanamide

### Example 146

### N-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-ylmethyl}morpholine-4-carboxamide

The title compound is prepared from N-[(3R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-ylmethyl]morpholine-4-carboxamide in analogy to method L.

The starting material is prepared as follows:
a) N-[(3R,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl]-morpholine-4-carboxamide
   3 mmol of triethylamine are added to a solution of 1 mmol of C-[(3R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yl]methylamine (Example 109b) and 1.1 mmol of morpholine-4-carbonyl chloride [15159-40-7] in 20 ml of dichloromethane at 0°C. After 1.5 hours, the reaction mixture is poured into 1 M sodium bicarbonate solution and extracted with tert-butyl methyl ether (3×), and the combined organic phases are washed with brine, dried with sodium sulphate and evaporated. The title compound is obtained as a yellowish oil from the residue by flash chromatography (SiO₂ 60F). Rf = 0.29 (dichloromethane/methanol/ 25% conc. ammonia = 200:20:1); Rt = 4.58 (gradient I).

### Example 151

### (R)-1-((3S,4S,5R)-4-[4-(2-Methoxyethoxymethyl)phenyl]-5-{2-[4-(3-methoxypropyl)-3,4-dihydro-2H-benzo[1,4]oxazin-6-yl]ethyl}piperidin-3-yloxy)propan-2-ol

The title compound is prepared from 0.420 g of (R)-1-[(3S,4S,5R)-4-[4-(2-methoxyethoxymethyl)phenyl]-5-{2-[4-(3-methoxypropyl)-3,4-dihydro-2H-benzo[1,4]oxazin-6-yl]ethyl}-1-(toluene-4-sulphonyl)piperidin-3-yloxy]propan-2-ol in analogy to method L.

The starting materials are prepared as follows:
a) (R)-1-[(3S,4S,5R)-4-[4-(2-Methoxyethoxymethyl)phenyl]-5-{2-[4-(3-methoxypropyl)-3,4-dihydro-2H-benzo[1,4]oxazin-6-yl]ethyl}-1-(toluene-4-sulphonyl)piperidin-3-yloxy]propan-2-ol
   0.10 g of sodium borohydride is added to a solution of 0.67 g of 6-{2-[(3R,4S,5S)-4-[4-(2-methoxyethoxymethyl)phenyl]-5-((R)-1-oxiranylmethoxy)-1-(toluene-4-sulphonyl)piperidin-3-yl]ethyl}-4-(3-methoxypropyl)-3,4-dihydro-2H-benzo[1,4]oxazine in 10 ml of ethanol and 0.75 ml of tetrahydrofuran, and the mixture is stirred at 45°C for 18 hours. The reaction mixture is poured into 1 M ammonium chloride (50 ml) and extracted with tert-butyl methyl ether (2 × 50 ml). The combined organic phases are washed with brine (50 ml), dried with sodium sulphate and evaporated. The title compound is obtained as a yellowish oil from the residue by flash chromatography (SiO₂ 60F). Rf = 0.17 (EtOAc/heptane 3: 1); Rt = 5.14 (gradient I).
b) 6-{2-[(3R,4S,5S)-4-[4-(2-Methoxyethoxymethyl)phenyl]-5-((R)-1-oxiranylmethoxy)-1-(toluene-4-sulphonyl)piperidin-3-yl]ethyl}-4-(3-methoxypropyl)-3,4-dihydro-2H-benzo[1,4]oxazine
   1.20 g of (3S,4S,5R)-4-[4-(2-methoxyethoxymethyl)phenyl]-5-{2-[4-(3-methoxypropyl)-3,4-dihydro-2H-benzo[1,4]oxazin-6-yl]ethyl}-1-(toluene-4-sulphonyl)piperidin-3-ol are reacted in analogy to Example 68a. The title compound is obtained as a yellowish oil. Rf = 0.25 (EtOAc/heptane 3:1); Rt = 5.33 (gradient I).
c) (3S,4S,5R)-4-[4-(2-Methoxyethoxymethyl)phenyl]-5-{2-[4-(3-methoxypropyl)-3,4-dihydro-2H-benzo[1,4]oxazin-6-yl]ethyl}-1-(toluene-4-sulphonyl)piperidin-3-ol
   2.0 g of 6-{2-[(3R,4S,5S)-4-[4-(2-methoxyethoxymethyl)phenyl]-1-(toluene-4-sulphonyl)-5-triisopropylsilanyloxypiperidin-3-yl]ethyl}-4-(3-methoxypropyl)-3,4-dihydro-2H-benzo[1,4]oxazine are reacted in analogy to method J. The title compound is obtained as a yellow oil. Rf = 0.14 (EtOAc/heptane 3:1); Rt = 4.95 (gradient I).
d) 6-{2-[(3R,4S,5S)-4-[4-(2-methoxyethoxymethyl)phenyl]-1-(toluene-4-sulphonyl)-5-triisopropylsilanyloxypiperidin-3-yl]ethyl}-4-(3-methoxypropyl)-3,4-dihydro-2H-benzo[1,4]oxazine
   0.747 ml of 2-bromoethyl methyl ether and 1.48 g of tetrabutylammonium iodide are successively added to a stirred solution of 3.0 g of {4-[(3R,4S,5S)-3-{2-[4-(3-methoxypropyl)-3,4-dihydro-2H-benzo[1,4]oxazin-6-yl]ethyl}-1-(toluene-4-sulphonyl)-5-triisopropylsilanyloxy-piperidin-4-yl]phenyl}methanol in 15 ml of N,N-dimethylformamide. The mixture is cooled to -5°C and, after addition of 0.316 g of sodium hydride dispersion (60% in oil), stirred at room temperature for 24 hours. The reaction mixture is poured into ice-water (60 ml) and extracted with dichloromethane (3 × 60 ml). The combined organic phases are washed with water (2 × 150 ml) and brine (150 ml), dried with sodium sulphate and evaporated. The title compound is obtained as a yellowish oil from the residue by flash chromatography (SiO₂ 60F). Rf = 0.26 (EtOAc/heptane 1:1).
e) {4-[(3R,4S,5S)-3-{2-[4-(3-Methoxypropyl)-3,4-dihydro-2H-benzo[1,4]oxazin-6-yl]ethyl}-1-(toluene-4-sulphonyl)-5-triisopropylsilanyloxypiperidin-4-yl]phenyl}methanol
   4.50 g of 4-[(3R,4S,5S)-3-{2-[4-(3-methoxypropyl)-3-oxo-3,4-dihydro-2H-benzo[1,4]oxazin-6-yl]ethyl}-1-(toluene-4-sulphonyl)-5-triisopropylsilanyloxypiperidin-4-yl]benzoic acid are reacted in analogy to method K. The title compound is obtained as a white faom. Rf = 0.19 (EtOAc/heptane 1:1).
f) 4-[(3R,4S,5S)-3-{2-[4-(3-Methoxypropyl)-3-oxo-3,4-dihydro-2H-benzo[1,4]oxazin-6-yl]-ethyl}-1-(toluene-4-sulphonyl)-5-triisopropylsilanyloxypiperidin-4-yl]benzoic acid
   6.0 g of methyl 4-[(3R,4S,5S)-3-{2-[4-(3-methoxypropyl)-3-oxo-3,4-dihydro-2H-benzo[1,4]oxazin-6-yl]ethyl}-1-(toluene-4-sulphonyl)-5-triisopropylsilanyloxypiperidin-4-yl]benzoate are reacted in analogy to Example 42g. The title compound is identified on the basis of the Rf. The title compound is obtained as a white faom. Rf = 0.05 (EtOAc/heptane 1:1); Rt = 6.27 (gradient I).
g) Methyl 4-[(3R,4S,5S)-3-{2-[4-(3-methoxypropyl)-3-oxo-3,4-dihydro-2H-benzo[1,4]oxazin-6-y]lethyl}-1-(toluene-4-sulphonyl)-5-triisopropylsilanyloxypiperidin-4-yl]benzoate
   A solution of 7.25 g of methyl 4-[(3R,4S,5S)-3-{2-[4-(3-methoxypropyl)-3-oxo-3,4-dihydro-2H-benzo[1,4]oxazin-6-yl]vinyl}-1-(toluene-4-sulphonyl)-5-triisopropylsilanyloxypiperidin-4-yl]benzoate in 80 ml of ethanol is hydrogenated in the presence of 0.80 g of Pd/C 10% at room temperature for 2 hours. The reaction mixture is clarified by filtration and the filtrate is evaporated. The title compound is obtained and identified on the basis of the Rf. The title compound is obtained as a white foam from the residue by flash chromatography (SiO₂ 60F). Rf = 0.28 (EtOAc/heptane 1:1); Rt = 6.81 (gradient I).
h) Methyl 4-[(3R,4S,5S)-3-{2-[4-(3-methoxypropyl)-3-oxo-3,4-dihydro-2H-benzo[1,4]oxazin-6-yl]vinyl}-1-(toluene-4-sulphonyl)-5-triisopropylsilanyloxypiperidin-4-yl]benzoate
   10.0 ml of n-butyllithium (1.6M in hexane) are added to a stirred suspension of 11.90 g of [4-(3-methoxypropyl)-3-oxo-3,4-dihydro-2H-benzo[1,4]oxazin-6-ylmethyl]triphenylphosphonium chloride (Example 151 s) and 100 ml of tetrahydrofuran at 0°C, and the mixture is stirred at room temperature for 1 hour. A solution of 8.0 g of methyl 4-[(3R,4S,5S)-3-formyl-1-(toluene-4-sulphonyl)-5-triisopropylsilanyloxypiperidin-4-yl]benzoate in 50 ml of tetrahydrofuran is added to the reaction mixture over the course of 10 minutes, and the mixture is then stirred at room temperature for 4 hours. The reaction mixture is poured into 1M ammonium chloride solution (250 ml) and extracted with tert-butyl methyl ether (2 × 250 ml). The combined organic phases are washed with brine (250 ml), dried with sodium sulphate and evaporated. The title compound is obtained as a white foam from the residue by flash chromatography (SiO₂ 60F). Rf = 0.10 (EtOAc/heptane 1:2); Rt = 6.67 (gradient I).
i) Methyl 4-[(3R,4S,5S)-3-formyl-1-(toluene-4-sulphonyl)-5-triisopropylsilanyloxypiperidin-4-yl]benzoate
   8.10 g of 3A molecular sieves and 2.54 g of 4-methylmorpholine N-oxide are added to a stirred solution of 8.10 g of methyl 4-[(3R,4S,5S)-3-hydroxymethyl-1-(toluene-4-sulphonyl)-5-triisopropylsilanyloxypiperidin-4-yl]benzoate in 160 ml of dichloromethane, and the mixture is stirred at room temperature for 10 minutes. 0.247 g of tetra-N-propylammonium perruthenate(VII) is added to the reaction mixture, which is then stirred at room temprature for 20 minutes. The resulting mixture is clarified by filtration and the filtrate is washed successively with 2M sodium sulphite (80 ml), brine (80 ml) and 2M copper(II) sulphate (80 ml). The organic phase is dried with sodium sulphate and evaporated. The title compound is obtained as a brown oil from the residue. Rf = 0.28 (EtOAc/heptane 1:2); Rt = 6.36 (gradient I).
j) Methyl 4-[(3R,4S,5S)-3-hydroxymethyl-1-(toluene-4-sulphonyl)-5-triisopropylsilanyloxy-piperidin-4-yl]benzoate
   1.90 g of p-toluenesulphonic acid are added to a stirred solution of 8.18 g of methyl 4-[(3S,4S,5R)-1-(toluene-4-sulphonyl)-3-triisopropylsilanyloxy-5-trityloxymethylpiperidin-4-yl]benzoate and 100 ml of methanol/tetrahydrofuran (1:1) at 0°C, and then the mixture is stirred for 20 hours at room temperature. The reaction mixture is poured into ice-cold 1M NaOH (250 ml) and extracted with tert-butyl methyl ether (2 × 250 ml). The combined organic phases are washed with brine (250 ml), dried with sodium sulphate and evaporated. The title compound is obtained as a white foam from the residue by flash chromatography (SiO₂ 60F). Rf = 0.13 (EtOAc/heptane 1:2); Rt = 6.31 (gradient I).
k) Methyl 4-[(3S,4S,5R)-1-(toluene-4-sulphonyl)-3-triisopropylsilanyloxy-5-trityloxymethyl-piperidin-4-yl]benzoate
   4.85 ml of triisopropyl trifluoromethanesulphonate are added to a solution of 9.92 g of methyl 4-[(3S,4S,5R)-3-hydroxy-1-(toluene-4-sulphonyl)-5-trityloxymethylpiperidin-4-yl]benzoate, 2.61 ml of 2,6-lutidine in 150 ml of dichloromethane over the course of 10 minutes at 0°C, and the mixture is stirred for 3 hours. The reaction mixture is poured into ice-water (250 ml) and extracted with tert-butyl methyl ether (2 × 250 ml). The organic phases are washed with brine (250 ml), dried with sodium sulphate and evaporated. The title compound is obtained as a colourless oil from the residue by flash chromatography (SiO₂ 60F). Rf = 0.39 (EtOAc/heptane 1:2); Rt = 7.25 (gradient I).
l) Methyl 4-[(3S,4S,5R)-3-hydroxy-1-(toluene-4-sulphonyl)-5-trityloxymethylpiperidin-4-yl]benzoate
   A mixture of 0.29 g of trityl chloride, 0.43 g of methyl 4-[(3S,4S,5R)-3-hydroxy-5-hydroxymethyl-1-(toluene-4-sulphonyl)-piperidin-4-yl]-benzoate and 0.006 g of 4-dimethylaminopyridine is diluted with 2 ml of pyridine and then the reaction mixture is stirred at 70°C for 12 hours. The reaction mixture is evaporated, diluted with 1:1 ice/1N aqueous hydrochloric acid and extracted twice with tert-butyl methyl ether. The combined organic phases are washed with 1M aqueous sodium bicarbonate solution and brine, dried with sodium sulphate and evaporated. The title compound is obtained as a white foam from the residue by flash chromatography (SiO₂ 60F). Rf = 0.28 (EtOAc/heptane 1:1); Rt = 5.65 (gradient I).
m) Methyl 4-[(3S,4S,5R)-3-hydroxy-5-hydroxymethyl-1-(toluene-4-sulphonyl)-piperidin-4-yl]-benzoate
   14.62 g of 4-[(3S,4S,5R)-3-hydroxy-5-hydroxymethyl-1-(toluene-4-sulphonyl)piperidin-4-yl]phenyl trifluoromethanesulphonate are reacted in analogy to Example 42i. The title compound is obtained as a yellowish foam. Rf = 0.25 (EtOAc/heptane 2:1); Rt = 3.63 (gradient I).
n) 4-[(3S,4S,5R)-3-Hydroxy-5-hydroxymethyl-1-(toluene-4-sulphonyl)piperidin-4-yl]phenyl trifluoromethanesulphonate
   11.30 g of (3S,4S,5R)-5-hydroxymethyl-4-(4-hydroxyphenyl)-1-(toluene-4-sulphonyl)piperidin-3-ol are reacted in analogy to Example 42j. The title compound is obtained as white crystals. Rf = 0.43 (dichloromethane/methanol 95:5); Rt = 4.32 (gradient I).
o) (3S,4S,5R)-5-Hydroxymethyl-4-(4-hydroxyphenyl)-1-(toluene-4-sulphonyl)piperidin-3-ol
   8.90 g of (3S,4S,5R)-5-hydroxymethyl-4-(4-hydroxyphenyl)piperidin-3-ol are reacted in analogy to Example 42d. The title compound is obtained as a white solid. Rf = 0.06 (EtOAc/heptane 1:1); Rt = 3.19 (gradient I).
p) (3S,4S,5R)-5-Hydroxymethyl-4-(4-hydroxyphenyl)piperidin-3-ol
   17.2 g of (3S,4S,5R)-1-benzyl-5-hydroxymethyl-4-(4-hydroxyphenyl)piperidin-3-ol hydrobromide are reacted in analogy to method B. The title compound is obtained as a yellow oil. Rt = 083 (gradient I)
q) (3S,4S,5R)-1-Benzyl-5-hydroxymethyl-4-(4-hydroxyphenyl)piperidin-3-ol hydrobromide
   160 ml of 1M boron tribromide (in dichloromethane) are added over the course of 15 minutes to a solution of 22.8 g of (3S,4S,5R)-1-benzyl-4-(4-methoxyphenyl)-5-trityloxymethylpiperidin-3-ol and 900 ml of dichloromethane at 0°C, and the mixture is stirred for 1 hour. The mixture is cooled to -15°C, and the crystals are filtered off with suction. The material on the filter is taken up in 900 ml of methanol and then evaporated to dryness in a rotary evaporator. The title compound is obtained as a yellow foam from the residue by flash chromatography (SiO₂ 60F). Rt = 2.17 (gradient I).
r) (3S,4S,5R)-1-Benzyl-4-(4-methoxyphenyl)-5-trityloxymethylpiperidin-3-ol
   1.46 g of (D)-(-)-mandelic acid are added to a solution of 9.12 g of (R,S)-(3S,4S,5R)-1-benzyl-4-(4-methoxyphenyl)-5-trityloxymethylpiperidin-3-ol [188879-88-1] in 110 ml of tetrahydrofuran at 60°C (oil bath temperature). 110 ml of n-hexane are slowly added dropwise at 60°C. The mixture is slowly cooled to room temperature over the course of 3 hours and, after a brief treatment in an ultrasonic bath, then cooled at 0°C for 2 hours. The precipitate is filtered off and washed with tetrahydrofuran/n-hexane 1:3 (2 × 20 ml). The salt is dissolved in ethyl acetate and washed with saturated aqueous sodium carbonate solution (2×). The combined organic phases are washed with brine, dried with sodium sulphate and evaporated. The title compound is obtained as a white foam from the residue. Rf = 0.20 (EtOAc/heptane 2:1); Rt = 24.10 (chiralpak AD 0.46 × 25 cm daicel; 95% hexane/5% isopropanol flow. 0.7 ml/minute (total 60 minutes).
s) [4-(3-Methoxypropyl)-3-oxo-3,4-dihydro-2H-benzo[1,4]oxazin-6-ylmethyl]triphenylphosphonium chloride
   10.3 g of triphenylphosphine are added to a stirred solution of 10.0 g of 6-chloromethyl-4-(3-methoxypropyl)-4H-benzo[1,4]oxazin-3-one (Example 42o) in 100 ml of xylene, and the mixture is refluxed for 18 hours. The reaction mixture is cooled to room temperature, and the solid is filtered off with suction. The title compound is obtained aswhite crystals. Rt = 3.93 (gradient I).

### Example 152

### (3S,4S,5R)-4-[4-((1S,2S)-2-Methoxycyclopropylmethoxymethyl)phenyl]-5-[4-(3-methoxypropyl)-3,4-dihydro-2H-benzo[1,4]oxazin-6-ylmethoxy]piperidin-3-ol

The title compound is prepared from (3S,4S,5R)-4-[4-((1S,2S)-2-methoxycyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol in analogy to method L and identified on the basis of the Rf.

The starting materials are prepared as follows:
a) (3S,4S,5R)-4-f4-((1S,2S)-2-Methoxycyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol
   The title compound is prepared from 6-[(3R,4R,5S)-4-[4-((1S,2S)-2-methoxycyclopropylmethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine in analogy to method J and identified on the basis of the Rf.
b) 6-[(3R,4R,5S)-4-[4-((1S,2S)-2-Methoxycyclopropylmethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   The title compound is prepared starting from 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) and ((1 R,2S)-2-methoxycyclopropyl)-methanol in analogy to Example 42b and identified on the basis of the Rf.
c) ((1R,2S)-2-Methoxycyclopropyl)methanol
   0.560 g of lithium borohydride is added to a solution of 4.410 g of (R)-4-benzyl-3-((1S,2S)-2-methoxycyclopropanecarbonyl)oxazolidin-2-one in 40 ml of tetrahydrofuran and 1 ml of methanol at 0°C. After the addition is complete, the reaction mixture is stirred for 3 hours at 0°C, and then phosphate buffer is added with pH 7. The mixture is extracted with ethyl acetate, and the combined organic extracts are washed with brine, dried with sodium sulphate and evaporated. The title compound is obtained from the residue by flash chromatography (SiO₂ 60F) and identified on the basis of the Rf.
d) (R)-4-Benzyl-3-((1S,2S)-2-methoxycyclopropanecarbonyl)oxazolidin-2-one and (R)-4-Benzyl-3-((1 R,2R)-2-methoxycyclopropanecarbonyl)oxazolidin-2-one
   A solution of 2.000 g of (R)-benzyl-2-oxazolidinone in 11 ml of dry tetrahydrofuran is cooled to -75°C. 7.10 ml of n-butyllithium solution (1.6M in hexane) are added dropwise to the solution at -75 - -70°C. After the addition is complete, the reaction mixture is stirred at -75°C for 10 minutes and then a solution of 1.346 g of trans-2-methoxycyclopropanecarbonyl chloride in 10 ml of tetrahydrofuran is added. The reaction solution is warmed to room temperature and saturated aqueous ammonium chloride solution is added, and the mixture is extracted with tert-butyl methyl ether. The combined organic extracts are washed with brine, dried with sodium sulphate and evaporated. The title compounds are identified on the basis of the Rf from the residue by flash chromatography (SiO₂ 60F).
e) trans-2-Methoxycyclopropanecarbonyl chloride
   1.01 ml of oxalyl chloride are added to a solution of 1.160 g of trans-2-methoxycyclopropanecarboxylic acid [60212-42-2] in 10 ml of dichloromethane at 0°C. One drop of N,N-dimethylformamide is added, and the reaction solution is stirred at 0°C for one hour and then evaporated. The residue is employed without further purification in the next stage.

### Example 153

### (3S,4S,5R)-4-[4-((11S,2S)-2-Methoxymethylcyclopropylmethoxymethyl)phenyl]-5-[4-(3-methoxypropyl)-3,4-dihydro-2H-benzo[1,4]oxazin-6-ylmethoxy]piperidin-3-ol

The title compound is prepared from (3S,4S,5R)-4-[4-((1S,2S)-2-methoxymethylcyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol in analogy to method L, and identified on the basis of the Rf.

The starting materials are prepared as follows:
a) (3S,4S,5R)-4-[4-((1S,2S)-2-Methoxymethylcyclopropylmethoxymethyl)phenyl]-5-[4-(3-methoxypropyl)-3,4-dihydro-2H-benzo[1,4]oxazin-6-ylmethoxyl-1-(toluene-4-sulphonyl)-piperidin-3-ol
   The title compound is obtained from 6-[(3R,4R,5S)-4-[4-2-((1S,2S)-2-methoxymethylcyclopropylmethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine in analogy to method J, and identified on the basis of the Rf.
b) 6-[(3R,4R,5S)-4-[4-2-((1S,2S)-2-Methoxymethylcyclopropylmethoxymethyl)phenyl]-1-(toluene-4-sulphonyl)-5-triisopropylsilanyloxypiperidin-3-yloxymethyl]-4-(3-methoxypropyl)-3,4-dihydro-2H-benzo[1,4]oxazine
   0.032 g of sodium hydride (60% dispersion in paraffin) is taken up in 5 ml of N,N-dimethylformamide, and the suspension is cooled to -10°C. A solution of 0.0406 g of ((1 S,2S)-2-methoxymethylcyclopropyl)methanol in 2 ml of N,N-dimethylformamide is added dropwise over the course of 5 minutes, and the reaction mixture is then stirred at -10°C for 10 minutes. A solution of 0.400 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c) in 3 ml of N,N-dimethylformamide is added dropwise, and the reaction mixture is stirred at room temperature for 16 hours. The reaction mixture is poured into water, and the aqueous phase is extracted with tert-butyl methyl ether. The combined organic extracts are washed with brine, dried with sodium sulphate and evaporated. The title compound is obtained as a colourless oil from the residue by flash chromatography (SiO₂ 60F). Rf = 0.31 (EtOAc/heptane 1:2).
c) ((1S,2S)-2-Methoxymethylcyclopropyl)methanol
   A suspension of 0.083 g of lithiumaluminium hydride in 5 ml of diethyl ether is cooled to 0°C. A solution of 0.220 g of ethyl (1S,2S)-2-methoxymethylcyclopropanecarboxylate in 5 ml of diethyl ether is added dropwise at 0°C, and the reaction mixture is stirred at this temperature for 2 hours. Water, 4M sodium hydroxide solution and again water are successively added to the reaction mixture, the resulting solid is filtered off through Hyflo, the filter cake is washed with diethyl ether, and the filtrate is evaporated. The title compound is obtained as a colourless liquid and employed without further purification in the next stage.
d) Ethyl (1S,2S)-2-methoxymethylcyclopropanecarboxylate
   4.60 ml of triethyl phosphonoacetate are added dropwise over 5 minutes to a suspension of 0.940 g of sodium hydride (60% dispersion in oil) in 10 ml of toluene. The reaction mixture is stirred for 10 minutes and then 1.01 g of (R)-(-)-glycidyl methyl ether are added, and the mixture is heated to reflux for 16 hours. The reaction mixture is cooled to room temperature and diluted with tert-butyl methyl ether, and saturated aqueous ammonium chloride solution is added. The phases are separated, the aqueous phase is extracted with tert-butyl methyl ether, and the combined organic phases are washed with brine, dried with sodium sulphate and evaporated. The title compound is obtained as a yellowish oil from the residue by flash chromatography (SiO₂ 60F). Rf = 0.10 (diethyl ether/hexane 1:4).

### Example 154

### (R)-1-{(3S,4R,5R)-4-[4-((1S,2S)-2-Methoxycyclopropylmethoxymethyl)phenyl]-5-[4-(3-methoxypropyl)-3,4-dihydro-2H-benzo[1,4]oxazin-6-ylmethoxy]piperidin-3-yloxy}propan-2-ol

The title compound is obtained starting from (3S,4S,5R)-4-[4-((1S,2S)-2-methoxycyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 152a) in analogy to the process described in Example 68. Deprotection of the protective group on the nitrogen (last stage of the synthesis) is carried out in analogy to method L. The title compound is identified on the basis of the Rf.

### Example 155

### (R)-1-{(3S,4R,5R)-4-[4-((1S,2S)-Methoxymethylcyclopropylmethoxymethyl)phenyl]-5-[4-(3-methoxypropyl)-3,4-dihydro-2H-benzo[1,4]oxazin-6-ylmethoxy]piperidin-3-yloxy}propan-2-ol

The title compound is obtained starting from (3S,4S,5R)-4-[4-((1S,2S)-2-methoxymethylcyclopropylmethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 153a) in analogy to the process described in Example 68. Deprotection of the protective group on the nitrogen (last stage of the synthesis) is carried out in analogy to method L. The title compound is identified on the basis of the Rf.

### Example 156

### (3S,4S,5R)-4-[4-(2-Methoxyethoxymethyl)phenyl]-5-[4-(3-methoxypropyl)-2,2-dimethyl-2H-chromen-6-ylmethoxylpiperidin-3-ol

The title compound is prepared from (3S,4S,5R)-4-[4-(2-methoxyethoxymethyl)phenyl]-5-[4-(3-methoxypropyl)-2,2-dimethyl-2H-chromen-6-ylmethoxy]-1-(toluene-4-sulphonyl)-piperidin-3-ol in analogy to method L.

The starting materials are prepared as follows:
a) (3S,4S,5R)-4-[4-(2-Methoxyethoxymethyl)phenyl]-5-[4-(3-methoxypropyl)-2,2-dimethyl-2H-chromen-6-ylmethoxy]-1-(toluene-4-sulphonyl)piperidin-3-ol
   The title compound is prepared from (3R,4R,5S)-4-[4-(2-methoxyethoxymethyl)phenyl]-3-[4-(3-methoxypropyl)-2,2-dimethyl-2H-chromen-6-ylmethoxy]-1-(toluene-4-sulphonyl)-5-triisopropylsilanyloxypiperidine in analogy to method J. The title compound is obtained as a yellowish oil. Rf = 0.28 (EtOAc/heptane 2:1); Rt = 5.31 (gradient I).
b) (3R,4R,5S)-4-[4-(2-Methoxyethoxymethyl)phenyl]-3-[4-(3-methoxypropyl)-2,2-dimethyl-2H-chromen-6-ylmethoxy]-1-(toluene-4-sulphonyl)-5-triisopropylsilanyloxypiperidine
   The title compound is prepared from {4-[(3R,4R,5S)-3-[4-(3-methoxypropyl)-2,2-dimethyl-2H-chromen-6-ylmethoxy]-1-(toluene-4-sulphonyl)-5-triisopropylsilanyloxypiperidin-4-yl]-phenyl}methanol in analogy to Example 59c. The title compound is obtained as a yellowish oil. Rf = 0.32 (EtOAc/heptane 4:1); Rt = 7.24 (gradient I).
c) {4-[(3R,4R,5S)-3-[4-(3-Methoxypropyl)-2,2-dimethyl-2H-chromen-6-ylmethoxy]-1-(toluene-4-sulphonyl)-5-triisopropylsilanyloxypiperidin-4-yl]phenyl}methanol
   A solution of 8.060 g of methyl 4-[(3R,4R,5S)-3-[4-(3-methoxypropyl)-2,2-dimethyl-2H-chromen-6-ylmethoxy]-1-(toluene-4-sulphonyl)-5-triisopropylsilanyloxypiperidin-4-yl]-benzoate in 50 ml of diethyl ether is added dropwise to a suspension of 0.759 g of lithium aluminium hydride in 50 ml of diethyl ether at 0°C. After the addition is complete, the reaction mixture is stirred at 0°C for 1 hour. Water, 4M sodium hydroxide solution and water are successively added to the reaction mixture, the resulting solid is filtered off through Hyflo, the filter cake is washed with diethyl ether, and the filtrate is evaporated. The title compound is obtained as a yellowish oil. Rf = 0.29 (EtOAc/heptane 1:2); Rt = 6.72 (gradient I)
d) Methyl 4-[(3R,4R,5S)-3-[4-(3-methoxypropyl)-2,2-dimethyl-2H-chromen-6-ylmethoxy]-1-(toluene-4-sulphonyl)-5-triisopropylsilanyloxypiperidin-4-yl]benzoate
   The title compound is obtained from methyl 4-[(3R,4R,5S)-3-hydroxy-1-(toluenesulphonyl)-5-triisopropylsilanyloxypiperidin-4-yl]benzoate and 6-bromomethyl-4-(3-methoxypropyl)-2,2-dimethyl-2H-chromene in analogy to method D. The title compound is obtained as a yellow oil. Rt = 7.13 (gradient I).
e) Methyl 4-[(3R,4R,5S)-3-hydroxy-1-(toluenesulphonyl)-5-triisopropylsilanyloxypiperidin-4-yl]benzoate
   130 ml of saturated aqueous sodium bicarbonate solution are added to a solution of 6.170 g of methyl 4-((3R,4R,5S)-3-hydroxy-5-triisopropylsilanyloxypiperidin-4-yl)benzoate in 130 ml of ethyl acetate. 3.210 g of p-toluenesulphonyl chloride are added in portions while stirring vigorously. The reaction mixture is stirred at 0°C for a further 2 hours and then the phases are separated. The aqueous phase is back-extracted with ethyl acetate, and the combined organic phases are washed with brine, dried with sodium sulphate and evaporated. The title compound is obtained as a white foam from the residue by flash chromatography (SiO₂ 60F). Rf = 0.63 (EtOAc/heptane 1:2); Rt = 6.28 (gradient I).
f) Methyl 4-((3R,4R,5S)-3-hydroxy-5-triisopropylsilanyloxypiperidin-4-yl)benzoate
   The title compound is obtained as a white foam from 5.400 g of benzyl (3R,4R,5S)-3-hydroxy-4-(4-methoxycarbonylphenyl)-5-triisopropylsilanyloxypiperidine-1-carboxylate (Example 42i) in analogy to method B. Rf = 0.36 (dichloromethane/methanol/25% conc. ammonia 200:20:1); Rt = 4.36 (gradient I).
g) 6-Bromomethyl-4-(3-methoxypropyl)-2,2-dimethyl-2H-chromene
   1.560 ml of trimethylsilyl bromide are added dropwise to a solution of 2.067 g of [4-(3-methoxypropyl)-2,2-dimethyl-2H-chromen-6-yl]methanol in chloroform at room temperature. The reaction solution is stirred at room temperature for 30 minutes and then evaporated. The title compound is obtained from the residue by flash chromatography (SiO₂ 60F) and identified on the basis of the Rf.
h) [4-(3-Methoxypropyl)-2,2-dimethyl-2H-chromen-6-yl]methanol
   0.0278 g of lithium borohydride is added in portions to a solution of 0.316 g of 4-(3-methoxypropyl)-2,2-dimethyl-2H-chromene-6-carbaldehyde in 5 ml of dry tetrahydrofuran at 0°C. The reaction mixture is stirred at 0°C for 2 hours and then 5 ml of methanol and 0.5 ml are added, and the mixture is evaporated. The title compound is obtained as a white solid and employed without further purification in the next stage. Rt = 4.00 (gradient I).
i) 4-(3-Methoxypropyl)-2,2-dimethyl-2H-chromene-6-carbaldehyde
   A solution of 1.000 g of 6-bromo-4-(3-methoxypropyl)-2,2-dimethyl-2H-chromene in 12 ml of dry tetrahydrofuran is cooled to -78°C. 1.77 ml of n-butyllithium solution (1.6M in hexane) are added dropwise at -78° - -70°C, and the reaction solution is then stirred at -78°C for 30 minutes. 0.398 ml of N,N-dimethylformamide is added dropwise, the solution is stirred at the same temperature for a further 45 minutes, and then saturated aqueous ammonium chloride solution is added. The mixture is warmed to room temperature and extracted with tert-butyl methyl ether. The combined organic extracts are washed with brine, dried with sodium sulphate and evaporated. The total compound is obtained as a colourless oil from the residue by flash chromatography (SiO₂ 60F). Rt = 4.79 (gradient I).
j) 6-Bromo-4-(3-methoxypropyl)-2,2-dimethyl-2H-chromene
   A solution of 17.10 g of 6-bromo-4-(3-methoxyprop-1-ynyl)-2,2-dimethyl-2H-chromene in 600 ml of ethyl acetate is mixed with 3.10 ml of acetic acid. The reaction mixture is cooled to -15 to -10°C, and 8.88 g of 10% Pd/C are added and a hydrogen atmosphere is provided by means of a balloon. The reaction mixture is then stirred at 0-25°C for 1 hour. The catalyst is subsequently filtered off through Hyflo, and the filtrate is washed with saturated aqueous sodium bicarbonate solution. The organic phase is dried with sodium sulphate and evaporated. The title compound is obtained as a yellowish oil from the residue by flash chromatography (SiO₂ 60F). Rf = 0.28 (EtOAc/heptane 1:5); Rt = 5.66 (gradient I).
k) 6-Bromo-4-(3-methoxyprop-1-ynyl)-2,2-dimethyl-2H-chromene
   497 ml of triethylamine are added to a suspension of 2.518 g of bis(triphenylphosphine)-palladium(II) chloride and 0.683 g of copper(I) iodide in 500 ml of dry tetrahydrofuran at room temperature. A solution of 29.60 g of 6-bromo-2,2-dimethyl-2H-chromen-4-yl trifluoromethanesulphonate and 7.698 g of methyl 2-propynyl ether in 200 ml of tetrahydrofuran is added, and the reaction mixture is heated to 50°C. The mixture is stirred at this temperature for 1.5 hours and then evaporated. The title compound is obtained as a yellowish oil from the residue by flash chromatography (SiO₂ 60F). Rf = 0.45 (EtOAc/heptane 1:10); Rt = 5.44 (gradient I).
l) 6-Bromo-2,2-dimethyl-2H-chromen-4-yl trifluoromethanesulphonate
   20.0 ml of N,N-diisopropylethylamine are added to a solution of 21.00 g of 6-bromo-2,2-dimethylchroman-4-one [99853-21-1] in 200 ml of dichloromethane at -15°C. 20.6 ml of trifluoromethanesulphonic anhydride are added dropwise over the course of 10 minutes at -15°C, and the reaction solution is then stirred at room temperature for 16 hours. Water is added to the reaction mixture, the phases are separated, and the aqueous phase is back-extracted with dichloromethane. The combined organic phases are dried with sodium sulphate and evaporated. The title compound is obtained as a yellowish oil from the residue by flash chromatography (SiO₂ 60F). Rf = 0.55 (EtOAc/heptane 1:10); Rt = 5.84 (gradient I).

### Example 157

### (R)-1-Methoxy-3-{(3S,4R,5R)-4-[4-(2-methoxyethoxymethyl)phenyl]-5-[4-(3-methoxypropyl)-2,2-dimethyl-2H-chromen-6-ylmethoxy]piperidin-3-yloxy}propan-2-ol

The title compound is obtained starting from (3S,4S,5R)-4-[4-(2-methoxyethoxymethyl)phenyl]-5-[4-(3-methoxypropyl)-2,2-dimethyl-2H-chromen-6-ylmethoxy]-1-(toluene-4-sulphonyl)piperidin-3-ol (Example 156a) in analogy to the process described in Example 59.

### Example 158

### (R)-4-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-yl}-butan-2-ol

The title compound is prepared from (R)-4-[(3S,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-3-yl]butan-2-ol in analogy to method L.

The starting materials are prepared as follows:
a) (R)-4-[(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yl]butan-2-ol
   The title compound is prepared from 6-[(3R,4R,5S)-4-[4-(2-methoxyethoxymethyl)phenyl]-1-(toluene-4-sulphonyl)-5-((R)-3-triisopropylsilanyloxybutyl)piperidin-3-yloxymethyl]-4-(3-methoxypropyl)-3,4-dihydro-2H-benzo[1,4]oxazine in analogy to method J and identified on the basis of the Rf.
b) 6-[(3R,4R,5S)-4-[4-(2-Methoxyethoxymethyl)phenyl]-1-(toluene-4-sulphonyl)-5-((R)-3-triisopropylsilanyloxybutyl)piperidin-3-yloxymethyl]-4-(3-methoxypropyl)-3,4-dihydro-2H-benzo[1,4]oxazine
   The title compound is prepared from [(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-ylmethyl]triphenylphosphonium bromide and (R)-2-triisopropylsilanyloxypropionaldehyde [178802-51-2] in analogy to the process described in Example 151g-h and identified on the basis of the Rf.
c) [(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl]triphenylphosphonium bromide
   1.66 mmol of triphenylphosphine are added to a stirred solution of 1.37 mmol of 6-[(3R,4R,5S)-5-bromomethyl-4-[4-(2-methoxyethoxymethyl)phenyl]-1-(toluene-4-sulphonyl)piperidin-3-yloxymethyl]-4-(3-methoxypropyl)-3,4-dihydro-2H-benzo[1,4]oxazine in 2 ml of acetonitrile, and the mixture is kept at 80°C for 18 hours. The reaction mixture is cooled to room temperature, and the solid is filtered off with suction. The title compound is identified on the basis of the Rf.
d) 6-[(3R,4R,5S)-5-Bromomethyl-4-[4-(2-methoxyethoxymethyl)phenyl]-1-(toluene-4-sulphonyl)piperidin-3-yloxymethyl]-4-(3-methoxypropyl)-3,4-dihydro-2H-benzo[1,4]oxazine
   20 mmol of lithium bromide are added to a solution of 2 mmol of (3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl methanesulphonate in 5 ml of N,N-dimethylformamide, and the mixture is heated at 65°C for 14 hours. The reaction mixture is cooled to room temperature and quenched with water. It is extracted with tert-butyl methyl ether (3x), and the combined organic phases are dried with sodium sulphate and evaporated. The title compound is identified on the basis of the Rf from the residue by flash chromatography (SiO₂ 60F).
e) (3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ylmethyl methanesulphonate
   6 mmol of methanesulphonyl chloride are added to a solution of 3 mmol of [(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yl]methanol (Example 105c) and 15 mmol of triethylamine in 30 ml of dichloromethane at 0°C, and the mixture is stirred at 0°C for 1 hour. It is diluted with dichloromethane and washed with 1 N HBr. The organic phase is dried with sodium sulphate and evaporated. The title compound is used without further purification in the next stage.

The following compound is prepared in an analogous manner to the process described in Example 158:

### Example

- 159: (R)-4-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yl}butan-2-ol
Using (S)-2-triisopropylsilanyloxypropionaldehyde [135614-51-7]

### Example 160

### N-((R)-2-{(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-3-yloxy}-1-methylethyl)acetamide

The title compound is prepared from (S)-2-[(3S,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-3-yloxy]-1-methylethyl toluene-4-sulphonate in analogy to the process described in Example 109.

The starting material is prepared as follows:
a) (S)-2-[(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]-1-methylethyl toluene-4-sulphonate
   The title compound is identified on the basis of the Rf from (S)-1-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol in analogy to method H.
b) (S)-1-[(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol
   The title compound is prepared from (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-ol (Example 59c) using (S)-1-oxiranymethyl toluene-4-sulphonate [70987-78-9] in analogy to the process described in Example 68, and identified on the basis of the Rf.

### Example 161

### 6-{(3R,4R,5S)-5-((R)-2-Ethoxypropoxy)-4-[4-(2-methoxyethoxymethyl)phenyl]piperidin-3-yloxymethyl}-4-(3-methoxypropyl)-3,4-dihydro-2H-benzo[1,4]oxazine

The title compound is prepared from 6-[(3R,4R,5S)-5-((R)-2-ethoxypropoxy)-4-[4-(2-methoxyethoxymethyl)phenyl]-1-(toluene-4-sulphonyl)piperidin-3-yloxymethyl]-4-(3-methoxypropyl)-3,4-dihydro-2H-benzo[1,4]oxazine in analogy to method L.

The starting materials are prepared as follows:
a) 6-[(3R,4R,5S)-5-((R)-2-Ethoxypropoxy)-4-[4-(2-methoxyethoxymethyl)phenyl]-1-(toluene-4-sulphonyl)piperidin-3-yloxymethyl]-4-(3-methoxypropyl)-3,4-dihydro-2H-benzo[1,4]oxazine
   1.0 mmol of (R)-1-[(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]-propan-2-ol and 1.5 mmol of ethyl iodide are reacted in analogy to method D. The title compound is identified on the basis of the Rf.
b) (R)-1-[(3S,4R,5R)-4-[4-(2-Methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy]propan-2-ol
   The title compound is prepared from (3S,4S,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)-piperidin-3-ol (Example 59c) in analogy to the process described in Example 68, and identified on the basis of the Rf.

The following compound is prepared in an analogous manner to the process described in Example 161:

### Example

- 164: 6-[(3R,4R,5S)-4-[4-(2-Methoxy-ethoxymethyl)-phenyl]-5-((R)-2-methoxy-propoxy)-piperidin-3-yloxymethyl]-4-(3-methoxy-propyl)-3,4-dihydro-2H-benzo[1,4]oxazine
Using methyl iodide

### Example 165

### 1-{(3S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-piperidin-3-yloxy}-2-methyl-propan-2-ol

The title compound is prepared in analogy to method L from 0.51 g of 1-[(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulphonyl)-piperidin-3-yloxy]-2-methyl-propan-2-ol.

The starting material is prepared as follows:
a) 1-[(3S,4R,5R)-4-[4-(2-Methoxy-ethoxymethyl)-phenyl-5-[4-(3-methoxy-propyl)-3,4-dihydro-2H-benzo[1,4]oxazin-6-ylmethoxy]-1-(toluene-4-sulphonyl)-piperidin-3-yloxy]-2-methyl-propan-2-ol
   1.67 ml of methyl magnesium bromide (3M in diethyl ether) is added dropwise to a solution of 0.73 g of methyl [(3S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-3-yloxy] acetat (Example 69c) in 6.7 ml of tetrahydrofuran at 0°C and then the mixture is stirred at 50°C for 1 hour. The reaction mixture is cooled to 0°C and quenched with 1 M aqueous potassium bisulphate solution. The mixture is partitioned between ethyl acetate and water- the aqueous layer is re-extracted with ethyl acetate. The combined organic phases are washed with brine, dried with sodium sulphate and evaporated. The title compound is obtained as a yellowish oil from the residue by flash chromatography (SiO₂ 60F). Rf = 0.11 (EtOAc/heptane 1:1); Rt = 5.05 (gradient I).

### Example 166

### (R)-1-{(3S,4R,5R)-4-[4-(2-Methoxy-ethylsulphanylmethyl)-phenyl]-5-[4-(3-methoxy-propyl)-3,4-dihydro-2H-benzo[1,4]oxazin-6-ylmethoxy]-piperidin-3-yloxy}-propan-2-ol

0.097 g of lithium aluminium hydride are added to a solution of 0.40 g of (R)-1-[(3S,4R,5R)-4-[4-(2-methoxy-ethylsulphanylmethyl)-phenyl]-5-[4-(3-methoxy-propyl)-3,4-dihydro-2H-benzo[1,4]oxazin-6-ylmethoxy]-1-(toluene-4-sulphonyl)-piperidin-3-yloxy]-propan-2-ol in 6 ml of tetrahydrofuran at room temperature and then the mixture is stirred at 50°C for 40 hours. (Note : an additional 0.097 g of lithium aluminium hydride were added after 24 hours.) The mixture was cooled to room temperature and diluted with tert-butyl methyl ether. Water, 4M sodium hydroxide solution and again water are successively added to the reaction mixturethe resulting solid is filtered off through Hyflo, the filter cake is washed with tert-butyl methyl ether, and the filtrate is evaporated. The title compound is obtained from the residue by flash chromatography (SiO₂ 60F).

The starting materials are prepared as follows:
a) (R)-1-[(3S,4R,5R)-4-[4-(2-methoxy-ethylsulphanylmethyl)-phenyl]-5-[4-(3-methoxypropyl)-3,4-dihydro-2H-benzo[1,4]oxazin-6-ylmethoxy]-1-(toluene-4-sulphonyl)-piperidin-3-yloxy]-propan-2-ol
   0.81 g of 6-[(3R,4R,5S)-4-[4-(2-methoxy-ethylsulphanylmethyl)-phenyl]-5-((R)-1-oxiranylmethoxy)-1-(toluene-4-sulphonyl)-piperidin-3-yloxymethyl]-4-(3-methoxy-propyl)-3,4-dihydro-2H-benzo[1,4]oxazine and 0.11 g of sodium borohydride are reacted in analogy to Example 80a. The title compound is obtained as a yellow oil. Rf = 0.20 (EtOAc/heptane 3:1); Rt = 5.09 (gradient I).
b) 6-[(3R,4R,5S)-4-[4-(2-Methoxy-ethylsulphanylmethyl)-phenyl]-5-((R)-1-oxiranylmethoxy)-1-(toluene-4-sulphonyl)-piperidin-3-yloxymethyl]-4-(3-methoxy-propyl)-3,4-dihydro-2H-benzo[1,4]oxazine
   0.80 g of (3S,4S,5R)-4-[4-(2-methoxy-ethylsulphanylmethyl)-phenyl]-5-[4-(3-methoxy-propyl)-3,4-dihydro-2H-benzo[1,4]oxazin-6-ylmethoxy]-1-(toluene-4-sulphonyl)-piperidin-3-ol and 0.55 g of (R)-1-oxiranymethyl toluene-4-sulphonate [113826-06-5] are reacted in analogy to Example 68a. The title compound is obtained as an orange-brown oil. Rf = 0.20 (EtOAc/heptane 3:1); Rt = 5.29 (gradient I).
c) (3S,4S,5R)-4-[4-(2-Methoxy-ethylsulphanylmethyl)-phenyl]-5-[4-(3-methoxy-propyl)-3,4-dihydro-2H-benzo[1,4]oxazin-6-ylmethoxyl-1-(toluene-4-sulphonyl)-piperidin-3-ol
   2.04 g of 6-[(3R,4R,5S)-4-[4-(2-methoxy-ethylsulphanylmethyl)-phenyl]-1-(toluene-4-sulphonyl)-5-triisopropylsilanyloxy-piperidin-3-yloxymethyl]-4-(3-methoxy-propyl)-3,4-dihydro-2H-benzo[1,4]oxazine are reacted in analogy to method J. The title compound is obtained as a yellow oil. Rf = 0.20 (EtOAc/heptane 1:1); Rt = 4.92 (gradient I).
d) 6-[(3R,4R,5S)-4-[4-(2-Methoxy-ethylsulphanylmethyl)-phenyl]-1-(toluene-4-sulphonyl)-5-triisopropylsilanyloxy-piperidin-3-yloxymethyl]-4-(3-methoxy-propyl)-3,4-dihydro-2H-benzo[1,4]oxazine
   0.16 g of sodium hydride (60% dispersion in oil) are added to a solution of 2.27 g of 2-{4-[(3R,4R,5S)-3-[4-(3-methoxy-propyl)-3,4-dihydro-2H-benzo[1,4]oxazin-6-ylmethoxy]-1-(toluene-4-sulphonyl)-5-triisopropylsilanyloxy-piperidin-4-yl]-benzylsulphanyl}-ethanol and 0.34 ml of methyl iodide in 25 ml of tetrahydrofuran at 0°C. The reaction mixture is stirred at 0°C for 1 hour and then at room temperature for 16 hours. The mixture quenched by pouring into a mixture of 1:1 ice-water/brine and extracting three times with dichloromethane -- the combined organic layers are washed with brine, dried with sodium sulphate and evaporated.

The title compound is obtained as a yellow oil from the residue by flash chromatography (SiO₂ 60F). Rf = 0.50 (EtOAc/heptane 1:1); Rt = 32.09 (gradient II).
e) 2-{4-[(3R,4R,5S)-3-[4-(3-Methoxy-propyl)-3,4-dihydro-2H-benzo[1,4]oxazin-6-ylmethoxy]-1-(toluene-4-sulphonyl)-5-triisopropylsilanyloxy-piperidin-4-yl]-benzylsulphanyl}-ethanol
   A mixture of 2.2 g of 6-[(3R,4R,5S)-4-(4-chloromethylphenyl)-1-(toluene-4-sulphonyl)-5-triisopropylsilanyloxypiperidin-3-yloxymethyl]-4-(3-methoxypropyl)-3,4-dihydro-2H-benzo[1,4]oxazine (Example 42c), 0.23 ml of 2-mercaptoethanol and 0.60 g of potassium carbonate in 10 ml of N,N-dimethylformamide is stirred at room temperature for 18 hours. The reaction mixture is diluted with water and extracted three times with tert-butyl methyl ether- the combined organic layers are washed with brine, dried with sodium sulphate and evaporated. The crude title compound is obtained as a yellow oil. Rt = 22.92 (gradient II).

## Claims

1. Use of a compound of formula 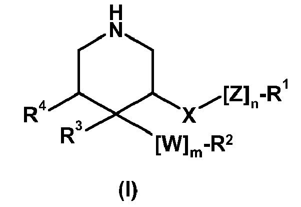 in which
(A) R¹ is heterocyclyl, optionally substituted with oxo or oxide, or as specified under (E) or (F), in particular azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1 a,7b-dihydro-1H-cyclopropa[c]chromenyl, dihydroimidazolyl, 1,3-dihydroindolyl, 2,3-dihydroindolyl, dihydro-1 H-pyrido[2,3-b][1,4]oxazinyl, 1,1-dioxodihydro-2H-benzo[1,4]thiazinyl, indazolyl, indolyl, 3H-isobenzofuranyl, [1,5]naphthyridyl, oxazolyl, 2-oxoazepanyl, 3-oxo-4H-benzo[1,4]oxazinyl, 2-oxobenzoxazolyl, 3-oxo-4H-benzo[1,4]thiazinyl, 2-oxodihydrobenzo[e][1,4]diazepinyl, 2-oxodihydrobenzo[d][1,3]oxazinyl, 2-oxodihydro-1 H-quinazolinyl, 4-oxodihydroimidazolyl, 2-oxo-1,3-dihydroindolyl, 1-oxo-3H-isobenzofuranyl, 2-oxopiperidinyl 2-oxo-1 H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, piperidinyl, pyrazolyl, 1 H-pyrido[2,3-b][1,4]oxazinyl, pyridyl, 1H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydro-quinoxalinyl, 1,1a,2,7b-tetrahydrocyclopropa[c]chromenyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl;
(B) R¹ is aryl when R² is tetrazolyl or imidazolyl which may be substituted by 1-3 halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₂₋₈-alkenyloxy-C₁₋₈-alkyl, C₁₋₈-alkoxy-C₁₋₈-alkylamino-C₁₋₈-alkyl, C₁₋₈-alkoxy-C₁₋₈-alkylsulfanyl-C₁₋₈-alkyl, C₁₋₈-alkoxy-C₃₋₈-cycloalkyl-C₀₋₈-alkoxy-C₁₋₈-alkyl, C₁₋₈-alkylsulfanyl-C₁₋₈-alkoxy-C₁₋₈-alkyl, C₁₋₈-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, optionally halogen-substituted C₁₋₈-alkoxy-C₁₋₈-alkoxy-C₁₋₈-alkyl, or heterocyclyl-C₀₋₈-alkoxy-C₁₋₈-alkyl groups, or a C₁₋₈-alkylenedioxy group, and/or by an L1-T1-L2-T2-L3-T3-L4-T4-L5-U radical; or
(C) R¹ is aryl when X is -O-CH-R¹¹-CO-NR⁹-; or
(D) R¹ is aryl when Z is -alk-NR⁹- where alk denotes C₁₋₈-alkylene, and n is 1; or
(E) R¹ is aryl which is substituted by 1-4 acetamidinyl-C₁₋₈-alkoxy, acetamidinyl-C₁₋₈-alkyl, acyl-C₁₋₈-alkoxy-C₁₋₈-alkyl, (N-acyl)-C₁₋₈-alkoxy-C₁₋₈-alkylamino, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, 1-C₁₋₈-alkoxy-C₁₋₈-alkylimidazol-2-yl, 2-C₁₋₈-alkoxy-C₁₋₈-alkyl-4-oxoimidazol-1-yl, 1-C₁₋₈-alkoxy-C₁₋₈-alkyltetrazol-5-yl, 5-C₁₋₈-alkoxy-C₁₋₈-alkyltetrazol-1-yl, 6-alkoxyaminocarbonyl-C₁₋₈-alkoxy, C₁₋₈-alkoxyaminocarbonyl-C₁₋₈-alkyl, C₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkylcarbonylamino, (N-C₁₋₈-alkyl)-C₁₋₈-alkoxycarbonylamino, (N-C₁₋₈-alkyl)-C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylamidinyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, C₁₋₈-alkylaminocarbonylamino-C₁₋₈-alkoxy, C₁₋₈-alkylaminocarbonylamino-C₁₋₈-alkyl, di-C₁₋₈-alkylaminocarbonyl-C₁₋₈-alkyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylcarbamoyl, di-C₁₋₈-alkylcarbamoyl, C₀₋₈-alkylcarbonyl, C₀₋₈-alkylcarbonylamino-C₁₋₈-alkoxy, C₀₋₈-alkylcarbonylamino, C₀₋₈-alkylcarbonylamino-C₁₋₈-alkyl, C₁₋₈-alkylcarbonyloxy-C₁₋₈-alkoxy, C₁₋₈-alkylcarbonyloxy-C₁₋₈-alkyl, C₁₋₈-alkylsulphonyl, C₁₋₈-alkylsulphonyl-C₁₋₈-alkoxy, C₁₋₈-alkylsulphonyl-C₁₋₈-alkyl, C₁₋₈-alkylsulphonylamino-C₁₋₈-alkoxy, C₁₋₈-alkylsulphonylamino-C₁₋₈-alkyl, carbamoyl, carbamoyl-C₁₋₈-alkoxy, carbamoyl-C₁₋₈-alkyl, carboxy-C₁₋₈-alkoxy, carboxy-C₁₋₈-alkoxy-C₁₋₈-alkyl, carboxy-C₁₋₈-alkyl , cyano, cyano-C₁₋₈-alkoxy, cyano-C₁₋₈-alkyl, C₃₋₈-cycloalkylcarbonylamino-C₁₋₈-alkoxy, C₃₋₈-cycloalkylcarbonylamino-C₁₋₈-alkyl, cyclopropyl-C₁₋₈-alkyl, O,N-dimethylhydroxylamino-C₁₋₈-alkyl, halo-C₁₋₈-alkoxy, halo-C₁₋₈-alkyl, halogen, hydroxy-C₁₋₈-alkoxy-C₁₋₈-alkoxy, hydroxy-C₁₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, (N-hydroxy)aminocarbonyl-C₁₋₈-alkoxy, (N-hydroxy)aminocarbonyl-C₁₋₈-alkyl, 2-oxooxazolidinyl-C₁₋₈-alkoxy, 2-oxooxazolidinyl-C₁₋₈-alkyl, O-methyloximyl-C₁₋₈-alkyl or trifluoromethyl; or
(F) R¹ is aryl which is substituted by 1-4 3-acetamidomethylpyrrolidinyl 3-C₁₋₈-alkoxy-C₁₋₈-alkylpyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolylalkoxy, imidazolylalkyl, 2-methylimidazolylalkoxy, 2-methylimidazolylalkyl, 3-methyl[1,2,4]oxadiazol-5-ylalkoxy, 5-methyl[1,2,4]oxadiazol-3-ylalkoxy, 3-methyl-[1,2,4]oxadiazol-5-ylalkyl, 5-methyl[1,2,4]oxadiazol-3-ylalkyl, 4-methylpiperazinyl, 5-methyltetrazol-1-ylalkoxy, 5-methyltetrazol-1-ylalkyl, morpholinyl, [1,2,4]oxadiazol-5-ylalkoxy, [1,2,4]oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]triazol-1-ylalkoxy, [1,2,4]triazol-4-ylalkoxy, [1,2,4]triazol-1-ylalkyl, [1,2,4]triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazo-4-ylalkyl or thiomorpholinyl;
R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl, furyl, tetrazolyl or imidazolyl, which radicals may be substituted by 1-3 halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₂₋₈-alkenyloxy-C₁₋₈-alkyl, C₁₋₈-alkoxy-C₁₋₈-alkylamino-C₁₋₈-alkyl, C₁₋₈-alkoxy-C₁₋₈-alkylsulfanyl-C₁₋₈-alkyl, C₁₋₈-alkoxy-C₃₋₈-Cycloalkyl-C₀₋₈-alkoxy-C₁₋₈-alkyl, C₁₋₈-alkylsulfanyl-C₁₋₈-alkoxy-C₁₋₈-alkyl, C₁₋₈-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, optionally halogen-substituted C₁₋₈-alkoxy-C₁₋₈-alkoxy-C₁₋₈-alkyl, heterocydyl-C₀₋₈-alkoxy-C₁₋₈-alkyl groups or a C₁₋₈-alkylenedioxy group, and/or by an L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, or are absent;
T1, T2, T3 and T4 are each independently
(a) a bond, or are absent, or are one of the groups
(b) -CH(OH)-
(c) -CH(OR⁶)-
(d) -CH(NR⁵R⁶)-
(e) -CO-
(f) -CR⁷R⁸-
(g) -O-oder-NR⁶-
(h) -S(O)₀₋₂-
(i) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) Pyrrolidinylen, Piperidinylen oder Piperazinylen
(t) -C(R¹¹)(R¹²),
where the bonds starting from (b)-(t) lead to a saturated or aromatic carbon atom of the adjacent group if the bond starts from a heteroatom, and where not more than two groups (b)-(f), three groups (g)-(h) and one group (i)-(t) is/are present;
R³ is hydrogen, hydroxyl, C₁₋₈-alkoxy or C₁₋₈-alkenyloxy;
R⁴ is optionally halogen- and/or hydroxy-substituted C₁₋₈alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₀₋₈-alkylcarbonyl-C₀₋₈-alkyl, C₁₋₈-alkoxy-C₀₋₈-alkylcarbonyl-C₀₋₈-alkyl, optionally N-C₁₋₈-alkylated C₁₋₈-alkoxycarbonylamino-C₁₋₈-alkyl, optionally N-C₁₋₈-alkylated C₁₋₈-alkoxy-C₁₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₁₋₈-alkylamino-C₁₋₈-alkyl, optionally N-C₁₋₈-alkylated or optionally halogen-substituted heterocyclyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈alkylated heterocydyl-C₀₋₈-alkylamino-C₀₋₈-alkylcarbonyl-C₀₋₈-alkyl, C₁₋₈-alkylsulphonyl-C₁₋₈-alkyl, C₂₋₈-alkinyl, heterocyclyl-C₂₋₈-alkinyl, optionally N-mono- or N,N-di-C₁₋₈-alkylated amino-C₂₋₈-alkinyl, heterocyclylcarbonyl-C₀₋₈-alkyl, heterocyclyloxy-C₁₋₈-alkyl, optionally N-mono- or N,N-di-C₁₋₈-alkylated amino, C₁₋₈-alkylcarbonyl-C₁₋₈-alkoxy, C₁₋₈-alkylcarbonyloxy, aryl-C₁₋₈-alkoxy, aryloxy, optionally N-mono- or N,N-di-C₃₋₈-cycloalkyl-C₁-C₆-alkylated carbamoyl-C₁₋₈-alkoxy, optionally N-mono- or N,N-di-C₁-C₆-alkylated carbamoyloxy, hydroxyl, hydroxy-C₁₋₈-alkoxy, hydroxy-C₁₋₈-alkoxy-C₁₋₈-alkoxy, optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₁₋₈-alkoxy, optionally N-mono- or N,N-di-C₁₋₈-alkylated or optionally hydroxy-substituted amino-C₀₋₈-alkylcarbonyl-C₁₋₈-alkoxy, hydroxy-C₀₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₋₈-alkoxycarbonylamino-C₁₋₈-alkoxy, optionally N-C₁₋₈-alkylated C₁₋₈-alkoxy-C₁₋₈-alkylamino-C₁₋₈-alkoxy, optionally N-C₁₋₈-alkylated or optionally halogen-substituted C₁₋₈-alkylcarbonylamino-C₁₋₈-alkoxy, cyano-C₁₋₈-alkoxy, optionally N-C₁₋₈-alkylated or optionally halogen-substituted C₃₋₈-cycloalkyl-C₀₋₈-alkylcarbonylamino-C₁₋₈-alkoxy, optionally N-C₁₋₈-alkylated hydroxy-C₁₋₈-alkylamino-C₁₋₈-alkoxy, optionally N-C₁₋₈-alkylated or optionally halogen-substituted heterocyclyl-C₀₋₈-alkylcarbonylamino-C₁₋₈-alkoxy, C₃₋₈-cycloalkyl-C₀₋₈-alkoxy, C₃₋₈-cycloalkyloxy-C₁₋₈-alkoxy, heterocyclyl-C₀₋₈-(optionally hydroxy-substituted)alkoxy, optionally N-C₁₋₈-alkylated heterocyclyl-C₀₋₈-alkylamino-C₀₋₈-alkylcarbonyl-C₀₋₈-alkoxy, C₁₋₈-alkylsulphonyl-C₁₋₈-alkoxy, C₂₋₈-alkinyl-oxy, heterocyclyl-C₂₋₈-alkinyl-oxy, optionally N-mono- or N,N-di-C₁₋₈-alkylated amino-C₂₋₈-alkinyl-oxy, heterocydylcarbonyl-C₀₋₈-alkoxy, heterocyclyloxy-C₁₋₈-alkyoxy or oxo;
R⁵ and R⁶ are each independently hydrogen, C₁₋₈-alkyl, C₂₋₈-alkenyl, aryl-C₁₋₈-alkyl or acyl, or, together with the N atom to which they are bonded, are a 5- to 6-membered heterocyclic ring which may contain an additional N, O or S atom or an -SO- or -SO₂- group, where the additional N atom may optionally be substituted by C₁₋₈-alkyl radicals;
R⁷ and R⁸, together with the carbon atom to which they are bonded, are a 3-8-membered ring which may contain one or two -O- or-S- atoms or-SO- or -SO₂- groups;
R⁹ is hydrogen, C₁₋₈-alkyl, C₁₋₈-alkoxy-C₁₋₈-alkyl, acyl, aryl-C₁₋₈-alkyl, C₃₋₈-cycloalkyl or C₃₋₈-cycloalkyl-C₁₋₈-alkyl;
R¹⁰ is carboxy-C₁₋₈-alkyl, C₁₋₈-alkoxycarbonyl-C₁₋₈-alkyl, C₁₋₈-alkyl or hydrogen;
R¹¹ is hydrogen, halogen, acyl, C₂₋₈-alkenyl, C₁₋₈-alkyl, or aryl-C₁₋₈-alkyl;
R¹² is hydrogen, halogen or C₁₋₈-alkyl;
R¹¹ and R¹², together with the C-atom to which they are attached, may also be C₃₋₈-cycloalkyl;
U is hydrogen, C₁₋₈-alkyl, cyano, trifluoromethyl, optionally substituted C₃₋₁₂-cycloalkyl, aryl, or heterocyclyl;
X is a bond, oxygen or sulphur or is >CR¹¹R¹², >CHOR⁹, -O-CO-, >CO, >C=NOR¹⁰, -O-CR¹¹R¹²-, -O-CR¹¹R¹²-CO-NR⁹-, -CO-NR⁹- or -NR⁹-, where a bond starting from a nitrogen, oxygen or sulphur atom leads to a saturated C atom of the Z group or to R¹;
W is oxygen or sulphur;
Z is C₁₋₈-alkylene, C₂₋₈-alkenylene, hydroxyl substituted-C₁₋₈-alkylene, -O-, -N-, -S-, -O-alk-, -NR⁹-alk, -S-alk-, -alk-O-, -alk-S- or -alk-NR⁹-, where alk denotes C₁₋₈-alkylene; and where
(a) if Z is -O- or -S-, X is -CR¹¹R¹²-; and
(b) if X is a bond, Z is C₁₋₈-alkylene, C₂₋₈-alkenylene, -NR⁹-alk-, -alk-NR⁹-, -alk-O-
or -alk-S-;
n is 1 or, when X is -O-CO-, is 0 or 1;
m is 0 or 1;
or pharmaceutically acceptable salt or prodrug thereof, or where one or more atoms are replaced by their stable, non-radioactive isotopes
for the manufacture of a medicament for the inhibition of beta-secretase, cathepsin D, plasmepsin II and/or HIV-protease.

2. Use according to claim 1 of a compound of formula (Ia) 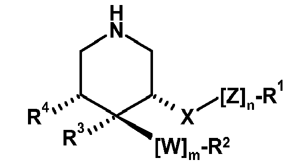 or pharmaceutically acceptable salt or prodrug thereof, or where one or more atoms are replaced by their stable, non-radioactive isotopes,
where R¹, R², R³, R⁴, W, X and Z, n and m are each as defined in claim 1.

3. Use according to claim 1 or 2, where X is oxygen, sulphur, -O-CHR¹¹-, -O-CHR¹¹-CO-NR⁹- or -CO-; and/or Z is methylene or -alk-O-.

4. Use according to any one of claims 1 to 3, where,
R² is phenyl or pyridyl, or phenyl or pyridyl, each of each is substituted by halogen, hydroxyl, cyano, trifluoromethyl, C₁₋₈-alkyl, halo-C₁₋₈-alkyl, hydroxy-C₁₋₈-alkyl, C₁₋₈-alkoxy-C₁₋₈-alkyl, cyano-C₁₋₈-alkyl, carboxy-C₁₋₈-alkyl, C₁₋₈-alkanoyloxy-C₁₋₈-alkyl, C₁₋₈-alkoxycarbonyloxy-C₁₋₈-alkyl, C₁₋₈-alkoxycarbonyl, C₁₋₈-alkoxy, C₁₋₈-alkylenedioxy, C₂₋₈-alkenyloxy-C₁₋₈-alkyl, C₁₋₈-alkoxy-C₁₋₈-alkylamino-C₁₋₈-alkyl, C₁₋₈-alkoxy-C₁₋₈-alkylsulfanyl-C₁₋₈-alkyl, C₁₋₈-alkoxy-C₃₋₈-cycloalkyl-C₀₋₈-alkoxy-C₁₋₈-alkyl, C₁₋₈-alkylsulfanyl-C₁₋₈-alkoxy-C₁₋₈-alkyl, C₁₋₈-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₈-alkoxy-C₁₋₈-alkoxy-C₁₋₈-alkyl, C₃₋₈-cydoalkyl-C₀₋₈-alkoxy-C₁₋₈-alkyl, optionally halogen-substituted C₁₋₈-alkoxy-C₁₋₈-alkoxy-C₁₋₈-alkyl or heterocydyl-C₀₋₈-alkoxy-C₁₋₈-alkyl.

5. Use according to any one of claims 1 to 4, where,
R¹ is optionally substituted benzimidazolyl or a substituted radical selected from chromenyl, 3,4-dihydro-2H-benzo[1,4]oxazinyl, 1 a,7b-dihydro-1 H-cyclopropa[c]chromenyl, indazolyl, indolyl, phenyl and 1,1a,2,7b-tetrahydrocyclopropa[c]chromenyl.

6. Use of a compound of the general formula (I) or (Ia), or a pharmaceutically acceptable salt thereof, according to any one of claims 1 to 5 for the preparation of a medication for the prevention, delay of progression or treatment of Alzheimer Disease, malaria or HIV infection.

7. Pharmaceutical preparation for the prevention, delay of progression or treatment of Alzheimer disease, malaria or HIV infection containing a compound of the general formula (I) or (la), or a pharmaceutically acceptable salt thereof, according to any one of claims 1 to 5 as well as commonly used ingredients.
